# EUROPEAN PATENT APPLICATION

(11) **EP 1 489 094 A1**
(43) Date of publication of application: **22.12.2004**
(21) Application number: 03712915.2
(22) Date of filing: 25.03.2003
(51) Int. Cl.: C07K 14/705, C12N 15/12, C12N 1/21, C12N 1/19, C12N 5/10, C12P 21/02, C07K 16/28, C12Q 1/68, G01N 33/53

(54) **NUCLEAR RECEPTOR RXR ALPHA ISOFORMS**

(30) Priority: 25.03.2002 JP 2002084559
(71) Applicant: FUJISAWA PHARMACEUTICAL CO., LTD., Osaka-shi Osaka 541-8514 (JP)
(72) Inventor: KOJO, Hitoshi, Kamigyoku, Kyoto 602-0 (JP); TAJIMA, Kaoru, Oozato-gun, Saitama 369-1203 (JP); FUKAGAWA, Masao, Osaka-shi, Osaka 541-8514 (JP); NISHIMURA, Shintaro, Osaka-shi, Osaka 541-8514 (JP); ISOGAI, Takao, Inashiki-gun, Ibaraki 300-030 (JP)
(74) Representative: Gille Hrabal Struck Neidlein Prop Roos
(86) International application number: PCT/JP2003/003612
(87) International publication number: WO 2003/080668

(57) **Abstract**

The present invention relates to the novel isoforms, RXRα2 and RXRα3, of nuclear receptor RXRα. Unlike known isoform RXRα1, the transcriptional activation functions of RXRα2 and RXRα3 are augmented by SRC-1. The present invention provides methods for evaluating the function of regulating augmentation by co-activators of these RXRα isoforms, and screening methods based on these evaluation methods. By controlling the interaction between isoforms and co-activators, transcription-controlling activity can be regulated in an isoform-specific manner.

SEQUENCE LISTING

<110> FUJISAWA PHARMACEUTICAL CO., LTD.

<120> ISOFORMS OF NUCLEAR RECEPTOR RXRα

<130> F3-A0201P

<140>
<141>

<150> JP 2002-084559
<151> 2002-03-25

<160> 24

<170> PatentIn Ver. 2.1

<210> 1
<211> 2393
<212> DNA
<213> Homo sapiens

<220>
<221> CDS
<222> (923)..(2230)

<400> 1

acagatgccc tgtgcatggg gtggggatga aggagggctg ggggctccgt gacagacagt 60

gggaggggag gctggctgtc ctgtggtgag ctgcttctgg ggcaccccga gatggggcct 120

cccttcactt cctggccatc caggcatctg tgtctgtgtc cgggaagtgg aggagggacc 180

ccaggaccga gtcgctgccc atcactgacc gtctgtggca ggcactgcca gtggccgtgg 240

cccagggtct tggtggctcg gcctcatcgg gggcttgctc caggccttgg gtgatgctcc 300

tgtgctccct gacggccgca gggtctcacc ctcctgctcc agcccctctc tgaccccacc 360

ctgcggtcct gggaggtagc aggacagccc cagtatgcgg atgagaaaac ctgaggccca 420

gcctgggcgg gttcgtgggt ccagctcagc ccctctgtgc ctgtggtctg tagggacctg 480

ggcccacctg cccctaagga tggagctcag tcttccccag ctccaaccct ggggccttgt 540

gtgggagggg cagtggccct gggatctggc cttggcagct ccagccccct ctccagtatg 600

gcagagcctg gaggcatgct ggtctctggg tctgagactc tggcctccca gcctgctccc 660

ctgggggttg gtggaggcct tggggctgtc ggagcttcag gattagggac tgaagcccag 720

caggggctcc cttttctgtg ccccctgcct gagcacaatc ttcctgccct ctcccctgca 780

gcccctctgt tgtcgtggtc agtcgtgccg cctgagggcc aagtgtggcc ttggcactgc 840

caccgtccac cagcggcaag tcagggcaga tttctccacc caggtgaact cctccctcac 900

ctccccgacg gggcgaggct cc atg gct gcc ccc tcg ctg cac ccg tcc ctg 952

Met Ala Ala Pro Ser Leu His Pro Ser Leu

1               5                   10

ggg cct ggc atc ggc tcc ccg gga cag ctg cat tct ccc atc agc acc 1000

Gly Pro Gly Ile Gly Ser Pro Gly Gln Leu His Ser Pro Ile Ser Thr

                15                  20                  25

ctg agc tcc ccc atc aac ggc atg ggc ccg cct ttc tcg gtc atc agc 1048
Leu Ser Ser Pro Ile Asn Gly Met Gly Pro Pro Phe Ser Val Ile Ser
            30                  35                  40

tcc ccc atg ggc ccc cac tcc atg tcg gtg ccc acc aca ccc acc ctg 1096
Ser Pro Met Gly Pro His Ser Met Ser Val Pro Thr Thr Pro Thr Leu
        45                  50                  55

ggc ttc agc act ggc agc ccc cag ctc agc tca cct atg aac ccc gtc 1144
Gly Phe Ser Thr Gly Ser Pro Gln Leu Ser Ser Pro Met Asn Pro Val
    60                  65                  70

agc agc agc gag gac atc aag ccc ccc ctg ggc ctc aat ggc gtc ctc 1192
Ser Ser Ser Glu Asp Ile Lys Pro Pro Leu Gly Leu Asn Gly Val Leu
75                  80                  85                  90

aag gtc ccc gcc cac ccc tca gga aac atg gct tcc ttc acc aag cac 1240
Lys Val Pro Ala His Pro Ser Gly Asn Met Ala Ser Phe Thr Lys His
                95                  100                 105

atc tgc gcc atc tgc ggg gac cgc tcc tca ggc aag cac tat gga gtg 1288
Ile Cys Ala Ile Cys Gly Asp Arg Ser Ser Gly Lys His Tyr Gly Val
            110                 115                 120

tac agc tgc gag ggg tgc aag ggc ttc ttc aag cgg acg gtg cgc aag 1336
Tyr Ser Cys Glu Gly Cys Lys Gly Phe Phe Lys Arg Thr Val Arg Lys

        125                 130                 135

gac ctg acc tac acc tgc cgc gac aac aag gac tgc ctg att gac aag 1384
Asp Leu Thr Tyr Thr Cys Arg Asp Asn Lys Asp Cys Leu Ile Asp Lys
    140                 145                 150

cgg cag cgg aac cgg tgc cag tac tgc cgc tac cag aag tgc ctg gcc 1432
Arg Gln Arg Asn Arg Cys Gln Tyr Cys Arg Tyr Gln Lys Cys Leu Ala
155                 160                 165                 170

atg ggc atg aag cgg gaa gcc gtg cag gag gag cgg cag cgt ggc aag 1480
Met Gly Met Lys Arg Glu Ala Val Gln Glu Glu Arg Gln Arg Gly Lys
                175                 180                 185

gac cgg aac gag aat gag gtg gag tcg acc agc agc gcc aac gag gac 1528
Asp Arg Asn Glu Asn Glu Val Glu Ser Thr Ser Ser Ala Asn Glu Asp
            190                 195                 200

atg ccg gtg gag agg atc ctg gag gct gag ctg gcc gtg gag ccc aag 1576
Met Pro Val Glu Arg Ile Leu Glu Ala Glu Leu Ala Val Glu Pro Lys
        205                 210                 215

acc gag acc tac gtg gag gca aac atg ggg ctg aac ccc agc tcg ccg 1624
Thr Glu Thr Tyr Val Glu Ala Asn Met Gly Leu Asn Pro Ser Ser Pro
    220                 225                 230

aac gac cct gtc acc aac att tgc caa gca gcc gac aaa cag ctt ttc 1672
Asn Asp Pro Val Thr Asn Ile Cys Gln Ala Ala Asp Lys Gln Leu Phe
235                 240                 245                 250

acc ctg gtg gag tgg gcc aag cgg atc cca cac ttc tca gag ctg ccc 1720
Thr Leu Val Glu Trp Ala Lys Arg Ile Pro His Phe Ser Glu Leu Pro
                255                 260                 265

ctg gac gac cag gtc atc ctg ctg cgg gca ggc tgg aat gag ctg ctc 1768
Leu Asp Asp Gln Val Ile Leu Leu Arg Ala Gly Trp Asn Glu Leu Leu
            270                 275                 280

atc gcc tcc ttc tcc cac cgc tcc atc gcc gtg aag gac ggg atc ctc 1816
Ile Ala Ser Phe Ser His Arg Ser Ile Ala Val Lys Asp Gly Ile Leu
        285                 290                 295

ctg gcc acc ggg ctg cac gtc cac cgg aac agc gcc cac agc gca ggg 1864
Leu Ala Thr Gly Leu His Val His Arg Asn Ser Ala His Ser Ala Gly
    300                 305                 310

gtg ggc gcc atc ttt gac agg gtg ctg acg gag ctt gtg tcc aag atg 1912
Val Gly Ala Ile Phe Asp Arg Val Leu Thr Glu Leu Val Ser Lys Met
315                 320                 325                 330

cgg gac atg cag atg gac aag acg gag ctg ggc tgc ctg cgc gcc atc 1960
Arg Asp Met Gln Met Asp Lys Thr Glu Leu Gly Cys Leu Arg Ala Ile

                335                 340                 345

gtc ctc ttt aac cct gac tcc aag ggg ctc tcg aac ccg gcc gag gtg 2008
Val Leu Phe Asn Pro Asp Ser Lys Gly Leu Ser Asn Pro Ala Glu Val
            350                 355                 360

gag gcg ctg agg gag aag gtc tat gcg tcc ttg gag gcc tac tgc aag 2056
Glu Ala Leu Arg Glu Lys Val Tyr Ala Ser Leu Glu Ala Tyr Cys Lys
        365                 370                 375

cac aag tac cca gag cag ccg gga agg ttc gct aag ctc ttg ctc cgc 2104
His Lys Tyr Pro Glu Gln Pro Gly Arg Phe Ala Lys Leu Leu Leu Arg
    380                 385                 390

ctg ccg gct ctg cgc tcc atc ggg ctc aaa tgc ctg gaa cat ctc ttc 2152
Leu Pro Ala Leu Arg Ser Ile Gly Leu Lys Cys Leu Glu His Leu Phe
395                 400                 405                 410

ttc ttc aag ctc atc ggg gac aca ccc att gac acc ttc ctt atg gag 2200
Phe Phe Lys Leu Ile Gly Asp Thr Pro Ile Asp Thr Phe Leu Met Glu
                415                 420                 425

atg ctg gag gcg ccg cac caa atg act tag gcctgcgggc ccatcctttg 2250
Met Leu Glu Ala Pro His Gln Met Thr
            430                 435

tgcccacccg ttctggccac cctgcctgga cgccagctgt tcttctcagc ctgagccctg 2310
tccctgccct tctctgcctg gcctgtttgg actttggggc acagcctgtc actgctctgc 2370
ctaagagatg tgttgtcacc ctc 2393

<210> 2
<211> 435
<212> PRT
<213> Homo sapiens

<400> 2
Met Ala Ala Pro Ser Leu His Pro Ser Leu Gly Pro Gly Ile Gly Ser
1               5                   10                  15
Pro Gly Gln Leu His Ser Pro Ile Ser Thr Leu Ser Ser Pro Ile Asn
            20                  25                  30
Gly Met Gly Pro Pro Phe Ser Val Ile Ser Ser Pro Met Gly Pro His
        35                  40                  45
Ser Met Ser Val Pro Thr Thr Pro Thr Leu Gly Phe Ser Thr Gly Ser
    50                  55                  60
Pro Gln Leu Ser Ser Pro Met Asn Pro Val Ser Ser Ser Glu Asp Ile
65                  70                  75                  80
Lys Pro Pro Leu Gly Leu Asn Gly Val Leu Lys Val Pro Ala His Pro
                85                  90                  95
Ser Gly Asn Met Ala Ser Phe Thr Lys His Ile Cys Ala Ile Cys Gly
            100                 105                 110
Asp Arg Ser Ser Gly Lys His Tyr Gly Val Tyr Ser Cys Glu Gly Cys

        115                 120                 125
Lys Gly Phe Phe Lys Arg Thr Val Arg Lys Asp Leu Thr Tyr Thr Cys
    130                 135                 140
Arg Asp Asn Lys Asp Cys Leu Ile Asp Lys Arg Gln Arg Asn Arg Cys
145                 150                 155                 160
Gln Tyr Cys Arg Tyr Gln Lys Cys Leu Ala Met Gly Met Lys Arg Glu
                165                 170                 175
Ala Val Gln Glu Glu Arg Gln Arg Gly Lys Asp Arg Asn Glu Asn Glu
            180                 185                 190
Val Glu Ser Thr Ser Ser Ala Asn Glu Asp Met Pro Val Glu Arg Ile
        195                 200                 205
Leu Glu Ala Glu Leu Ala Val Glu Pro Lys Thr Glu Thr Tyr Val Glu
    210                 215                 220
Ala Asn Met Gly Leu Asn Pro Ser Ser Pro Asn Asp Pro Val Thr Asn
225                 230                 235                 240
Ile Cys Gln Ala Ala Asp Lys Gln Leu Phe Thr Leu Val Glu Trp Ala
                245                 250                 255
Lys Arg Ile Pro His Phe Ser Glu Leu Pro Leu Asp Asp Gln Val Ile
            260                 265                 270
Leu Leu Arg Ala Gly Trp Asn Glu Leu Leu Ile Ala Ser Phe Ser His
        275                 280                 285
Arg Ser Ile Ala Val Lys Asp Gly Ile Leu Leu Ala Thr Gly Leu His
    290                 295                 300
Val His Arg Asn Ser Ala His Ser Ala Gly Val Gly Ala Ile Phe Asp
305                 310                 315                 320
Arg Val Leu Thr Glu Leu Val Ser Lys Met Arg Asp Met Gln Met Asp

                325                 330                 335

Lys Thr Glu Leu Gly Cys Leu Arg Ala Ile Val Leu Phe Asn Pro Asp

            340                 345                 350

Ser Lys Gly Leu Ser Asn Pro Ala Glu Val Glu Ala Leu Arg Glu Lys

        355                 360                 365

Val Tyr Ala Ser Leu Glu Ala Tyr Cys Lys His Lys Tyr Pro Glu Gln

    370                 375                 380

Pro Gly Arg Phe Ala Lys Leu Leu Leu Arg Leu Pro Ala Leu Arg Ser

385                 390                 395                 400

Ile Gly Leu Lys Cys Leu Glu His Leu Phe Phe Phe Lys Leu Ile Gly

                405                 410                 415

Asp Thr Pro Ile Asp Thr Phe Leu Met Glu Met Leu Glu Ala Pro His

            420                 425                 430

Gln Met Thr

        435

<210> 3

<211> 2421

<212> DNA

<213> Homo sapiens

<220>

<221> CDS

<222> (924)..(2021)

<400> 3
   actagaccag cgagccctgg agggctgggc agggccttgt cccctggtgc ctcccggagc 60
   ccagcacggg gcagggccag ctgggcactt agtttggacg agctctgtcc ctccaggggc 120
   ttcactttcg ccttctgcag cgtgggggat catgtgtgac cttggccagc ccctgacctc 180
   cctgagccgc agcctctgca tctgggacac ccagggtttc ccatgcaggg agtgtcaagt 240
   gcgggtgcaa agcccaggca ccgcacccct ctgcagacgt ctggggcttt ctcttgcccc 300
   ttcagttccc tcccttctct gagaagcgca cctgctgcct gtggacatga acactgggca 360
   tttggtgagt cacacagtgg ccacccggtt ctccagtggg agcttcatgg cacccccact 420
   gtgctgagtt gtcacggggg acctcctggc ctctcgcagc ccacctgagt cctgcaggga 480
   ccaagcatcc ttgtaaagcc tcacagccca cctgggcaga tcgaggaaga acctagaagc 540
   cagaacaagc tggggctgga agactcattc caggcaccga ggtccatgtg aaaggaggct 600
   ttgcggtggt ttctgctttg cttgcattga gcctcaaact cttcctgtcc cgtgagaagg 660
   ggggatggga tttggaggcc tgaggaagga aggaggggag cttcctgcgc acaagcggcg 720
   gcaggggttt gcagaagtgg aggctggggg ctgcctggcc aagcgcttac cgccctgcgc 780
   agccgggctg gctggcaggc tgcagcggga agcgcctgtg ggtcctcggc gctgactgca 840
   gagctgggtg gaggcagcgg aaccaaaact gctgtgtcac tgcacgctgc agctgttgcc 900
   agggtgaccg gctcagctca cct atg aac ccc gtc agc agc agc gag gac atc 953
   Met Asn Pro Val Ser Ser Ser Glu Asp Ile
   1 5 10
   aag ccc ccc ctg ggc ctc aat ggc gtc ctc aag gtc ccc gcc cac ccc 1001
   Lys Pro Pro Leu Gly Leu Asn Gly Val Leu Lys Val Pro Ala His Pro
   15 20 25
   tca gga aac atg gct tcc ttc acc aag cac atc tgc gcc atc tgc ggg 1049
   Ser Gly Asn Met Ala Ser Phe Thr Lys His Ile Cys Ala Ile Cys Gly
   30 35 40
   gac cgc tcc tca ggc aag cac tat gga gtg tac agc tgc gag ggg tgc 1097
   Asp Arg Ser Ser Gly Lys His Tyr Gly Val Tyr Ser Cys Glu Gly Cys
   45 50 55
   aag ggc ttc ttc aag cgg acg gtg cgc aag gac ctg acc tac acc tgc 1145
   Lys Gly Phe Phe Lys Arg Thr Val Arg Lys Asp Leu Thr Tyr Thr Cys
   60 65 70
   cgc gac aac aag gac tgc ctg att gac aag cgg cag cgg aac cgg tgc 1193
   Arg Asp Asn Lys Asp Cys Leu Ile Asp Lys Arg Gln Arg Asn Arg Cys
   75 80 85 90
   cag tac tgc cgc tac cag aag tgc ctg gcc atg ggc atg aag cgg gaa 1241
   Gln Tyr Cys Arg Tyr Gln Lys Cys Leu Ala Met Gly Met Lys Arg Glu
   95 100 105
   gcc gtg cag gag gag cgg cag cgt ggc aag gac cgg aac gag aat gag 1289
   Ala Val Gln Glu Glu Arg Gln Arg Gly Lys Asp Arg Asn Glu Asn Glu
   110 115 120
   gtg gag tcg acc agc agc gcc aac gag gac atg ccg gtg gag agg atc 1337
   Val Glu Ser Thr Ser Ser Ala Asn Glu Asp Met Pro Val Glu Arg Ile
   125 130 135
   ctg gag gct gag ctg gcc gtg gag ccc aag acc gag acc tac gtg gag 1385
   Leu Glu Ala Glu Leu Ala Val Glu Pro Lys Thr Glu Thr Tyr Val Glu
   140 145 150
   gca aac atg ggg ctg aac ccc agc tcg ccg aac gac cct gtc acc aac 1433
   Ala Asn Met Gly Leu Asn Pro Ser Ser Pro Asn Asp Pro Val Thr Asn
   155 160 165 170
   att tgc caa gca gcc gac aaa cag ctt ttc acc ctg gtg gag tgg gcc 1481
   Ile Cys Gln Ala Ala Asp Lys Gln Leu Phe Thr Leu Val Glu Trp Ala
   175 180 185
   aag cgg atc cca cac ttc tca gag ctg ccc ctg gac gac cag gtc atc 1529
   Lys Arg Ile Pro His Phe Ser Glu Leu Pro Leu Asp Asp Gln Val Ile
   190 195 200
   ctg ctg cgg gca ggc tgg aat gag ctg ctc atc gcc tcc ttc tcc cac 1577
   Leu Leu Arg Ala Gly Trp Asn Glu Leu Leu Ile Ala Ser Phe Ser His
   205 210 215
   cgc tcc atc gcc gtg aag gac ggg atc ctc ctg gcc acc ggg ctg cac 1625
   Arg Ser Ile Ala Val Lys Asp Gly Ile Leu Leu Ala Thr Gly Leu His
   220 225 230
   gtc cac cgg aac agc gcc cac agc gca ggg gtg ggc gcc atc ttt gac 1673
   Val His Arg Asn Ser Ala His Ser Ala Gly Val Gly Ala Ile Phe Asp
   235 240 245 250
   agg gtg ctg acg gag ctt gtg tcc aag atg cgg gac atg cag atg gac 1721
   Arg Val Leu Thr Glu Leu Val Ser Lys Met Arg Asp Met Gln Met Asp
   255 260 265
   aag acg gag ctg ggc tgc ctg cgc gcc atc gtc ctc ttt aac cct gac 1769
   Lys Thr Glu Leu Gly Cys Leu Arg Ala Ile Val Leu Phe Asn Pro Asp
   270 275 280
   tcc aag ggg ctc tcg aac ccg gcc gag gtg gag gcg ctg agg gag aag 1817
   Ser Lys Gly Leu Ser Asn Pro Ala Glu Val Glu Ala Leu Arg Glu Lys
   285 290 295
   gtc tat gcg tcc ttg gag gcc tac tgc aag cac aag tac cca gag cag 1865
   Val Tyr Ala Ser Leu Glu Ala Tyr Cys Lys His Lys Tyr Pro Glu Gln
   300 305 310
   ccg gga agg ttc gct aag ctc ttg ctc cgc ctg ccg gct ctg cgc tcc 1913
   Pro Gly Arg Phe Ala Lys Leu Leu Leu Arg Leu Pro Ala Leu Arg Ser
   315 320 325 330
   atc ggg ctc aaa tgc ctg gaa cat ctc ttc ttc ttc aag ctc atc ggg 1961
   Ile Gly Leu Lys Cys Leu Glu His Leu Phe Phe Phe Lys Leu Ile Gly
   335 340 345
   gac aca ccc att gac acc ttc ctt atg gag atg ctg gag gcg ccg cac 2009
   Asp Thr Pro Ile Asp Thr Phe Leu Met Glu Met Leu Glu Ala Pro His
   350 355 360
   caa atg act tag gcctgcgggc ccatcctttg tgcccacccg ttctggccac 2061
   Gln Met Thr
   365
   cctgcctgga cgccagctgt tcttctcagc ctgagccctg tccctgccct tctctgcctg 2121
   gcctgtttgg actttggggc acagcctgtc actgctctgc ctaagagatg tgttgtcacc 2181
   ctccttattt ctgttactac ttgtctgtgg cccagggcag tggctttcct gaggcagcag 2241
   ccttcgtggc aagaactagc gtgagcccag ccaggcgcct ccccaccggg ctctcaggac 2301
   accctgccac accccacggg gcttgggcga ctacagggtc ttcgggcccc agccctggag 2361
   ctgcaggagt tgggaacggg gcttttgttt ccgttgctgt ttatcgatgc tggttttcag 2421

<210> 4
<211> 365
<212> PRT
<213> Homo sapiens

<400> 4
Met Asn Pro Val Ser Ser Ser Glu Asp Ile Lys Pro Pro Leu Gly Leu
1               5                   10                  15
Asn Gly Val Leu Lys Val Pro Ala His Pro Ser Gly Asn Met Ala Ser

            20                  25                  30
Phe Thr Lys His Ile Cys Ala Ile Cys Gly Asp Arg Ser Ser Gly Lys
        35                  40                  45
His Tyr Gly Val Tyr Ser Cys Glu Gly Cys Lys Gly Phe Phe Lys Arg
    50                  55                  60
Thr Val Arg Lys Asp Leu Thr Tyr Thr Cys Arg Asp Asn Lys Asp Cys
65                  70                  75                  80
Leu Ile Asp Lys Arg Gln Arg Asn Arg Cys Gln Tyr Cys Arg Tyr Gln
                85                  90                  95
Lys Cys Leu Ala Met Gly Met Lys Arg Glu Ala Val Gln Glu Glu Arg
            100                 105                 110
Gln Arg Gly Lys Asp Arg Asn Glu Asn Glu Val Glu Ser Thr Ser Ser
        115                 120                 125
Ala Asn Glu Asp Met Pro Val Glu Arg Ile Leu Glu Ala Glu Leu Ala
    130                 135                 140
Val Glu Pro Lys Thr Glu Thr Tyr Val Glu Ala Asn Met Gly Leu Asn
145                 150                 155                 160
Pro Ser Ser Pro Asn Asp Pro Val Thr Asn Ile Cys Gln Ala Ala Asp
                165                 170                 175
Lys Gln Leu Phe Thr Leu Val Glu Trp Ala Lys Arg Ile Pro His Phe
            180                 185                 190
Ser Glu Leu Pro Leu Asp Asp Gln Val Ile Leu Leu Arg Ala Gly Trp
        195                 200                 205
Asn Glu Leu Leu Ile Ala Ser Phe Ser His Arg Ser Ile Ala Val Lys
    210                 215                 220
Asp Gly Ile Leu Leu Ala Thr Gly Leu His Val His Arg Asn Ser Ala

225                 230                 235                 240

His Ser Ala Gly Val Gly Ala Ile Phe Asp Arg Val Leu Thr Glu Leu

                245                 250                 255

Val Ser Lys Met Arg Asp Met Gln Met Asp Lys Thr Glu Leu Gly Cys

            260                 265                 270

Leu Arg Ala Ile Val Leu Phe Asn Pro Asp Ser Lys Gly Leu Ser Asn

        275                 280                 285

Pro Ala Glu Val Glu Ala Leu Arg Glu Lys Val Tyr Ala Ser Leu Glu

    290                 295                 300

Ala Tyr Cys Lys His Lys Tyr Pro Glu Gln Pro Gly Arg Phe Ala Lys

305                 310                 315                 320

Leu Leu Leu Arg Leu Pro Ala Leu Arg Ser Ile Gly Leu Lys Cys Leu

                325                 330                 335

Glu His Leu Phe Phe Phe Lys Leu Ile Gly Asp Thr Pro Ile Asp Thr

            340                 345                 350

Phe Leu Met Glu Met Leu Glu Ala Pro His Gln Met Thr

        355                 360                 365

<210> 5

<211> 5421

<222> (76)..(1464)

<400> 5

gaattccggc gccgggggcc gcccgcccgc cgcccgctgc ctgcgccgcc ggccgggcat 60

gagttagtcg cagac atg gac acc aaa cat ttc ctg ccg ctc gat ttc tcc 111

Met Asp Thr Lys His Phe Leu Pro Leu Asp Phe Ser

1               5                   10

acc cag gtg aac tcc tcc ctc acc tcc ccg acg ggg cga ggc tcc atg 159

Thr Gln Val Asn Ser Ser Leu Thr Ser Pro Thr Gly Arg Gly Ser Met

        15                  20                  25

gct gcc ccc tcg ctg cac ccg tcc ctg ggg cct ggc atc ggc tcc ccg 207

Ala Ala Pro Ser Leu His Pro Ser Leu Gly Pro Gly Ile Gly Ser Pro

    30                  35                  40

gga cag ctg cat tct ccc atc agc acc ctg agc tcc ccc atc aac ggc 255

Gly Gln Leu His Ser Pro Ile Ser Thr Leu Ser Ser Pro Ile Asn Gly

45                  50                  55                  60

atg ggc ccg cct ttc tcg gtc atc agc tcc ccc atg ggc ccc cac tcc 303

Met Gly Pro Pro Phe Ser Val Ile Ser Ser Pro Met Gly Pro His Ser

                65                  70                  75

atg tcg gtg ccc acc aca ccc acc ctg ggc ttc agc act ggc agc ccc 351

Met Ser Val Pro Thr Thr Pro Thr Leu Gly Phe Ser Thr Gly Ser Pro
            80                  85                  90

cag ctc agc tca cct atg aac ccc gtc agc agc agc gag gac atc aag 399
Gln Leu Ser Ser Pro Met Asn Pro Val Ser Ser Ser Glu Asp Ile Lys
        95                  100                 105

ccc ccc ctg ggc ctc aat ggc gtc ctc aag gtc ccc gcc cac ccc tca 447
Pro Pro Leu Gly Leu Asn Gly Val Leu Lys Val Pro Ala His Pro Ser
    110                 115                 120

gga aac atg gct tcc ttc acc aag cac atc tgc gcc atc tgc ggg gac 495
Gly Asn Met Ala Ser Phe Thr Lys His Ile Cys Ala Ile Cys Gly Asp
125                 130                 135                 140

cgc tcc tca ggc aag cac tat gga gtg tac agc tgc gag ggg tgc aag 543
Arg Ser Ser Gly Lys His Tyr Gly Val Tyr Ser Cys Glu Gly Cys Lys
                145                 150                 155

ggc ttc ttc aag cgg acg gtg cgc aag gac ctg acc tac acc tgc cgc 591
Gly Phe Phe Lys Arg Thr Val Arg Lys Asp Leu Thr Tyr Thr Cys Arg
            160                 165                 170

gac aac aag gac tgc ctg att gac aag cgg cag cgg aac cgg tgc cag 639
Asp Asn Lys Asp Cys Leu Ile Asp Lys Arg Gln Arg Asn Arg Cys Gln
        175                 180                 185

tac tgc cgc tac cag aag tgc ctg gcc atg ggc atg aag cgg gaa gcc 687
Tyr Cys Arg Tyr Gln Lys Cys Leu Ala Met Gly Met Lys Arg Glu Ala
    190                 195                 200

gtg cag gag gag cgg cag cgt ggc aag gac cgg aac gag aat gag gtg 735
Val Gln Glu Glu Arg Gln Arg Gly Lys Asp Arg Asn Glu Asn Glu Val
205                 210                 215                 220

gag tcg acc agc agc gcc aac gag gac atg ccg gtg gag agg atc ctg 783
Glu Ser Thr Ser Ser Ala Asn Glu Asp Met Pro Val Glu Arg Ile Leu
                225                 230                 235

gag gct gag ctg gcc gtg gag ccc aag acc gag acc tac gtg gag gca 831
Glu Ala Glu Leu Ala Val Glu Pro Lys Thr Glu Thr Tyr Val Glu Ala
            240                 245                 250

aac atg ggg ctg aac ccc agc tcg ccg aac gac cct gtc acc aac att 879
Asn Met Gly Leu Asn Pro Ser Ser Pro Asn Asp Pro Val Thr Asn Ile
        255                 260                 265

tgc caa gca gcc gac aaa cag ctt ttc acc ctg gtg gag tgg gcc aag 927
Cys Gln Ala Ala Asp Lys Gln Leu Phe Thr Leu Val Glu Trp Ala Lys
    270                 275                 280

cgg atc cca cac ttc tca gag ctg ccc ctg gac gac cag gtc atc ctg 975

Arg Ile Pro His Phe Ser Glu Leu Pro Leu Asp Asp Gln Val Ile Leu
285                 290                 295                 300

ctg cgg gca ggc tgg aat gag ctg ctc atc gcc tcc ttc tcc cac cgc 1023
Leu Arg Ala Gly Trp Asn Glu Leu Leu Ile Ala Ser Phe Ser His Arg
                305                 310                 315

tcc atc gcc gtg aag gac ggg atc ctc ctg gcc acc ggg ctg cac gtc 1071
Ser Ile Ala Val Lys Asp Gly Ile Leu Leu Ala Thr Gly Leu His Val
            320                 325                 330

cac cgg aac agc gcc cac agc gca ggg gtg ggc gcc atc ttt gac agg 1119
His Arg Asn Ser Ala His Ser Ala Gly Val Gly Ala Ile Phe Asp Arg
        335                 340                 345

gtg ctg acg gag ctt gtg tcc aag atg cgg gac atg cag atg gac aag 1167
Val Leu Thr Glu Leu Val Ser Lys Met Arg Asp Met Gln Met Asp Lys
    350                 355                 360

acg gag ctg ggc tgc ctg cgc gcc atc gtc ctc ttt aac cct gac tcc 1215
Thr Glu Leu Gly Cys Leu Arg Ala Ile Val Leu Phe Asn Pro Asp Ser
365                 370                 375                 380

aag ggg ctc tcg aac ccg gcc gag gtg gag gcg ctg agg gag aag gtc 1263
Lys Gly Leu Ser Asn Pro Ala Glu Val Glu Ala Leu Arg Glu Lys Val
                385                 390                 395

tat gcg tcc ttg gag gcc tac tgc aag cac aag tac cca gag cag ccg 1311
Tyr Ala Ser Leu Glu Ala Tyr Cys Lys His Lys Tyr Pro Glu Gln Pro
            400                 405                 410

gga agg ttc gct aag ctc ttg ctc cgc ctg ccg gct ctg cgc tcc atc 1359
Gly Arg Phe Ala Lys Leu Leu Leu Arg Leu Pro Ala Leu Arg Ser Ile
        415                 420                 425

ggg ctc aaa tgc ctg gaa cat ctc ttc ttc ttc aag ctc atc ggg gac 1407
Gly Leu Lys Cys Leu Glu His Leu Phe Phe Phe Lys Leu Ile Gly Asp
    430                 435                 440

aca ccc att gac acc ttc ctt atg gag atg ctg gag gcg ccg cac caa 1455
Thr Pro Ile Asp Thr Phe Leu Met Glu Met Leu Glu Ala Pro His Gln
445                 450                 455                 460

atg act tag gcctgcgggc ccatcctttg tgcccacccg ttctggccac 1504
Met Thr

cctgcctgga cgccagctgt tcttctcagc ctgagccctg tccctgccct tctctgcctg 1564
gcctgtttgg actttggggc acagcctgtc actgctctgc ctaagagatg tgttgtcacc 1624
ctccttattt ctgttactac ttgtctgtgg cccagggcag tggctttcct gagcagcagc 1684
cttcgtggca agaactagcg tgagcccagc caggcgcctc cccaccgggc tctcaggacg 1744
ccctgccaca cccacggggc ttgggcgact acagggtctt cggccccagc cctggagctg 1804
caggagttgg gaacggggct tttgtttccg ttgctgttta tcgatgctgg ttttcagaat 1864

tcctgtgtgg ccctcctgtc tggagtgaca tcttcatctg ctctgaatac tggtgcccag 1924
ccagcccgtg acagcttccc cctaatcagg aggggacagc tgggggcgca agctggtgtg 1984
tcatcagcaa agacctcagc cgcctcgggg atganagggg actcgtgggg caagcaagct 2044
gccctgtgct ctgagtgagg gggaaggtag cccctttttc caaaggtaac tcacagtttt 2104
gccctcgagc caatgagaac atgagctgcc ctctgtgcaa ggtttcgggg ccacctccag 2164
gctgcagggg cgggtcactc gcccccctgt tttctctctg ccttggtgtt ctggtttcag 2224
actcccgact ccccgttcag accagagtgc cccagcccct ccccagcctg agtcttctcc 2284
ttgctctgcg gggtgggctg agacttgtcc ttgtttcctg cagggctggc cctggctcgg 2344
gcagggtggg gcatcaccac ctcactggcc ttgctggagg cacagggctc tgcggacctg 2404
cagccatctg tgaggcccgc ggggatgggg ggggaggagg gtggcctgtt ggtttccctc 2464
agagggggca ggtggcctgg agagagaggg gctcaggaac tgggagcctg gtgggtgggg 2524
cagatgctcc gcggcctgga gtggttctgc cggggcattg gtgggacccc tgctcaggcc 2584
ttctctctgg ctgccagttg tgtctaaaag actcttggaa tctgagaacc cggagtcgca 2644
gcgccctcgg gcctgggcca cacgcaggcc ctggtgggac cacccagcct ggtattgtcc 2704
acggacagcg ttgttcaccc agagccttac ttgggagcct cactgaacgc ctgctctggt 2764
tgaaggtggg gtgggggcgg ggcttggggc ctccctggct cagcccagtg cggcctggcg 2824
ctcctcccgc aggctctgcc cccgggctcc ggtggtgcgg ggccctctca ggttgaactc 2884
gcctcttttg cactggaagg tcctcccttt ggcctgagta cttttcctgt tcacgcctca 2944
gtcccgtgga cccagccttt gtcagtggca ggtgcctgaa cagagggtgg atggggggga 3004
taccggaggg ggtcttgtct tcccagccgc agtctaggaa tgatgcgggg gggtggacgc 3064
cttctccata gtctttcccc acctggagca ggggcttcct cagtggtgag gggagctgcc 3124
tacaggttgg accgggaggc agtggcttgg agaggcagct ttccagcctt ggtggggaag 3184
aaagtgtcca ttctttgcct tcctggagct cccagccaga gctgagctta ggcacccgag 3244
tggagcctgc agctgagtct gtgcccgaga caggctgtca gagattccag aagcctctcc 3304
tccccgccgc cctccacccc tgcctttcag cgttgtggat ccctagaggt ggccccctgc 3364
ccgatccacc gtcctgaggc agagtgttga gcctcatacc tgtaccaggt ccccggccag 3424

ctgggcccct cccaggcact gccaggaagc cccagctgcc cctggcgggt gtggtggaaa 3484

tggcaggagg gtgcaggtac tcttggggcc ccagcggtgg gagtgcaaaa gacccaacgc 3544

caacacctgg tgccttttgc agccagcgcc cacccatccg tgcccggacc cttgggaatg 3604

cccgcggctc cagaggaaaa agcccaggga cggggcctcc gttgcggggg gtcggctgct 3664

tcttgggaac tttgtcgttt ccggcgctgg ctggctggct ggctgtaaag cactgaagcc 3724

ccccggccgc caacccctga aagcagaacc tggcctccct ggccacagca gccttaccca 3784

ccgctctacg tgtcccgggc acttcccgca gccttcccgt ccctttctca tcggccttgt 3844

agttgtacag tgctgttggt ttgaaaaggt gatgtgtggg gagtgcggct catcactgag 3904

tagagaggta gaatttctat ttaaccagac ctgtagtagt attaccaatc cagttcaatt 3964

aaggtgattt tctgtaatta ttattatttt ggtgggacaa tctttaatnt tnctaaagat 4024

agcactaaca tcagctcatt agccacctgt gcctgtcccc gccttggccc ggctggatga 4084

agcggcttcc ccgcagggcc cccacttccc agtggctgct tcctggggac ccagggcacc 4144

ccggcacctt caggcacgct cctcagctgg tcacctcccg gctttgccgt tcagatgggg 4204

ctcctgaggc tcaggagtga agatgccaca gagccgggct cccctaggct gcgtcgggca 4264

tgcttggaag ctggcctgcc aggaccttcc accctggggc ctgtgtcagc cgccggccct 4324

ccgcaccctg gaagcacacg gcctctggga aggacagccc tgaccttcgg ttttccgagc 4384

acggtgtttc ccaagaattc tgggctggcg gcctggtggc agtgctggag atgaccccga 4444

gcccctcccc gtggggcacc caggaggacc ctgccggaat gtgcagcctg tgggtagtcg 4504

gctggtgtcc ctgtcgtgga gctggggtgc gtgatctggt gctcgtccac gcaggtgtgt 4564

ggtgtaaaca tgtatgtgct gtacagagag acgcgtgtgg agagagccgc acaccagcgc 4624

cacccaggaa aggcggagcg gttaccagtg ttttgtgttt atttttaatc aagacgtttc 4684

ccctgttttc ctataaattt gcttcgtgta agcaagtaca taaggaccct cctttggtga 4744

aatccgggtt cgaatgaata tctcaaggca ggagatgcat ctattttaag atgctttgga 4804

gcagacagct ttagccgttc ccaatcctta gcaatgcctt agctgggacg catagctaat 4864

actttagaga ggatgacaga tccataaaga gagtaaagat aagagaaaat gtctaaagca 4924

tctggaaggg taaaaaaaaa aatctatttt tgtacaaatg taattttatc cctcatgtat 4984

acttggatat ggcgggggga gggctgggac tgtttcgttt ctgcttctag agattgaggt 5044

gaaagcttcg tccgagaaac gccaggacag acgatggcag aggagagggc tcctgtgacg 5104

gcggcgaggc ttgggaggaa accgccgcaa tgggggtgtc ttccctcggg gcaggagggt 5164

gggcctgtgg ctttcaaggg ttttcttccc tttcgagtaa tttttaaagc cttgctctgt 5224

tgtgtcctgt tgccggctct ggcctttctg tgactgactg tgaagtggct tctccgtacg 5284

attgtctctg aaacatcgtg gccgcaggtg cagggtttga tggacagtag cattagaatt 5344

gtggaaaagg aacacgcaaa gggagaagtg tgagaggaga aacaaaatat gagcgtttaa 5404

aatacatcgc cattcag 5421

<210> 6

<211> 462

<212> PRT

<213> Homo sapiens

<400> 6

Met Asp Thr Lys His Phe Leu Pro Leu Asp Phe Ser Thr Gln Val Asn

1               5                   10                  15

Ser Ser Leu Thr Ser Pro Thr Gly Arg Gly Ser Met Ala Ala Pro Ser

Thr Thr Pro Thr Leu Gly Phe Ser Thr Gly Ser Pro Gln Leu Ser Ser
85 90 95
Pro Met Asn Pro Val Ser Ser Ser Glu Asp Ile Lys Pro Pro Leu Gly
100 105 110
Leu Asn Gly Val Leu Lys Val Pro Ala His Pro Ser Gly Asn Met Ala
115 120 125
Ser Phe Thr Lys His Ile Cys Ala Ile Cys Gly Asp Arg Ser Ser Gly
130 135 140
Lys His Tyr Gly Val Tyr Ser Cys Glu Gly Cys Lys Gly Phe Phe Lys
145 150 155 160
Arg Thr Val Arg Lys Asp Leu Thr Tyr Thr Cys Arg Asp Asn Lys Asp
165 170 175
Cys Leu Ile Asp Lys Arg Gln Arg Asn Arg Cys Gln Tyr Cys Arg Tyr
180 185 190
Gln Lys Cys Leu Ala Met Gly Met Lys Arg Glu Ala Val Gln Glu Glu
195 200 205
Arg Gln Arg Gly Lys Asp Arg Asn Glu Asn Glu Val Glu Ser Thr Ser
210 215 220
Ser Ala Asn Glu Asp Met Pro Val Glu Arg Ile Leu Glu Ala Glu Leu
225 230 235 240
Ala Val Glu Pro Lys Thr Glu Thr Tyr Val Glu Ala Asn Met Gly Leu
245 250 255
Asn Pro Ser Ser Pro Asn Asp Pro Val Thr Asn Ile Cys Gln Ala Ala
260 265 270
Asp Lys Gln Leu Phe Thr Leu Val Glu Trp Ala Lys Arg Ile Pro His
275 280 285

Phe Ser Glu Leu Pro Leu Asp Asp Gln Val Ile Leu Leu Arg Ala Gly
290 295 300
Trp Asn Glu Leu Leu Ile Ala Ser Phe Ser His Arg Ser Ile Ala Val
305 310 315 320
Lys Asp Gly Ile Leu Leu Ala Thr Gly Leu His Val His Arg Asn Ser
325 330 335
Ala His Ser Ala Gly Val Gly Ala Ile Phe Asp Arg Val Leu Thr Glu
340 345 350
Leu Val Ser Lys Met Arg Asp Met Gln Met Asp Lys Thr Glu Leu Gly
355 360 365
Cys Leu Arg Ala Ile Val Leu Phe Asn Pro Asp Ser Lys Gly Leu Ser
370 375 380
Asn Pro Ala Glu Val Glu Ala Leu Arg Glu Lys Val Tyr Ala Ser Leu
385 390 395 400
Glu Ala Tyr Cys Lys His Lys Tyr Pro Glu Gln Pro Gly Arg Phe Ala
405 410 415
Lys Leu Leu Leu Arg Leu Pro Ala Leu Arg Ser Ile Gly Leu Lys Cys
420 425 430
Leu Glu His Leu Phe Phe Phe Lys Leu Ile Gly Asp Thr Pro Ile Asp
435 440 445
Thr Phe Leu Met Glu Met Leu Glu Ala Pro His Gln Met Thr
450 455 460

<210> 7
<211> 30

<212> RNA
<213> Artificial Sequence

<220>
<223> Description of Artificial Sequence:an artificially
      synthesized oligo-cap linker sequence

<400> 7
agcaucgagu cggccuuguu ggccuacugg                                        30

<210> 8
<211> 42
<212> DNA
<213> Artificial Sequence

<220>
<223> Description of Artificial Sequence:an artificially
      synthesized oligo(dT) primer sequence

<400> 8
gcggctgaag acggcctatg tggccttttt tttttttttt tt                          42

<210> 9
<211> 21

<212> DNA
<213> Artificial Sequence

<220>
<223> Description of Artificial Sequence:an artificially
      synthesized primer sequence

<400> 9
agcatcgagt cggccttgtt g                                                21

<210> 10
<211> 21
<212> DNA
<213> Artificial Sequence

<220>
<223> Description of Artificial Sequence:an artificially
      synthesized primer sequence

<400> 10
gcggctgaag acggcctatg t                                                21

<210> 11
<211> 21

<212> DNA

<213> Artificial Sequence

<220>

<223> Description of Artificial Sequence:an artificially

synthesized primer sequence

<400> 11

aacatttcct gccgctcgat t 21

<210> 12

<211> 22

<212> DNA

<213> Artificial Sequence

<220>

<223> Description of Artificial Sequence:an artificially

synthesized primer sequence

<400> 12

cctcattctc gttccggtcc tt 22

<210> 13

<211> 24

<212> DNA
<213> Artificial Sequence

<220>
<223> Description of Artificial Sequence:an artificially
      synthesized primer sequence

<400> 13
   cccttttctg tgccccctgc ctga                                              24

<210> 14
<211> 26
<212> DNA
<213> Artificial Sequence

<220>
<223> Description of Artificial Sequence:an artificially
      synthesized primer sequence

<400> 14
cttgttgtcg cggcaggtgt aggtca                                           26

<210> 15
<211> 22

<212> DNA

<213> Artificial Sequence

<220>

<223> Description of Artificial Sequence:an artificially

synthesized primer sequence

<400> 15

gggaagcgcc tgtgggtcct cg 22

<210> 16

<211> 28

<212> DNA

<213> Artificial Sequence

<220>

<223> Description of Artificial Sequence:an artificially

synthesized primer sequence

<400> 16

ggttcagccc catgtttgcc tccacgta 28

<210> 17

<211> 26

<212> DNA

<213> Artificial Sequence

<220>

<223> Description of Artificial Sequence:an artificially

synthesized sequence

<400> 17

gatctgaggt cagaggtcag agagca 26

<210> 18

<211> 26

<212> DNA

<213> Artificial Sequence

<220>

<223> Description of Artificial Sequence:an artificially

synthesized sequence

<400> 18

gatctgctct ctgacctctg acctca 26

<210> 19

<211> 27

<212> DNA
<213> Artificial Sequence

<220>
<223> Description of Artificial Sequence:an artificially
      synthesized primer sequence

<400> 19
gagaagctta gtcgcagaca tggacac                                          27

<210> 20
<211> 27
<212> DNA
<213> Artificial Sequence

<220>
<223> Description of Artificial Sequence:an artificially
      synthesized primer sequence

<400> 20
gcgtctagac aaagtccaaa caggcca                                          27

<210> 21
<211> 27

<212> DNA

<213> Artificial Sequence

<220>

<223> Description of Artificial Sequence:an artificially

synthesized primer sequence

<400> 21

cacaagcttc acctatgaac cccgtca 27

<210> 22

<211> 27

<212> DNA

<213> Artificial Sequence

<220>

<223> Description of Artificial Sequence:an artificially

synthesized primer sequence

<400> 22

cgctctagaa cacatctctt aggcaga 27

<210> 23

<211> 27

<212> DNA

<213> Artificial Sequence

<220>

<223> Description of Artificial Sequence:an artificially

synthesized primer sequence

<400> 23

cacaagcttc aagtcagggc agatttc 27

<210> 24

<211> 27

<212> DNA

<213> Artificial Sequence

<220>

<223> Description of Artificial Sequence:an artificially

synthesized primer sequence

<400> 24

gggtctagat gacaacacat ctcttag 27

## Description

### Technical Field

The present invention relates to novel nuclear receptor isoforms, and their use.

### Background Art

Nuclear receptors constitute a superfamily of a group of transcription factors whose activities are induced in a ligand-dependent manner. Nuclear receptors have specific structures, including a DNA-binding domain and a ligand-binding domain, which are highly homologous among family members.

By taking advantage of this high sequence homology, a number of novel nuclear receptor genes have been discovered. To date, forty or more nuclear receptor genes have been reported in humans. These include genes whose ligands have not yet been identified, and those that do not require a ligand to function.

Retinoid X receptor (RXR) α was discovered using homology with the DNA binding domain of retinoic acid receptor (RAR) α (Mangelsdorf D.J., Ong E.S., Dyck J.A., and Evans R.M. "Nuclear receptor that identifies a novel retinoic acid response pathway." Nature 345: 224-229 (1990)). It was later found that although RXRα comprises transcription-controlling activity alone, it can also function as a co-receptor in transcriptional activation by forming a heterodimer with other nuclear receptors, such as peroxisome proliferator activated receptor (PPAR), RAR, thyroid receptor (TR), and vitamin D3 receptor (VDR). RXRα is thought to play important roles in hormone-related physiological events (Yu V.C., Deisert C., Andersen B., Holloway J.M., Devary O.V., Naar A.M., Kim S.Y., Boutin J.M., Glass C.K., and Rosenfeld M.G. "RXR beta: a coregulator that enhances binding of retinoic acid, thyroid hormone, and vitamin D receptors to their cognate response elements." Cell 67: 1251-1266 (1991)).

With regards to RXRα ligands, 9-cis retinoic acid has been identified, in addition to retinoids, as an RXR-specific ligand (Heyman R.A., Mangelsdorf D.J., Dyck J.A., Stein R.B., Eichele G., Evans R.M, and Thaller C. "9-cis retinoic acid is a high affinity ligand for the retinoid X receptor." Cell 68: 397-406 (1992)). Furthermore, RXR-specific agonists and antagonists are being developed. Clinical application of these antagonists as anti-tumor drugs or therapeutic agents for diabetes is being considered (Mukheerjee R., Davies P.J., Crombie D.L., Bischoff E.D., Cesario R.M., Jow L., Hamann L.G., Boehm M.F., Mondon C.E., Nadzan A.M., Paterniti J.R.J. , and Heyman R.A. "Sensitization of diabetic and obese mice to insulin by retinoid X receptor agonists." Nature 386: 407-410 (1997); Wu K., Lamph W.W., and Brown P.H. "RXR-selective retinoids function independently of RAR and PPAR gamma to inhibit breast cell growth." Breast Cancer Research and Treatment. 69: 371 (2001)).

RXR is known to have three subtypes: α, β, and γ (Pemrick S.M., Lucas D.A., and Grippo J.F. "The retinoid receptors." Leukemia 8: 1797-1806 (1994)). Three isoforms of RXRβ, and two isoforms of RXRγ have been reported (Pemrick S.M., Lucas D.A., and Grippo J.F. "The retinoid receptors." Leukemia 8: 1797-1806 (1994); Mahajna J., Shi B., and Bruskin A. "A four-amino-acid insertion in the ligand-binding domain inactivates hRXRbeta and renders dominant negative activity." DNA Cell Biol. 16: 463-76 (1997)).

Three RXRα isoforms have been identified in mice (Brocaard J., Kastner P., and Chambon P. "Two novel RXR alpha isoforms from mouse testis." BBRC 229: 211-218 (1996)). However, there has been no report of RXRα isoforms in humans; cloning using mouse isoform sequence information was attempted but failed (Li G. , Walch E. , Yang X. , Lipman S.M., and Clifford J.L. "Cloning and characterization of the human retinoid X receptor alpha gene: conservation of structure with the mouse homolog." BBRC 269: 54-57 (2000)).

All except one of the known RXR isoforms comprise a structure in which the N-terminal A/B domains vary in size, (Mahajna J., Shi B. , and Bruskin A. "A four-amino-acid insertion in the ligand-binding domain inactivates hRXRbeta and renders dominant negative activity." DNA Cell Biol. 16: 463-76 (1997)). Since the A/B domains are responsible for ligand-independent transcriptional activation function, and also involved in interactions with other transcription factors, RXR isoforms are presumed to share in the transcription control of groups of genes whose expression is controlled under different physiological conditions.

Nuclear receptors are presumed to dissociate from co-repressors when a ligand binds to a ligand-binding domain. They are then presumed to recruit co-activators to form complexes that interact with RNA polymerase II complexes, initiating transcription. Recently, a number of co-repressors and co-activators have been discovered, some of which are known for their tissue-specific or physiological stimuli-specific control of expression. Interactions between nuclear receptors and co-repressors/co-activators are thought to play important roles in the diverse transcription-controlling functions of nuclear receptors (Freedman L.P. "Increasing the complexity of coactivation in nuclear receptor signaling." Cell 97: 5-8 (1999)).

### Disclosure of the Invention

An objective of the present invention is to provide novel RXRα isoforms whose transcriptional activation function is enhanced by co-activators that differ from those that enhance the effect of known RXRα isoforms. In addition, another objective of the invention is to provide novel methods for evaluating the function of regulating RXRα transcription control activity, based on the functions of the novel isoforms.

The present inventors attempted to discover novel nuclear receptors by searching for genes that encode proteins comprising a nuclear receptor specific DNA binding domain (DBD) and ligand-binding domain (LBD). They succeeded in isolating novel RXRα isoforms. Furthermore, the present inventors analyzed the functions of the genes narrowed down by this search. As a result, they found that some RXRα isoforms exhibited transcriptional activation function due to the action of co-activators different to those of known RXRα isoforms.

The present inventors then established an assay system for evaluating the transcription-controlling activity of the RXRα isoforms in the presence of co-activators that activate nuclear receptors. Further, the present inventors used this system to confirm that the effects of test substances on the transcription-controlling activity of RXRα can be specifically evaluated for each isoform. Thus, they accomplished the present invention.

Hence, the present invention relates to the polynucleotides below, and the proteins encoded by these polynucleotides. It also relates to methods for evaluating the effects of test substances on the transcription-controlling activity of each nuclear receptor RXRα isoform, in the presence of co-activators. Furthermore, the invention relates to methods, based on these evaluation methods, of screening for test substances that can regulate the transcription-controlling activity of RXRα isoforms.
[1] A polynucleotide of any one of the following (A) to (E):
   (A) a polynucleotide comprising the coding region of the nucleotide sequence of SEQ ID NO: 1 or 3;
   (B) a polynucleotide encoding a protein comprising the amino acid sequence of SEQ ID NO: 2 or 4;
   (C) a polynucleotide encoding a protein comprising the amino acid sequence wherein one or more amino acids are replaced, deleted, inserted, and/or added to the amino acid sequence of SEQ ID NO: 2 or 4, encoding a protein comprising the following (a) and (b):
      (a) The transcriptional activation function is enhanced in response to binding to the ligand retinoic acid, or its agonist; and
      (b) The protein requires SRC-1 as a co-activator;
   (D) a polynucleotide that hybridizes under stringent conditions with a polynucleotide comprising the nucleotide sequence of SEQ ID NO: 1 or 3, encoding a protein comprising the following (a) and (b):
      (a) The transcriptional activation function is enhanced in response to binding to the ligand retinoic acid, or its agonist; and
      (b) The protein requires SRC-1 as a co-activator;
   (E) a polynucleotide comprising a nucleotide sequence in which the nucleotide sequence from 1 to 102 of SEQ ID NO: 5 is deleted, or replaced with another nucleotide sequence, where the sequence has 70% or more homology to the nucleotide sequence from 103 to 1461 of SEQ ID NO: 5;
[2] a protein encoded by the polynucleotide of [1];
[3] a vector comprising the polynucleotide of [1];
[4] a transformant maintaining the polynucleotide of [1]; or the vector of [3];
[5] a method for producing the protein of [2], comprising the steps of culturing the transformant of [4], and recovering the expressed product;
[6] a method for detecting the activity of a test substance that regulates the transcription-controlling activity of a nuclear receptor, wherein the nuclear receptor is a protein encoded by the polynucleotide of any one of the following (A) to (E):
   (A) a polynucleotide comprising the coding region of the nucleotide sequence of SEQ ID NO: 1 or 3;
   (B) a polynucleotide encoding a protein comprising the amino acid sequence of SEQ ID NO: 2 or 4;
   (C) a polynucleotide encoding a protein comprising an amino acid sequence wherein one or more amino acids are replaced, deleted, inserted, and/or added to the amino acid sequence of SEQ ID NO: 2 or 4, where the protein comprises the following (a) and (b):
      (a) The transcriptional activation function is enhanced in response to binding to the ligand retinoic acid, or its agonist; and
      (b) The protein requires SRC-1 as a co-activator;
   (D) a polynucleotide that hybridizes under stringent conditions with a polynucleotide comprising the nucleotide sequence of SEQ ID NO: 1 or 3, encoding a protein comprising the following (a) and (b):
      (a) The transcriptional activation function is enhanced in response to binding to the ligand retinoic acid, or its agonist; and
      (b) The protein requires SRC-1 as a co-activator;
   (E) a polynucleotide comprising a nucleotide sequence in which the nucleotide sequence from 1 to 102 of SEQ ID NO: 5 is deleted, or replaced with another nucleotide sequence, and which comprises 70% or more homology to the nucleotide sequence from 103 to 1461 of SEQ ID NO: 5;
   wherein the method comprises the following steps:
   (1) contacting a test substance with cells, which express a nuclear receptor and comprise an expression cassette in which a reporter gene is inserted downstream of a responsive element for the nuclear receptor, in the presence of a co-activator that activates the nuclear receptor;
   (2) culturing the cells under conditions enabling the nuclear receptor expression, and measuring the expression level of the reporter gene within the cells; and
   (3) detecting the activity of the test substance regulating the transcription-controlling activity of the nuclear receptor, using the measurement result of step (2) as an index;
[7] the method of [6], wherein the method additionally comprises the step of detecting the activity of a test substance on a nuclear receptor that is a protein encoded by the polynucleotide of the following (A') to (E'):
   (A') a polynucleotide comprising the coding region of the nucleotide sequence of SEQ ID NO: 5;
   (B') a polynucleotide encoding a protein comprising the amino acid sequence of SEQ ID NO: 6;
   (C') a polynucleotide encoding a protein comprising an amino acid sequence wherein one or more amino acids are replaced, deleted, inserted, and/or added to the amino acid sequence of SEQ ID NO: 6, wherein the protein is functionally equivalent to a protein comprising the amino acid sequence of SEQ ID NO: 6;
   (D') a polynucleotide that hybridizes with a polynucleotide comprising the nucleotide sequence of SEQ ID NO: 5 under stringent conditions, encoding a protein functionally equivalent to a protein comprising the amino acid sequence of SEQ ID NO: 6; and
   (E') a polynucleotide comprising 60% or more homology to the nucleotide sequence of SEQ ID NO: 5, encoding a protein functionally equivalent to a protein comprising the amino acid sequence of SEQ ID NO: 6;
[8] a method for detecting the activity of a test substance controlling the binding between a nuclear receptor and a co-activator that activates the receptor, wherein the nuclear receptor is a protein encoded by the polynucleotide of any one of the following (A) to (E) :
   (A) a polynucleotide comprising the coding region of the nucleotide sequence of SEQ ID NO: 1 or 3;
   (B) a polynucleotide encoding a protein comprising the amino acid sequence of SEQ ID NO: 2 or 4;
   (C) a polynucleotide encoding a protein comprising an amino acid sequence wherein one or more amino acids are replaced, deleted, inserted, and/or added to the amino acid sequence of SEQ ID NO: 2 or 4, encoding a protein comprising the following (a) and (b):
      (a) The transcriptional activation function is enhanced in response to binding to the ligand retinoic acid, or its agonist; and
      (b) The protein requires SRC-1 as a co-activator;
   (D) a polynucleotide that hybridizes with a polynucleotide comprising the nucleotide sequence of SEQ ID NO: 1 or 3 under stringent conditions, encoding a protein comprising the following (a) and (b):
      (a) The transcriptional activation function is enhanced in response to binding to the ligand retinoic acid, or its agonist; and
      (b) The protein requires SRC-1 as a co-activator;
   (E) a polynucleotide comprising a nucleotide sequence in which the nucleotide sequence from 1 to 102 of SEQ ID NO: 5 is deleted, or replaced with another nucleotide sequence, and which comprises 70% or more homology to the nucleotide sequence from 103 to 1461 of SEQ ID NO: 5;
   wherein the method comprises the following steps:
   (1) contacting the nuclear receptor, SRC-1, and the test substance in any of the following orders i) to iii):
      i) contacting the nuclear receptor and the test substance first, and then contacting with SRC-1;
      ii) contacting the nuclear receptor and SRC-1 in the presence of the test substance; or
      iii) contacting the nuclear receptor and SRC-1 first, and then contacting with the test substance;
   (2) measuring the amount of SRC-1 or test substance bound to the nuclear receptor; and
   (3) detecting the activity of the test substance in binding to the nuclear receptor, using the measurement result of (2) as an index;
[9] a method for evaluating a substance capable of regulating the transcription-controlling activity of a nuclear receptor, comprising the following steps:
   (1) detecting the activity of a test substance in regulating the transcription-controlling activity of a nuclear receptor using the method of [6]; and
   (2) selecting a test substance capable of inhibiting or enhancing the transcriptional activation function of the nuclear receptor by comparing with control;
[10] a method for evaluating a test substance capable of regulating the transcription-controlling activity of a specific nuclear receptor isoform, where the method comprises the following steps:
   (1) detecting the activity of a test substance in regulating the transcription-controlling activity of different kinds of nuclear receptors that comprise a protein encoded by the polynucleotide of any one of [6] (A) to (E) , using the method of [6];
   (2) comparing the activity of a test substance in regulating the transcription-controlling activity of each of the different kinds of nuclear receptor; and
   (3) selecting a test substance whose effect on transcription-controlling activity differs between isoforms;
[11] a method for evaluating a test substance capable of regulating the transcription-controlling activity of a nuclear receptor, comprising the following steps:
   (1) detecting the activity of the test substance in controlling the binding between the nuclear receptor and a co-activator activating it, using the method of [8], and
   (2) selecting, by comparison with a control, a test substance capable of inhibiting or enhancing the binding of the co-activator to the nuclear receptor;
[12] an agent for controlling the activity of a nuclear receptor, comprising as an active ingredient a substance selected by the method of [9];
[13] an agent for controlling the activity of a nuclear receptor in an isoform-specific manner, comprising as an active ingredient a substance selected by the method of [10];
[14] an agent for controlling the activity of a nuclear receptor, comprising as an active ingredient a substance selected by the method of [11];
[15] an agent for controlling the activity of a nuclear receptor, comprising as the main ingredient a polynucleotide of any one of the following (A) to (E), or a protein encoded by the polynucleotide,
   (A) a polynucleotide comprising the coding region of the nucleotide sequence of SEQ ID NO: 1 or 3;
   (B) a polynucleotide encoding the amino acid sequence of SEQ ID NO: 2 or 4;
   (C) a polynucleotide encoding a protein comprising the amino acid sequence wherein one or more amino acids are replaced, deleted, inserted, and/or added to the amino acid sequence of SEQ ID NO: 2 or 4, encoding a protein comprising the following (a) and (b):
      (a) The transcriptional activation function is enhanced in response to binding to the ligand retinoic acid, or its agonist; and
      (b) The protein requires SRC-1 as a co-activator;
   (D) a polynucleotide that hybridizes under stringent conditions with a polynucleotide comprising the nucleotide sequence of SEQ ID NO: 1 or 3, encoding a protein comprising the following (a) and (b):
      (a) The transcriptional activation function is enhanced in response to binding to the ligand retinoic acid, or its agonist; and
      (b) The protein requires SRC-1 as a co-activator;
   (E) a polynucleotide comprising the nucleotide sequence in which the nucleotide sequence from 1 to 102 of SEQ ID NO: 5 is deleted, or replaced with another nucleotide sequence, and which comprises 70% or more homology to the nucleotide sequence from 103 to 1461 of SEQ ID NO: 5;
[16] an agent for suppressing the activity of a nuclear receptor, comprising as an active ingredient a component of any one of the following (1) to (4);
   (1) an antisense polynucleotide of the polynucleotide of the above (A) to (E);
   (2) an antibody that recognizes a protein encoded by the polynucleotide of the above (A) to (E);
   (3) a protein exerting a dominant-negative effect against a protein encoded by the polynucleotide of the above (A) to (E) ; and
   (4) a double-stranded RNA that comprises 21 to 23 base pairs, comprising a sense RNA corresponding to a partial sequence of the nucleotide sequence of the polynucleotide of the above (A) to (E) , and its antisense RNA;
[17] a model animal wherein the activity of a nuclear receptor is controlled, wherein the animal is a transgenic non-human animal in which the expression of a protein encoded by the polynucleotide of any one of the following (A) to (E) is controlled,
   (A) a polynucleotide comprising the coding region of the nucleotide sequence of SEQ ID NO: 1 or 3;
   (B) a polynucleotide encoding the amino acid sequence of SEQ ID NO: 2 or 4;
   (C) a polynucleotide encoding a protein comprising an amino acid sequence wherein one or more amino acids are replaced, deleted, inserted, and/or added to the amino acid sequence of SEQ ID NO: 2 or 4, encoding a protein comprising the following (a) and (b):
      (a) The transcriptional activation function is enhanced in response to binding to the ligand retinoic acid, or its agonist; and
      (b) The protein requires SRC-1 as a co-activator;
   (D) a polynucleotide that hybridizes with a polynucleotide comprising the coding region of the nucleotide sequence of SEQ ID NO: 1 or 3 under stringent conditions, encoding a protein comprising the following (a) and (b):
      (a) The transcriptional activation function is enhanced in response to binding to the ligand retinoic acid, or its agonist; and
      (b) The protein requires SRC-1 as a co-activator;
   (E) a polynucleotide comprising a nucleotide sequence in which the nucleotide sequence from 1 to 102 of SEQ ID NO: 5 is deleted, or replaced with another nucleotide sequence, and which comprises 70% or more homology to the nucleotide sequence from 103 to 1461 of SEQ ID NO: 5; and
[18] a method of diagnosing a disease caused by the abnormal activity of a nuclear receptor, comprising the steps of measuring the expression level of a polynucleotide in a biological sample collected from a subject, and judging that, if the expression level is low compared with a control, the subject has a disease resulting from impaired function of a protein encoded by the polynucleotide, and that, in contrast, if the expression level is high, the subject has a disease resulting from elevated function of the protein, wherein the polynucleotide is any one of the following (A) to (E):
   (A) a polynucleotide comprising the coding region of the nucleotide sequence of SEQ ID NO: 1 or 3;
   (B) a polynucleotide encoding the amino acid sequence of SEQ ID NO: 2 or 4;
   (C) a polynucleotide encoding a protein comprising an amino acid sequence wherein one or more amino acids are replaced, deleted, inserted, and/or added to the amino acid sequence of SEQ ID NO: 2 or 4, encoding a protein comprising the following (a) and (b):
      (a) The transcriptional activation function is enhanced in response to binding to the ligand retinoic acid, or its agonist; and
      (b) The protein requires SRC-1 as a co-activator;
   (D) a polynucleotide that hybridizes under stringent conditions with a polynucleotide comprising the coding region of the nucleotide sequence of SEQ ID NO: 1 or 3, encoding a protein comprising the following (a) and (b):
      (a) The transcriptional activation function is enhanced in response to binding to the ligand retinoic acid, or its agonist; and
      (b) The protein requires SRC-1 as a co-activator;
   (E) a polynucleotide comprising a nucleotide sequence in which the nucleotide sequence from 1 to 102 of SEQ ID NO: 5 is deleted, or replaced with another nucleotide sequence, and which comprises 70% or more homology to the nucleotide sequence from 103 to 1461 of SEQ ID NO: 5.

To discover novel nuclear receptors, the present inventors targeted full-length cDNA libraries, searching for genes that encode proteins comprising a DNA binding domain (DBD) and a ligand-binding domain (LBD) that are specific for a nuclear receptor. Of the genes screened, the present inventors focused on C-BNGH42008649, and C-BRAMY2003287 , which were confirmed to be highly homologous to RXRα.

C-BNGH42008649 is a cDNA of 2393 base pairs, comprising a coding sequence (923-2230) that corresponds to a sequence of 435 amino acids. Compared to the known RXRα1 isoform, C-BNGH42008649 lacks 27 N-terminal amino acids. Based on structural features, C-BNGH42008649 was judged a novel RXRα isoform, and named human RXRα2.

C-BRAMY2003287 is a cDNA of 2421 base pairs comprising a coding sequence (924-2021) that corresponds to a sequence of 365 amino acids. Comparing to known RXRα1 isoforms, it lacks 97 N-terminal amino acids. Based on structural features, C-BNGH42008649 was judged a novel RXRα isoform, and named human RXRα3.

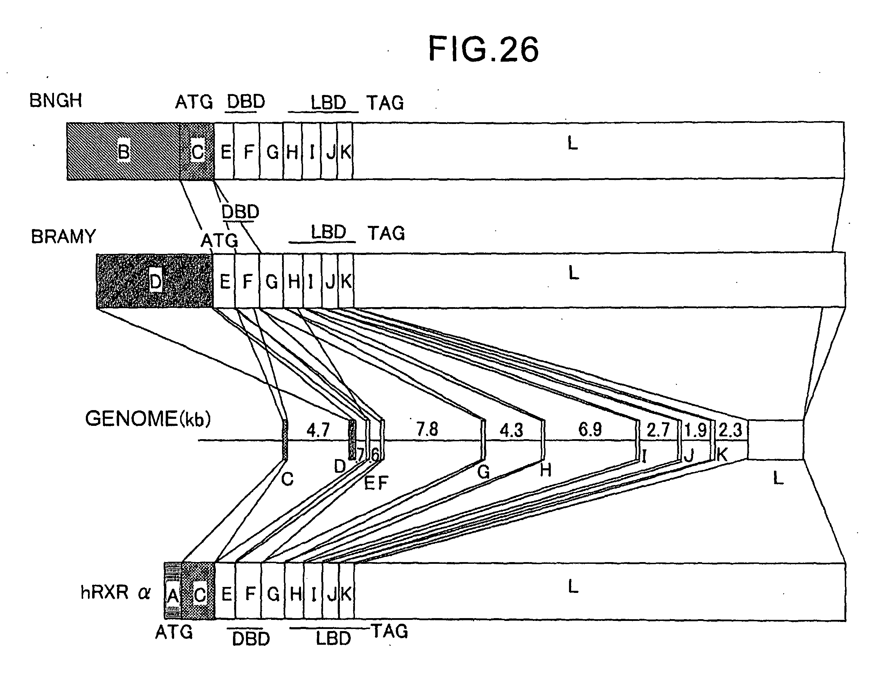

The nucleotide sequences of these isoforms were searched against the human genomic sequence database using BLAST homology, and were found to match the sequence of a clone of human chromosome 9q34.3. RXRα3 and RXRα2 were revealed to comprise 9 and 10 exons respectively. Similar homology searches were performed for RXRα1, and the three were compared with each other. In comparison with RXRα1, RXRα2 uses common exons as for the downstream exons from the exon 2, but uses a different exon as for its 5'-most exon. In comparison with RXRα1, RXRα3 uses common exons as for the downstream exons from the exon 3, but uses a different exon as for its 5'-most exon (Fig. 26).

The present invention relates to a polynucleotide encoding RXRα2, comprising the coding region of the nucleotide sequence of SEQ ID NO: 1; and a polynucleotide encoding a protein comprising the amino acid sequence of SEQ ID NO: 2. The invention also relates to a polynucleotide encoding RXRα3, comprising the coding region of the nucleotide sequence of SEQ ID NO: 3; and a polynucleotide encoding a protein comprising the amino acid sequence of SEQ ID NO: 4.

The present invention also comprises a polynucleotide encoding a protein functionally equivalent to a protein comprising the amino acid sequence of SEQ ID NO: 2 or 4. Herein, a protein is defined as being functionally equivalent to the RXRα2 or RXRα3 proteins of the present invention if the protein of interest comprises the following (a) and (b):
(a) The transcriptional activation function is enhanced in response to binding to the ligand retinoic acid, or its agonist, and
(b) The protein requires SRC-1 as a co-activator.

For example, the present invention comprises a polynucleotide encoding a protein which comprises the aforementioned (a) and (b), and which is encoded by the following polynucleotide:
(C) a polynucleotide encoding a protein comprising an amino acid sequence wherein one or more amino acids are replaced, deleted, inserted, and/or added to the amino acid sequence of SEQ ID NO: 2 or 4;
(D) a polynucleotide that hybridizes under stringent conditions with a polynucleotide comprising the nucleotide sequence of SEQ ID NO: 1 or 3; and
(E) a polynucleotide comprising a nucleotide sequence in which the nucleotide sequence from 1 to 102 of SEQ ID NO: 5 is deleted, or replaced with another nucleotide sequence, and which comprises 70% or more homology to the nucleotide sequence from 103 to 1461 of SEQ ID NO: 5.

Herein, increased transcriptional activation function of a protein due to binding with the ligand retinoic acid, or its agonist, can be examined as follows: In the following explanation, proteins to be examined for functional equivalence are described as test proteins. For example, a reporter gene assay may be used to examine the aforementioned attribute (a). Specifically, a test protein examined for attribute (a) is expressed in cells. The cells used as hosts are introduced with an expression vector for a reporter gene, under the regulation of a responsive element for the test protein. RXR responsive elements are already known.

If the test protein comprises attribute (a) , treating the cells with the ligand retinoic acid or its agonist will lead to an increase in reporter gene expression level. Therefore, based on a comparison of reporter gene expression levels before and after ligand treatment, a test protein can be determined to comprise attribute (a).

Attribute (b), the use of SRC-1 as a co-activator, is another index for functional equivalence in the present invention. As for attribute (a), it can also be confirmed by reporter gene assay. As described above, host cells are introduced with a vector for expressing a reporter gene under the control of a test protein responsive element. If reporter gene expression level in the presence of SRC-1 is elevated in response to a ligand, the test protein may be confirmed to use SRC-1 as a co-activator.

In general, co-activators are molecules required for the expression of a nuclear receptor's transcriptional activation function. Ligand-responsive nuclear receptors exhibit transcriptional activation function in response to a ligand in the presence of a co-activator. While nuclear receptors exhibit transcriptional activation function in response to the amount of a ligand, co-activators have a qualitative effect. Thus, while the presence of a certain amount of co-activator is required for transcriptional activation function, changing the amount of co-activator has little effect.

Herein, a nuclear receptor's transcription-controlling activity means a nuclear receptor's activity in initiating gene transcription via a transcriptional unit. In general, nuclear receptors are considered to initiate transcription by recruiting multiple factors called transcriptional cofactors. Transcriptional activation brought by the transcription-controlling activity of a nuclear receptor is called transcriptional activation function.

Proteins functionally equivalent to the nuclear receptors RXRα2 or RXRα3 of the present invention require SRC-1 as a co-activator. In a more preferable embodiment, and similar to known isoform RXRα1, the proteins functionally equivalent to the nuclear receptors of the present invention comprise a transcriptional activation function that is also elevated in response to a ligand in the presence of CBP. Thus, the transcriptional activation function can be also achieved by co-activator CBP.

Proteins functionally equivalent to the proteins identified in the examples of the present invention can be prepared by those skilled in the art, for example, by using a method of introducing mutations to the amino acid sequence of a protein. An example of such method for introducing mutations is the method of site-directed mutagenesis (Current Protocols in Molecular Biology, edit. Ausubel et al., Publish. John Wiley & Sons, Section 8.1-8.5 (1987)). In addition, this kind of protein may also be generated as a result of naturally occurring amino acid mutations. The present invention comprises proteins wherein one or more amino acids are replaced, deleted, inserted, and/or added to the amino acid sequence of SEQ ID NO: 2 or 4, as long as these proteins are functionally equivalent to the proteins identified in the examples of the present invention.

The number of mutated amino acids in a protein, or the site of mutation, is not limited, as long as the protein retains its function. Typically, the number of mutated amino acids is 10% or less of the total number of the amino acids in a protein sequence, preferably 5% or less, and more preferably 1% or less. To maintain protein function, amino acids are preferably replaced with amino acids comprising similar characteristics. For example, since Ala, Val, Leu, Ile, Pro, Met, Phe and Trp are all grouped into non-polar amino acids, they are considered to comprise similar characteristics. Uncharged amino acids include Gly, Ser, Thr, Cys, Tyr, Asn and Gln. Acidic amino acids include Asp and Glu, and basic amino acids include Lys, Arg and His.

Alternatively, proteins functionally equivalent to the proteins of the present invention can be isolated using techniques known to those skilled in the art, such as hybridization or gene amplification. Those skilled in the art can conventionally carry out hybridization (Current Protocols in Molecular Biology, edit. Ausubel et al., Publish. John Wiley & Sons, Section 6.3-6.4 (1987)) using the nucleotide sequence of SEQ ID NO: 1 or 3, which encode the proteins of the present invention, or their partial sequences, to isolate polynucleotides comprising high homology to these nucleotides and obtain functionally equivalent proteins. The present invention comprises proteins encoded by polynucleotides that hybridize with polynucleotides encoding the proteins of the present invention, as long as these proteins are functionally equivalent.

Stringent hybridization conditions for isolating polynucleotides that encode functionally equivalent proteins are normally washing in "1x SSC, 0.1% SDS at 37°C". More stringent conditions are "0.5x SSC, 0.1% SDS at 42°C", and the most stringent conditions are "0.1x SSC, 0.1% SDS at 65°C". By increasing the stringency of the conditions for hybridization, one can expect to isolate DNAs comprising greater homology to the probe sequence. While the above sets of SSC, SDS, and temperature conditions are given as examples, one skilled in the art can readily achieve stringencies similar to the above by appropriately combining these or other conditions that determine hybridization stringency. The other conditions influencing stringency include probe concentration, probe length, and incubation time for hybridization.

The amino acid sequences of the proteins isolated using the above hybridization, or the nucleotide sequences encoding these proteins, normally comprise high homology with the proteins of the present invention of SEQ ID NO: 2 or 4. High homology means sequence identity of at least 60% or higher, preferably 70% or higher, and more preferably 80% or higher (for example, 90% or higher). Homology can be determined using BLAST2 homology search algorithm (Altschul, S.F. et al. "Gapped BLAST and PSI-BLAST: a new generation of protein database search programs." Nucleic Acids Res. 25: 3389-3402 (1997)).

The polynucleotides of the present invention comprise polynucleotides with 70% or more homology to the nucleotide sequence from 103 to 1461 of SEQ ID NO: 5, and a nucleotide sequence such that the nucleotide sequence from 1 to 102 of SEQ ID NO: 5 is deleted, or replaced with another sequence.

The nucleotide sequence described in SEQ ID NO: 5 is that of the known RXRα1 isoform. The sequence from 1 to 102 corresponds to exon A of RXRα1. Herein, any nucleotide sequence can replace the exon A nucleotide sequence. For example, the replaceable nucleotide sequence may be a sequence for an untranslated region. However, the nucleotide sequence for exon A comprises an initiation codon at a position from 76 to 78. Thus, when this region is deleted or replaced, a novel initiation codon must be added to the region corresponding to the nucleotide sequence starting from 103.

The initiation codons of the polynucleotides of the present invention can be, for example, 'atg' starting with 'a' at 157 in SEQ ID NO: 5. This initiation codon corresponds to that for the desired nucleotide sequence of SEQ ID NO: 1 of the present invention, as described later. The initiation codon in the nucleotide sequence of SEQ ID NO: 1 is the first 'atg' in the nucleotide sequence corresponding to the exon, adjacent to exon A. Thus, the first initiation codon utilized when exon A is deleted can be presented as an example of a desired initiation codon for the polynucleotides of the present invention.

The initiation codon in the nucleotide sequence of SEQ ID NO: 3 can be also presented as such an example of an initiation codon for the polynucleotides of the present invention. The initiation codon at 924 of SEQ ID NO: 3 corresponds to the initiation codon at 367 of SEQ ID NO: 5, the first one appearing in the third exon counting from the 5' -terminus. Thus , it is not necessary to actively introduce an initiation codon after deleting or replacing exon A, the sequence from 1 to 102 of SEQ ID NO: 5. The first initiation codon found in the adjacent exon can be used for translation initiation.

Proteins encoded by polynucleotides that comprise the replaced nucleotide sequence can be confirmed to comprise the above attributes (a) and (b) by using the aforementioned methods. Methods for deleting a particular region of a polynucleotide that comprises a certain nucleotide sequence, or replacing a region with another nucleotide sequence, are known to those skilled in the art. For example, the nucleotide sequence of SEQ ID NO: 1 or 3 is a desired polynucleotide of the present invention.

As shown in Fig. 26, the nucleotide sequence (C-BNGH42008649) of SEQ ID NO: 1 comprises exon B in place of exon A in SEQ ID NO: 5 (RXRα1). The nucleotide sequence (C-BRAMY2003287) of SEQ ID NO: 3 comprises exon D in place of exons A and C in SEQ ID NO: 5 (RXRα1). Accordingly, compared to SEQ ID: 6 (RXRα1), the amino acid sequences encoded by polynucleotides RXRα2, and RXRα3 are missing 27 and 97 N-terminal amino acids respectively.

In addition, proteins functionally equivalent to the proteins of the present invention can be obtained by performing PCR (Current protocols in Molecular Biology, edit. Ausubel et al. , Publish. John Wiley & Sons, Section 6.1-6.4 (1987)) using primers designed based on the parts of the nucleotide sequences identified in the Examples of the present invention (SEQ ID NO: 1 or 3), and isolating DNA fragments comprising high homology to the nucleotide sequences or their parts. The proteins of the present invention, and functionally equivalent proteins, can be used in the methods for evaluating a compound that modifies a protein's function, as described below. In addition, the polynucleotides encoding these proteins are useful for producing the proteins, or as primers or probes for examining expression level.

The present invention also relates to polynucleotides that encode proteins comprising a dominant negative form against the proteins of the present invention. When expressed, the polynucleotide encoding a protein capable of conferring a dominant negative effect exerts the function of eliminating or reducing the activity of endogenous wild type proteins originally contained in cells. For example, an inactive nuclear receptor protein, without transcriptional activation function, can be obtained by altering the part of an amino acid sequence of a protein of the present invention that corresponds to a region considered to influence its activity, such as a region required for interaction with another nuclear receptor, or a ligand binding domain. By expressing in cells a gene that encodes such an amino acid sequence, the expression of RXRα2 or RXRα3 of the present invention can be suppressed by dominant negative effect (competitive inhibition by an inactive form). Thus, the activity of a nuclear receptor can be controlled by using gene transfection technologies such as viral vectors to introduce cells with a gene that encodes the inactive form.

The present invention also provides expression vectors comprising any of the above polynucleotides. In addition, the present invention relates to transformants carrying the above polynucleotides, or any of the above expression vectors, and methods of producing the proteins of the present invention or their partial peptides, which comprise culturing the transformant and isolating a protein of the present invention from the culture. Moreover, the present invention provides proteins produced by the above methods, or their partial peptides.

Those skilled in the art know that in using recombinant technology to produce polypeptides, the desired polypeptides can be obtained with different types or degrees of glycosylation, depending on the type of host cell. Those skilled in the art also know that when producing desired polypeptides as secreted proteins, the proteins comprise different kinds of terminal amino acid sequences (N- and/or C-termini), since the precursor polypeptides expressed in host cells undergo processing by signal peptidases and such. Therefore, those skilled in the art should easily understand that these polypeptides are comprised in the scope of the proteins of the present invention.

The Examples below illustrate an example of the construction of an expression vector capable of functioning in mammalian cells. On disclosure of a DNA encoding a protein of the present invention herein, those skilled in the art can readily construct expression vectors capable of expressing and producing the protein when introduced into host cells, including fungi such as yeast, and prokaryotic cells. Therefore, the present invention comprises expression vectors that can be constructed using methods known in the art, based on the nucleotide sequences of the present invention.

Microorganisms that can be used for expressing the polynucleotides that encode the proteins of the present invention include prokaryotic bacteria such as *Escherichia coli* and *Bacillus subtilis,* and eukaryotic yeast such as *Saccharomyces cerevisiae*. Mammalian cells include tissue-cultured human cells, and cultured animal cells. Furthermore, cultured plant cells may be used.

Examples of microorganism host cells are bacteria belonging to *Escherichia*, and *Saccharomyces cerevisiae*. More specifically, the following strains are examples of microorganism hosts:

Bacteria belonging to *Escherichia*:
E.coli HB101 (ATCC 33694)
*E.coli* HB101-16 (FERM BP-1872)
*E.coli* MM294 (ATCC 31446)
*E.coli* DH1 (ATCC 33849)

Yeast:
*S.cerevisiae* AH22 (ATCC 38626)

Examples of mammalian host cells include human embryonic kidney-derived HEK293 cells, mouse L929 cells, and Chinese hamster ovary (CHO) cells.

Normally, when the host cells are prokaryotic bacteria cells, particularly *E.coli*, the expression vectors comprise at least a promoter, an initiation codon, a polynucleotide encoding an amino acid sequence of a protein of the present invention, a termination codon, and an autonomously replicable unit. When used in eukaryotic host cells such as yeast and mammalian cells, the expression vector preferably comprises at least a promoter, an initiation codon, a polynucleotide encoding an amino acid sequence of a protein of the present invention, and a termination codon. It may further comprise insertion of an enhancer sequence, 5'- and 3'-untranslated regions of the protein of the present invention, a polyadenylation site, and an autonomously replicable unit.

The above autonomously replicable units preferably comprise a transformant selection marker (e.g. ampicillin resistance). In an expression vector for a microorganism host cells, a promoter means a promoter/operator region, comprising a promoter, operator, and Shine-Dalgarno (SD) sequence (e.g. AAGG). Conventional promoter operator region are examples of such promoters. Specifically, the lactose operon, PL-promoter, trp-promoter, or such can be used. An example of an expression vector for use in yeast host cells is the pho5 promoter. In addition, basic amino acids with affinity for metal ion chelates may be added to either end of the proteins of the present invention to facilitate purification. When adding basic amino acids, oligopeptides can be added to an arbitrary end of a gene of interest by performing PCR using a primer to which is attached at the 5'-end a sequence comprising a consecutive nucleotide sequence encoding the desired amino acids. Basic amino acids include histidine, lysine or arginine.

Promoters used in expression vectors in mammalian cells include the HTLV-LTR promoter, SV40 early and late promoters, CMV promoter, and mouse metallothionein-I (MMT) promoter. Preferable initiation codons include the methionine codon (ATG).

The termination codons include conventional termination codons (e.g. TAG, TGA and such). Autonomously replicable units are DNA sequences capable of autonomously replicating the entire DNA of the expression vector that comprises them within a host cell, and these include natural plasmids, artificially modified plasmids, and synthetic plasmids. Artificially modified plasmids include DNA fragments prepared from natural plasmids. When using *E. coli* as a host, preferred examples of such desired plasmids include plasmid pBR322 and its artificial modifications. The artificially modified plasmids include DNA fragments obtained by treating pBR322 with an appropriate restriction enzyme. When using yeast host cells, pJDB219, and pJDB207 may be used as examples. Further, plasmids used in animal cells include plasmid pcDNA3.1 (Invitrogen) , pMM324, pSV2dhfr (ATCC 37145), pdBPV-MMTneo (ATCC 37224), and pSV2neo (ATCC 37149).

An enhancer sequence may be the SV40 enhancer sequence, for example. The polyadenylation sites include the SV40 polyadenylation site.

Polynucleotides encoding the amino acid sequences of the proteins of the present invention are obtainable by synthesizing the entire or partial sequence using a DNA synthesizer, for example. Alternatively, they can be obtained from human cDNA libraries by using probes or primers whose design is based on the nucleotide sequence of SEQ ID NO: 1 or 3. Furthermore, genomic DNAs that encode the proteins of the present invention may be prepared by processing genomic DNAs of standard methods. For example, genomic DNAs may be processed by methods that comprise the steps of digestion with restriction enzymes, dephosphorylation with bacterial alkaline phosphatase, phosphorylation with T4 polynucleotide kinase, and ligation with T4 DNA ligase. Furthermore, genomic DNAs obtained as above can be used to identify transcription initiation sites for the genes of the present invention that occur in the genome, and to specify the expression regulatory regions located further upstream. Regulatory regions, such as promoters and enhancers that regulate the expression of genes encoding the proteins of the present invention, are useful as target regions for detecting abnormal protein expression. Alternatively, for example, decoy nucleic acid medicines targeted at such regions may be used to achieve expression regulation.

Methods known to those skilled in art or in the literature may be used to perform those procedures required to implement the present invention, such as DNA cloning, construction of each plasmid, transfection into host cells, culture of transformants, and recovery of proteins from the culture (Molecular Cloning, 2nd. edition, Sambrook J. et al., Cold Spring Harbor Laboratory (1989) ; DNA Cloning, Glover D.M., IRL PRESS (1985); Molecular Cloning, 3rd. edition, Sambrook J. et al., Cold Spring Harbor Laboratory (2001)).

The host cells of the present invention also comprise cells used for the functional analysis of RXRα2 or RXRα3 of the present invention, or in the methods for evaluating function-inhibiting and function-enhancing agents which use these proteins. Vector transfection into host cells may be performed by calcium phosphate precipitation, electroporation (Current protocols in Molecular Biology, edit. Ausubel et al., Publish. John Wiley & Sons., Section 9.1-9.9 (1987)), by using LipofectAMINE (Gibco BRL) , microinj ection, or such. The proteins of the present invention may be prepared from transformants using methods of protein separation and purification commonly known to those skilled in the art. The present invention relates to substantially pure proteins that are purified as above. Herein, "substantially pure" means free from contamination with other human-derived proteins. Alternatively, substantially pure proteins of the present invention are proteins comprising a purity of, for example, 80% or more, normally 90% or more, 95% or more, preferably 98% or more, and more preferably 99% or more.

The present invention also provides polynucleotides comprising a nucleotide sequence of SEQ ID NO: 1 or 3, or a polynucleotide comprising at least 15 nucleotides that are complementary to the complementary strand of the polynucleotide. Herein, the "complementary strand" means the other strand to one strand of a double-stranded polynucleotide comprising A:T (A:U) and G:C base pairs. In addition, "complementary" refers not only to complete complementarity to a region of at least 15 consecutive nucleotides, but also to homology of at least 70% or more, preferably at least 80% or more, more preferably 90% or more, and further preferably 95% or more. Homology may be determined by the algorithms described in the present invention.

Such polynucleotides may be used as probes for detecting or isolating DNAs or RNAs that encode the proteins of the present invention, or as primers for amplifying the polynucleotides of the present invention. When used a primer, the polynucleotide length is normally from 15 to 100 base pairs , and preferably from 15 to 35 base pairs. In addition, when used as a probe, the polynucleotides comprise at least a part or an entire sequence of a polynucleotide of the present invention, and comprise at least 15 base pairs. When used as a primer, the 3'-region must be complementary, but a recognition site for a restriction enzyme, tag or such can be added to the 5'-region.

In particular, regions within the nucleotide sequence of SEQ ID NO: 1 or 3, and which comprise exons that are not shared with the known RXRα1 isoform, are useful for detecting DNAs that comprise the nucleotide sequence of SEQ ID NO: 1 or 3. An oligonucleotide capable of hybridizing with the nucleotide sequence of an above region or its complementary sequence may be useful as a probe or primer for specifically detecting RXRα2 or RXRα3.

For example, a set of primers that anneals to a region where the exons are not shared is useful for specific amplification of the isoforms.

The polynucleotides of the present invention may be used for testing and diagnosing abnormalities of the proteins of the present invention. For example, Northern hybridization or RT-PCR that uses the polynucleotides of the present invention as probes or primers may be performed to test for abnormal expression. In addition, genomic DNA PCR and RT-PCR may be performed by carrying out polymerase chain reactions (PCR) using the polynucleotides of the present invention as primers. Furthermore, by amplifying genes that encode the proteins of the present invention, or their expression regulatory regions, sequence abnormalities may be examined and diagnosed using RFLP analysis, SSCP, sequencing, and such.

The RXRα isoforms of the present invention exhibit a transcriptional activation function that increases in response to stimulus with the ligand, retinoic acid. In addition, the RXRα isoforms of the present invention require SRC-1 as a co-activator. Therefore, polynucleotides encoding such factors can be used for gene therapy of diseases associated with the factors. Specifically, by expressing the RXRα2 or RXRα3 of the present invention through introducing a polynucleotide such that it is appropriately expressed at a host's disease site, diseases associated with that factor can be treated or prevented. Herein, "expression" includes transcription and/or translation. The expression of a gene at the transcription level can be examined and diagnosed by analyzing expression using a polynucleotide of the present invention. In addition, gene expression at the translation level can be examined and diagnosed using antibodies against the proteins of the present invention.

RXRα was only known to have a single isoform, RXRα1. The present invention revealed novel isoforms that differ in their co-activator requirements. Strictly speaking, diseases caused by RXRα dysfunction are caused by impaired function of different isoforms. Therefore, in patients for whom enhanced RXRα1 activity does not improve symptoms, RXRα2 or RXRα3 dysfunction may be contributing to the disease. RXRα1 requires a different co-activator to RXRα2 or RXRα3. Thus, agonists screened using RXRα1 may not have any effect on RXRα2 or RXRα3. Agonists that can be obtained using the present invention may be effective therapeutic agents for diseases for which agonists against the known RXRα1 isoform cannot achieve sufficient therapeutic effect.

In addition, a "polynucleotide comprising the nucleotide sequence of SEQ ID NO: 1 or 3, or a polynucleotide comprising at least 15 nucleotides that are complementary to the complementary strand of the polynucleotide" comprises an antisense polynucleotide capable of suppressing the expression of a protein of the present invention. Herein, the antisense polynucleotide means a polynucleotide that suppresses the expression of a protein comprising the amino acid sequence of SEQ ID NO: 2 or 4.

A "double-stranded RNA of 21 to 23 base pairs, comprising a sense RNA corresponding to a partial sequence of the nucleotide sequence of the polynucleotide of SEQ ID NO: 1 or 3, and its antisense RNA" comprises small interfering RNA (siRNA) for suppressing the expression of a protein of the present invention. The double-stranded RNAs of the present invention comprise not only double-stranded RNAs with two strands, but also single-stranded RNAs with hairpin loop structures comprising sense and antisense sequences.

Antisense or siRNAs that suppress the expression of RXRα2 or RXRα3 may be used as agents to control their activity.

The antisense polynucleotides may be antisense DNAs, or antisense oligonucleotides. Antisense DNAs are at least 15 base pairs or longer, preferably 100 base pairs or longer, more preferably 500 base pairs or longer, and normally 3000 base pairs or shorter, preferably 2000 base pairs or shorter. The antisense DNAs may be used alone, or by insertion in the antisense orientation into appropriate vectors, such as retrovirus vectors, or adenovirus vectors. The antisense oligonucleotides are at least ten base pairs or longer, and normally 100 base pairs or shorter; preferably 20 base pairs or longer and 50 base pairs or shorter. The antisense oligonucleotides may be prepared, for example, based on the nucleotide sequence information of SEQ ID NO: 1, or 3 by using the phosphorothioate method (Stein "Physicochemical properties of phosphorothioate oligodeoxynucleotides." Nucleic Acids Res. 16: 3209-3221 (1988)).

In addition, siRNAs may be introduced into an living organism by administering a vector that carry DNAs for both their sense and antisense strands. Vectors for siRNA expression are known.

The antisense or siRNAs may be used for gene therapy of diseases caused by abnormalities of proteins comprising an amino acid sequence of SEQ ID NO: 2 or 4. Protein abnormalities include abnormal protein function, abnormal protein expression, and such. When used for gene therapy, patients may be administered using *ex vivo* or *in vivo* methods, by using viral vectors such as retrovirus vectors, adenovirus vectors, and adeno-associated virus vectors, or non-viral vectors such as liposomes.

When used for gene therapy, the polynucleotides and antisense polynucleotides may be administered to patients using *ex vivo* or *in vivo* methods, by using viral vectors such as retrovirus vectors, adenovirus vectors, and adeno-associated virus vectors, or non-viral vectors such as liposomes.

The present invention also relates to methods of detecting the activity of a test substance in regulating the transcription-controlling activity of a nuclear receptor, wherein the nuclear receptor is a protein encoded by a polynucleotide of any one of the following (A) to (E):
(A) a polynucleotide comprising the coding region of the nucleotide sequence of SEQ ID NO: 1 or 3;
(B) a polynucleotide encoding a protein comprising the amino acid sequence of SEQ ID NO: 2 or 4;
(C) a polynucleotide encoding a protein comprising an amino acid sequence wherein one or more amino acids are replaced, deleted, inserted, and/or added to the amino acid sequence of SEQ ID NO: 2 or 4, encoding a protein comprising the following (a) and (b):
   (a) The transcriptional activation function is enhanced in response to binding to the ligand retinoic acid, or its agonist; and
   (b) The protein requires SRC-1 as a co-activator;
(D) a polynucleotide that hybridizes under stringent conditions with a polynucleotide comprising the nucleotide sequence of SEQ ID NO: 1 or 3, encoding the protein comprising the (a) and (b) of (C); and
(E) a polynucleotide comprising a nucleotide sequence in which the nucleotide sequence from 1 to 102 of SEQ ID NO: 5 is deleted, or replaced with another nucleotide sequence, and which has 70% or higher homology to the nucleotide sequence from 103 to 1461 of SEQ ID NO: 5;
wherein the methods comprise the following steps:
(1) contacting test substances with cells expressing the nuclear receptor and comprising an expression cassette where a reporter gene is linked downstream of the nuclear receptor's responsive element;
(2) culturing the cells under conditions that enable the expression of the nuclear receptor, and measuring the expression level of the reporter gene in the cells; and
(3) detecting the activity of the test substances in regulating the transcription regulating activity of the nuclear receptor, using the measurement results of step (2) as an index.

In the above-mentioned methods of detection, proteins functionally equivalent to proteins comprising the amino acid sequence of SEQ ID NO: 2, or 4 refer to proteins comprising the above attributes (a) and (b). Proteins comprising the amino acid sequence of SEQ ID NO: 2 or 4 include the novel isoforms of RXRα protein discovered by the present inventors. In general, subtypes represent a group of molecules comprising similar structures. However, isoforms not only comprise common structures, and isoform can also refer to sharing exons, in particular. Human RXRα isoforms were unknown until the present inventors discovered two isoforms for the first time.

Methods for testing whether a given protein can use retinoic acid or its agonists as a ligand are described above. In addition, methods for confirming the transcriptional activation function of a given protein by co-activator RC-1 are also described above. By using these methods, proteins can be confirmed to be functionally equivalent proteins of the present invention.

Herein, the cells expressing a nuclear receptor, and comprising an expression cassette in which a reporter gene is linked downstream of a nuclear receptor's responsive element, may be obtained as follows:

First, a DNA encoding a nuclear receptor of interest is introduced into the cells for expressing the nuclear receptor. DNAs encoding nuclear receptors for use in the present invention may be DNAs comprising a coding region of the nucleotide sequence of SEQ ID NO: 1 or 3. In addition, DNAs encoding proteins functionally equivalent to a protein comprising the amino acid sequence of SEQ ID NO: 2 or 4 may be used. As shown in the Examples, proteins comprising the amino acid sequence of SEQ ID NO: 2 or 4 bind to retinoic acid as a ligand, and exhibit transcriptional activation function in the presence of SRC-1 co-activator.

Such DNAs are obtainable by screening cDNA libraries prepared from humans or other animals, such as nematodes, and yeast using PCR or hybridization based on respective nucleotide sequence information. Alternatively, DNAs comprising the essential nucleotide sequences may be synthesized based on nucleotide sequence information.

The DNAs are cloned in expression vectors, and transfected into cells. The expression vectors include pcDNA3.1 (Invitrogen), pMM324, pSV2dhfr (ATCC 37145) , pdBPV-MMTneo (ATCC 37224), and pSV2neo (ATCC 37149). pcDNA3.1(+) is a vector enabling high level expression of a foreign gene in mammalian cells.

Herein, cells transfected with a DNA encoding a nuclear receptor are those cells capable of translating the DNA into a protein, and comprising an expression cassette wherein a reporter gene is linked down stream of a responsive element for the introduced nuclear receptor. Herein, expression cassettes represent a responsive element and reporter gene set, in which reporter genes are located such that they are transcribed under the regulation of the responsive element. Expression cassettes may be introduced into cells as plasmids, or integrated into the cell genome.

Responsive elements are nucleotide sequences capable of binding to the DBD of a nuclear receptor, and are necessary for the initiation of transcription to mRNA by the transcription unit. RXRα responsive elements are already known (Mangelsdorf D.J., Umesono K., Kliewer S.A., Borgmeyer U., Ong E.S., and Evans R.M. A direct repeat in the cellular retinol-binding protein type II gene confers differential regulation by RXR and RAR. Cell 66: 555-561 (1991)). For example, the retinoic acid responsive element RRE used in the Examples comprises the nucleotide sequence of SEQ ID NO: 17.

Expressing reporter genes under the regulation of the responsive elements may be achieved by replacing a gene under the regulation of a responsive element with a reporter gene. Plasmids comprising an expression cassette that comprises a reporter gene downstream of a responsive element are called reporter plasmids.

Reporter plasmids may be readily constructed using reporter vectors. Reporter vectors comprise reporter genes, and upstream of that, cloning sites for inserting given responsive elements. For example, the reporter vector PGVP2, which comprises the luciferase gene as a reporter, is commercially available. A reporter plasmid for use in the present invention may be obtained by inserting an above responsive element into the cloning site of a reporter vector. Multiple responsive elements may be inserted in tandem to enhance the response to a nuclear receptor. Thus, increased sensitivity in detecting transcriptional activation is expected. The responsive elements to be inserted may be chemically synthesized.

Herein, the reporter genes can be any reporter genes for which signals can be easily measured. Reporter genes for analyzing the gene expression regulatory mechanisms are known. Specifically, genes encoding luciferase, catalase, β-galactosidase, green fluorescent protein (GFP), and such may be used as reporter genes. The cells transfected with the vectors may be, for example, animal cells in which that gene is deleted.

The cells transfected with the different kinds of genes may be animal cells, yeast cells, insect cells, or such. One example of preferable cells in the present invention are the monkey-kidney-derived CV-1 cell line used in the Examples.

Herein, the cells are cultured with co-activators in the presence of a test substance, under conditions enabling expression of the above nuclear receptor. The expression level of the reporter gene in the cells is then measured. Herein, conditions enabling the expression of an above nuclear receptor refer to conditions where a nuclear receptor gene comprised in an introduced vector is expressed in the host cells. For example, if the vector utilizes an inducible promoter, the cells are cultured under conditions required to induce expression.

If the nuclear receptor functions in a ligand-dependent manner, the cells are preferably provided with the ligand. On the other hand, if the nuclear receptor exhibits the transcriptional activation function ligand-independently, the above methods may be performed without providing a ligand. SRC-1 co-activators may be supplied to cells by transfecting the cells with an expression plasmid carrying the co-activator gene inserted into it, and expressing the co-activator within the cells. The amount of DNA used for transfection may be, for example, the same as the amount of RXRα expression plasmid.

In the methods of detection of the present invention, test substances are added to the culture to make contact with the above cells. Alternatively, if the test substances are proteins, the genes encoding them may be transfected into the same cells, and contacted with the nuclear receptors. Furthermore, cells expressing a test substance may also be contacted by co-culturing with cells expressing an above nuclear receptor.

Once a nuclear receptor is expressed, the reporter gene is transcribed depending on the co-activator SRC-1, and the protein is expressed. The expression level of the reporter gene can be determined by measuring the intensity of the signal originating from this protein. For example, when using the luciferase gene as the reporter gene, luciferase activity can be measured as chemiluminescent intensity. Alternatively, if the reporter gene is GFP, the fluorescent intensity of GFP can be measured.

If a coexisting test substance is capable of regulating the transcription-controlling activity, the transcription level of the reporter gene will change. If the test substance inhibits transcription, reporter gene transcription is suppressed and the signal will be reduced, based on the extent of this change. In contrast, a test substance comprising the function of enhancing transcriptional activation function will increase the reporter gene signal. Therefore, the influence of test substances on transcription-controlling activity can be evaluated using reporter gene transcription level as an index.

By using substances whose influence on transcription-controlling activity is already known, the relationship between the degree of influence on transcription-controlling activity and the intensity of the reporter gene signal can be clarified. Thus, the effect of substances whose influence on transcription-controlling activity is unknown can be quantitatively evaluated based on the intensity of this signal.

The methods of detection in the present invention may further comprise a step of detecting a test substance' s activity in regulating the transcription-controlling activity of other RXRα isoforms. Other isoforms of RXRα may include proteins encoded by the polynucleotide of any one of the following (A') to (E'):
(A') a polynucleotide comprising the coding region of the nucleotide sequence of SEQ ID NO: 5;
(B') a polynucleotide encoding a protein comprising the amino acid sequence of SEQ ID NO: 6;
(C') a polynucleotide encoding a protein comprising an amino acid sequence wherein one or more amino acids are replaced, deleted, inserted, and/or added to the amino acid sequence of SEQ ID NO: 6, wherein the protein is functionally equivalent to a protein comprising the amino acid sequence of SEQ ID NO: 6;
(D') a polynucleotide that hybridizes under stringent conditions with a polynucleotide comprising the nucleotide sequence of SEQ ID NO: 5, encoding a protein functionally equivalent to a protein comprising the amino acid sequence of SEQ ID NO: 6; and
(E') a polynucleotide comprising homology of 60% or more with the nucleotide sequence of SEQ ID NO: 5, encoding a protein functionally equivalent to a protein comprising the amino acid sequence of SEQ ID NO: 6.
Human RXRα1 protein, or its functionally equivalent proteins, are proteins encoded by the polynucleotide of any the above (A') to (E'). Herein, proteins functionally equivalent to human RXRα1 refer to proteins comprising the following attributes (a') and (b'):
   (a') The transcriptional activation function is enhanced in response to binding to the ligand retinoic acid or its agonist, and
   (b') The protein requires CBP as a co-activator.

RXRα1 is a known protein deposited in GenBank as accession number NM_002957. By evaluating the effect of test substances on the transcriptional activation function of not only the RXRα isoforms of the present invention, but also of other isoforms using similar techniques, the differences between a substance's specificity for each isoform can be identified. For example, each isoform shows a different level of activation depending on the type of co-activator. For example, the novel isoforms discovered in the present invention, RXRα2 and RXRα3, require CBP or SRC-1 as a co-activator, whereas the known isoform RXRα1 does not use SRC-1 as a co-activator. These differences may be the cause of functional differences between the isoforms in vivo. Therefore, the specificity of test substances for each isoform provides important information for enabling specific regulation of these nuclear receptors.

For example, isoform-selective agents selected by the evaluation methods of the present invention are expected to function specifically in tissues or organs where the isoform is expressed specifically, or under the physiological conditions to which the isoforms respond specifically. Such agents can be expected to have reduced side effects compared to conventional drugs that are not isoform-selective.

Herein, to detect the function of regulating the transcription-controlling activity of other isoforms, the above methods may be performed in the presence of a co-activator capable of activating each isoform. CBP, for example, is a preferred co-activator for proteins encoded by the polynucleotide of the above (A') to (E').

The activity of regulating the transcription-controlling activity ty of a nuclear receptor activated by SRC-1 can be evaluated by a detection method of the present invention. The method of detection may be used to evaluate substances capable of regulating the transcription-controlling activity. Thus, the present invention relates to methods for evaluating substances capable of regulating a transcription-controlling activity, wherein the methods comprise the following steps:
(1) using an above method to detect a test substance' s activity in regulating the transcription-controlling activity of a nuclear receptor, and
(2) selecting those test substances capable of suppressing or enhancing the transcription-controlling activity of the nuclear receptor, by comparison with a control.

The cells introduced with an expression vector for a nuclear receptor produce a signal generated from the reporter gene, in line with nuclear receptor expression. The obtained cells are seeded into a 96-well plate, and cultured under conditions that induce expression of the introduced genes. By adding a test substance to be evaluated into each well, the function of suppressing or enhancing the transcriptional activation function of the nuclear receptor may be readily detected, using reporter gene expression level as an index.

For example, when GFP is used as the reporter gene, it is possible to evaluate influence on the expression regulatory region by comparing the fluorescence intensities of GFP in conditions with or without the test substance. A significant difference may be judged from this comparison when the intensity ratio is, for example, two fold or more, or 1/2 or less, preferably five fold or more, or 1/5 or less, and more preferably ten fold or more, or 1/10 or less. The methods may be applicable not only to animal cells, but to any host cells, regardless of whether they are eukaryotic or prokaryotic cells, as long as the transcriptional activation function of an introduced nuclear receptor can be reproduced. The cells are cultured under conditions that enable reporter gene expression.

The test substances for the evaluation methods of the present invention are not limited. Specifically, they include cell extracts, products expressed from DNA libraries, synthetic low molecular weight compounds, synthetic peptides, and natural compounds, for example. Alternatively, antibodies that recognize a protein encoded by a polynucleotide of any one of the above (A) to (E) may be useful as candidate substances for screening.

Compounds known for their effect on transcription-controlling activity can be used as controls for the evaluation methods of the present invention. More specifically, compounds that clearly have no effect on transcription-controlling activity, for example, may be used as controls. Data measured without any test substance influence can be used as a control for cases without any effect on transcription-controlling activity.

Alternatively, by using substances already known to comprise a target function as controls, substances that induce a larger change in signal may be searched for selecting substances comprising relatively greater function.

The evaluation methods of the present invention may be used, for example, for screening for compounds that comprise desired activities, or for characterizing compounds. For example, screening may be performed by applying the evaluation methods to a broad range of compounds, and repeatedly selecting compounds that excel in the activity of interest. Alternatively, these detection methods may be used to analyze specific compound attributes.

Furthermore, the present invention relates to methods of detecting the activity of test substances in controlling the interaction between a nuclear receptor and a co-activator that activates the receptor, wherein the nuclear receptor is a protein encoded by the polynucleotide of any one of the following (A) to (E) :
(A) a polynucleotide comprising the coding region of the nucleotide sequence of SEQ ID NO: 1 or 3;
(B) a polynucleotide encoding a protein comprising the amino acid sequence of SEQ ID NO: 2 or 4;
(C) a polynucleotide encoding a protein comprising an amino acid sequence wherein one or more amino acids are replaced, deleted, inserted, and/or added to the amino acid sequence of SEQ ID NO: 2 or 4, wherein the protein comprises the following attributes (a) and (b) :
   (a) The transcriptional activation function is enhanced in response to binding to the ligand retinoic acid, or its agonist; and
   (b) The protein requires SRC-1 as a co-activator;
(D) a polynucleotide that hybridizes under stringent conditions with a polynucleotide comprising the nucleotide sequence of SEQ ID NO: 1 or 3, encoding a protein comprising the attributes of (a) and (b) in (C); and
(E) a polynucleotide comprising a nucleotide sequence in which the nucleotide sequence from 1 to 102 of SEQ ID NO: 5 is deleted, or replaced with another nucleotide sequence, and which has 70% or more homology to the nucleotide sequence from 103 to 1461 of SEQ ID NO: 5;
wherein the methods comprise the following steps:
(1) contacting the nuclear receptor, SRC-1, and a test substance any of the following orders i) to iii):
   i) contacting the nuclear receptor and the test substance first, and then contacting with SRC-1;
   ii) contacting the nuclear receptor and SRC-1 in the presence of the test substance; or
   iii) contacting the nuclear receptor and SRC-1 first, and then contacting with the test substance;
(2) measuring the amount of SRC-1 or test substance bound to the nuclear receptor; and
(3) detecting the activity of the test substance in binding to the nuclear receptor, using the measurement result of (2) as an index.

In contrast to the aforementioned methods of detecting effect on transcription-controlling activity using behavior within cells as an index, these methods detect the activity of binding to a nuclear receptor based on the interaction between substances. RXRα2 or RXRα3, or proteins functionally equivalent to these isoforms, which were discovered by the present invention, are the nuclear receptors used in the present invention's methods of detecting a test substance's ability to bind a nuclear receptor. The activity of binding a nuclear receptor can be detected by monitoring competition between co-activators.

Herein, co-activators for nuclear receptors may be SRC-1 or CBP, for example. SRC-1 enhances the transcriptional activation function of the isoforms RXRα2 and RXRα3 in a manner dependent on the amount of the ligand. CBP can function as a co-activator not only for RXRα2 and RXRα3, but also for known isoform RXRα1.

Herein, the nuclear receptors, their co-activators, and the test substances are exposed to each other in any of the following orders i) to iii) : First, i) by contacting the nuclear receptor with the test substance, and then with the co-activator, the function of inhibiting the co-activator's binding to the nuclear receptor can be evaluated. Next, ii) by contacting the nuclear receptor with the co-activator in the presence of the test substance, the function of the substance in competiting with the co-activator for nuclear receptor binding can be evaluated. Furthermore, iii) by contacting the nuclear receptor with the co-activator, and then with the test substance, the function of the substance in replacing co-activator binding to the nuclear receptor can be evaluated. Compounds comprising such functions, as detected by these methods, are useful as candidates for RXRα2 or RXRα3 agonists or antagonists.

Furthermore, the detection methods of the present invention comprise measuring the amount of co-activators or test substances bound to nuclear receptors. The amount of co-activator or test substance bound to a nuclear receptor may be measured by labeling these substances. Co-activators or test substances may be labeled with radioisotopes, substances with chemiluminescent, fluorescent, or enzymatic active substances, or tags. The amount of co-activator or test substance bound to a nuclear receptor may be measured by isolating the nuclear receptor from the reaction mixture, and measuring the label in the isolated fraction. The nuclear receptor may first be immobilized onto a solid phase to facilitate its isolation from the reaction mixture. Such solid phases commonly include magnetic beads, or the inside wall of reaction containers.

In the methods of detection of the present invention, the amount of co-activator bound to a nuclear receptor is correlated to the activity of a test substance in binding to that nuclear receptor: when the amount of co-activator bound to a nuclear receptor decreases compared with the amount in the absence of the test substance, the test substance is judged capable of binding to the nuclear receptor. Alternatively, when the amount of test substance bound to a nuclear receptor is measured, the test. substance is judged capable of inhibiting the interaction between the co-activator and the nuclear receptor if the binding of the substance decreases in the presence of the co-activator, compared with the amount in the absence of the co-activator.

Methods for evaluating compounds capable of binding to nuclear receptors are provided based on the methods for detecting binding activity of the present invention. Specifically, compounds that comprise the ability to bind nuclear receptors can be screened by measuring the ability to bind nuclear receptors, the results of the above detection methods, and then selecting compounds with strong binding ability compared to a control. Substances obtained by the evaluation methods are very likely to function as agonists or antagonists of the nuclear receptors, since these substances are capable of binding to the receptors. Such substances are useful as agents for controlling the activity of RXRα2 or RXRα3, the nuclear receptors of the present invention.

Herein, in the evaluation methods of the present invention, test substances similar to those used in the methods for evaluating the function of regulating transcription-controlling activity may be used. In addition, cases where test substances are not contacted may be used as controls. Alternatively, substances that comprise a binding ability greater that these compounds may be screened by using substances that clearly comprise the ability to bind a nuclear receptor of the present invention as a control.

Compounds isolated by the evaluation methods of the present invention may be candidates for compounds that regulate the transcription-controlling activity of the nuclear receptors (i.e. agonists and antagonists). They may be also candidates for compounds that control in vivo interactions between nuclear receptors, between nuclear receptors and responsive elements, or between nuclear receptors and co-activators. Herein, regulating transcription-controlling activity or interaction includes inhibiting or elevating transcriptional activation function. These compounds may be applied as medicines for preventing or treating diseases associated with nuclear receptors.

Furthermore, by comparing the functions of test substances for multiple isoforms, including the known RXRα isoform, the effect of substances on the transcription-controlling activity of each isoform can be compared. Thus, by selecting substances in which a different effect on the transcription-controlling activities of the isoforms was found, substances that selectively function on a particular isoform can be selected. Hence, the present invention provides methods of screening for the function of controlling activity in an RXRα isoform-specific manner. The present invention also relates to agents obtained by these methods that are capable of regulating transcription-controlling activity in RXRα isoform-specific manner. In addition, the invention relates to methods of controlling the transcription-controlling activity of RXRα in an isoform-specific manner, comprising the step of administrating a compound obtained by an above method. Alternatively, the invention relates to uses of the compounds obtained by the above methods for producing agents for regulating transcription-controlling activity in an RXRα isoform-specific manner.

In particular, it is likely that impaired RXRα2 or RXRα3function constitutes the disease of patients whose symptoms do not improve on enhancement of RXRα1 activity. RXRα1 requires different co-activators to RXRα2 or RXRα3. Thus, agonists screened using RXRα1 may not be useful for RXRα2 or RXRα3. The agonists obtained by the present invention may be effective therapeutic agents for diseases in which the agonists against the known isoform RXRα1 fail to achieve therapeutic effects.

The components required for the evaluation methods of the present invention may be provided as kits. The kits of the present invention comprise, for example, cells expressing a polynucleotide of (A) to (E), and expressing a reporter gene under the control of a responsive element of a nuclear receptor, and a co-activator required for maintaining the activity of the nuclear receptor.

The kits of the present invention may be further packaged with a medium for cell culture, and culture vessels. The culture vessels are preferably formed in a shape for application in high-throughput screening. The kits may further comprise an instruction manual illustrating the assay method, and a control that is a substance tested for a certain level of effect on the transcription-controlling activity of the nuclear receptor.

The present invention further relates to the medicinal use of substances obtained by the evaluation methods. Thus, the invention relates to the use of compounds selected by the evaluation methods of the present invention in the regulation of the transcription-controlling activity of a nuclear receptor. The invention also relates to therapeutic agents for diseases characterized by nuclear receptor abnormalities, particularly comprising the above compounds as effective ingredients. In addition, the invention relates to methods of regulating the transcription-controlling activity of a nuclear receptor, comprising the step of administrating compounds selected by the evaluation methods. The invention also relates to the use of compounds selected by the evaluation methods for producing agents that regulate the transcription-controlling activity of nuclear receptors.

Herein, diseases characterized by nuclear receptor abnormalities refer to diseases caused by abnormal levels of nuclear receptor expression or activity. Such diseases include a variety of cancers, hyperlipemia, arteriosclerosis, diabetes, a variety of inflammatory diseases such as inflammatory intestine, and psoriasis. The cancers include prostate cancer, acute promyelocytic leukemia, and hepatic cancer. All of these are diseases that accompany reduced RXR expression or function. Therefore, compounds promoting the transcriptional activation function of a nuclear receptor of the present invention are useful for treatment or prevention of these diseases.

Furthermore, the present invention relates to agents that regulate the transcription-controlling activity of the nuclear receptors, comprising a polynucleotide of any one of the following (A) to (E) , or a protein encoded by that polynucleotide as the main ingredient. In addition, the present invention relates to methods of controlling the activity of a nuclear receptor, comprising the step of administrating a polynucleotide any one of the following (A) to (E), or a protein encoded by that polynucleotide. The invention also relates to the use of a polynucleotide any one of the following (A) to (E), or a protein encoded by that polynucleotide, in the production of agents that regulating the transcription-controlling activity of a nuclear receptor.
(A) A polynucleotide comprising the coding region of the nucleotide sequence of SEQ ID NO: 1 or 3;
(B) a polynucleotide encoding a protein comprising the amino acid sequence of SEQ ID NO: 2 or 4;
(C) a polynucleotide encoding a protein comprising an amino acid sequence wherein one or more amino acids are replaced, deleted, inserted, and/or added to the amino acid sequence of SEQ ID NO: 2 or 4, encoding a protein comprising the following attributes (a) and (b):
   (a) The transcriptional activation function is enhanced in response to binding to the ligand retinoic acid, or its agonist; and
   (b) The protein requires SRC-1 as a co-activator;
(D) a polynucleotide that hybridizes under stringent conditions with a polynucleotide comprising the nucleotide sequence of SEQ ID NO: 1 or 3, encoding the protein comprising the attributes (a) and (b) of (C); and
(E) a polynucleotide comprising a nucleotide sequence in which the nucleotide sequence from 1 to 102 of SEQ ID NO: 5 is deleted, or replaced with another nucleotide sequence, and which comprises 70% or more homology to the nucleotide sequence from 103 to 1461 of SEQ ID NO: 5.

The present invention also relates to agents that inhibit the activity of a nuclear receptor, comprising any one of the following (1) to (4) as an effective ingredient. In addition, the invention relates to methods of inhibiting the activity of a nuclear receptor, comprising the step of administrating any one of the following (1) to (4). The invention also relates to the use of any one of the following (1) to (4) in producing agents that inhibit the activity of a nuclear receptor.
(1) An antisense polynucleotide of a polynucleotide of the above (A) to (E);
(2) an antibody that recognizes a protein encoded by a polynucleotide of the above (A) to (E);
(3) a protein exerting a dominant-negative effect against a protein encoded by the polynucleotide of the above (A) to (E) ; and
(4) a double-stranded RNA of 21 to 23 base pairs, comprising a sense RNA corresponding to a partial sequence of the nucleotide sequence of a polynucleotide of the above (A) to (E), and its antisense RNA.

The proteins, polypeptides, antibodies and proteins with dominant negative form of the present invention, and the substances isolated by the above evaluation methods, are useful for regulating the transcription-controlling activity of nuclear receptors. Herein, these may be used as medicines by themselves, or formulated as drugs by using common pharmaceutical methods. For example, they may be formulated by appropriately mixing with a pharmacologically acceptable carrier or vehicle; specifically, sterile water, saline, plant oils, emulsions, suspensions and such.

Administration to patients may be performed by standard methods known to those skilled in the art, such as arterial injection, intravenous injection, and subcutaneous injection. Dosages may vary depending on patient body weight and age, or the method of administration; however, one skilled in the art can appropriately select a suitable dose. If the compound can be encoded by DNA, the DNA may be incorporated into a gene therapy vector to perform gene therapy.

The dosages and methods of administration may vary depending on patient body weight, age, or symptoms. Those skilled in the art can appropriately select suitable dosages and methods of administration.

Furthermore, the present invention relates to model animals in which the activity of a nuclear receptor is regulated, comprising transgenic non-human animals in which the expression of a protein encoded by a polynucleotide of any one of the following (A) to (E) is controlled:
(A) a polynucleotide comprising the coding region of the nucleotide sequence of SEQ ID NO: 1 or 3;
(B) a polynucleotide encoding a protein comprising the amino acid sequence of SEQ ID NO: 2 or 4;
(C) a polynucleotide encoding an amino acid sequence wherein one or more amino acids are replaced, deleted, inserted, and/or added to the amino acid sequence of SEQ ID NO: 2 or 4, encoding a protein comprising the following attributes (a) and (b):
   (a) The transcriptional activation function is enhanced in response to binding to the ligand retinoic acid, or its agonist; and
   (b) The protein requires SRC-1 as a co-activator;
(D) a polynucleotide that hybridizes under stringent conditions with a polynucleotide comprising the coding region of the nucleotide sequence of SEQ ID NO: 1 or 3, encoding a protein comprising the attributes (a) and (b) of (C); and
(E) a polynucleotide comprising a nucleotide sequence in which the nucleotide sequence from 1 to 102 of SEQ ID NO: 5 is deleted, or replaced with another nucleotide sequence, and which comprises 70% or more homology to the nucleotide sequence from 103 to 1461 of SEQ ID NO: 5.

For example, transgenic animals in which the expression or activity of a protein of any one of (A) to (E) is reduced may be useful as model animals in which diseases associated with reduced transcriptional activation function of the nuclear receptor can be induced. More specifically, the animals may be used for evaluating therapeutic agents for treatment of diseases such as a variety of cancers, hyperlipemia, arteriosclerosis, diabetes, a variety of inflammatory diseases such as inflammatory intestine, or psoriasis. Cancers include prostate cancer, acute promyelocytic leukemia, and hepatic cancer.

The transgenic animals of the present invention comprise animals in which the expression or activity of a protein of any one of (A) to (E) is regulated. For example, by obtaining in an animal a gene that is counterpart to human RXRα2 or RXRα3, and controlling the expression level or activity of this gene, the expression and activity of the protein can be controlled. By changing the initiation codon of the gene into another codon, gene expression can be suppressed. Alternatively, the activity of a protein encoded by the gene may be diminished by introducing a mutation or deletion into the region essential for maintaining the protein activity. Such methods of genetic alteration are commonly known to those skilled in the art.

Furthermore, the present invention relates to methods of diagnosing diseases caused by abnormal nuclear receptor activity. Such methods comprise the steps of measuring the expression level of a polynucleotide of any one of the following (A) to (E) in a biological sample collected from a test subject, and judging the subject to have a disease that results from impaired function of a protein encoded by the polynucleotide if the expression level is low compared with a control level. In contrast, if the expression level is high the subject is judged to have a disease that results from elevated function of the protein.
(A) A polynucleotide comprising the coding region of the nucleotide sequence of SEQ ID NO: 1 or 3;
(B) a polynucleotide encoding the amino acid sequence of SEQ ID NO: 2 or 4;
(C) a polynucleotide encoding a protein comprising an amino acid sequence wherein one or more amino acids are replaced, deleted, inserted, and/or added to the amino acid sequence of SEQ ID NO: 2 or 4, encoding a protein comprising the following attributes (a) and (b):
   (a) The transcriptional activation function is enhanced in response to binding to the ligand retinoic acid, or its agonist; and
   (b) The protein requires SRC-1 as a co-activator;
(D) a polynucleotide that hybridizes under stringent conditions with a polynucleotide comprising the coding region of the nucleotide sequence of SEQ ID NO: 1 or 3, encoding a protein comprising the attributes (a) and (b) of (C);
(E) a polynucleotide comprising a nucleotide sequence in which the nucleotide sequence from 1 to 102 of SEQ ID NO: 5 is deleted, or replaced with another nucleotide sequence, and which comprises 70% or more homology to the nucleotide sequence from 103 to 1461 of SEQ ID NO: 5.

The expression level of the polynucleotides may be measured by, for example, quantifying mRNA, or protein, which is the translated product, or using the protein activity as an index. Methods for quantifying mRNA or protein are commonly known. For example, cells can be collected from a target organ for a disease of a test subject by biopsy, an RNA fraction can be prepared using standard methods, and quantitative RT-PCR using a sequence specific to RXRα2 or RXRα3 as a primer can be performed, and the expression level of the isoforms in the disease organ can thus be measured.

Proteins encoded by the above polynucleotides of any one of (A) to (E) are capable of exhibiting transcriptional activation function in a ligand-dependent manner in the presence of SRC-1 co-activator. Thus, by using this effect as an index, expression levels may be determined based on the activity of these proteins.

If a significant difference is found on comparing the obtained data with that of normal subjects, the transcription-controlling activity of the nuclear receptor is suspected to be abnormal.

Alternatively, diseases caused by abnormal RXRα2 or RXRα3 activity can be diagnosed using the mutation or polymorphism of the RXRα2 or RXRα3 genes. For example, a variety of cancers, hyperlipemia, arteriosclerosis, diabetes, a variety of inflammatory diseases such as inflammatory intestine, or psoriasis are considered to be caused by reduced RXR function. Cancers include prostate cancer, acute promyelocytic leukemia, and hepatic cancer. These diseases, which are caused by impaired RXR function, can carry mutations that lead to reduced RXRα2 or RXRα3 activity. Once a correlation between a genetic variation such as a SNP and susceptibility to an above disease is proved with regard to RXRα2 or RXRα3, the mutation can be identified using methods for determining mutations within the RXRα2 or RXRα3 gene (for example, PCR-SSCP method) by using blood collected from a test subj ect. This information may be used to predict disease onset, or to clarify onset mechanisms.

For example, patients with a disease that accompanies impaired RXR function and who do not show improvement on enhancing RXRα1 activity may have a disease caused by RXRα2 or RXRα3 dysfunction. The methods of diagnosis of the present invention may be useful for identifying the cause of disease in such patients. If the patients are predicted to have impaired RXRα2 or RXRα3 function, administration of RXRα2 or RXRα3 agonists can be selected as a treatment regimen.

### Brief Description of the Drawings

Fig. 1 shows a schematic map of the pME18SFL3 vector.
Fig. 2 shows the nucleotide sequence of the cDNA for RXRα2 (C-BNGH42008649) (SEQ ID NO: 1).
Fig. 3 shows the nucleotide sequence of the cDNA for RXRα2 (C-BNGH42008649) , and its translated amino acid sequence (SEQ ID NO: 2). (continued from Fig. 2)
Fig. 4 shows the nucleotide sequence of the cDNA for RXRα2 (C-BNGH42008649), and its translated amino acid sequence. (continued from Fig. 3)
Fig. 5 shows the nucleotide sequence of the cDNA for RXRα2 (C-BNGH42008649), and its translated amino acid sequence. (continued from Fig. 4)
Fig. 6 shows the nucleotide sequence of the cDNA for RXRα3 (C-BRAMY2003287) (SEQ ID NO: 3).
Fig. 7 shows the nucleotide sequence of the cDNA for RXRα3 (C-BRAMY2003287), and its translated amino acid sequence (SEQ ID NO: 4). (continued from Fig. 6)
Fig. 8 shows the nucleotide sequence of the cDNA for RXRα3 (C-BRAMY2003287), and its translated amino acid sequence. (continued from Fig. 7)
Fig. 9 shows the nucleotide sequence of the cDNA for RXRα3 (C-BRAMY2003287), and its translated amino acid sequence. (continued from Fig. 8)
Fig. 10 shows the results of comparing the nucleotide sequences of the cDNAs for RXRα2 (C-BNGH42008649; SEQ ID NO: 1), RXRα3 (C-BRAMY2003287; SEQ ID NO: 3), and RXRα1 (SEQ ID NO: 5). From the top, the nucleotide sequences of RXRα1, RXRα2, and RXRα3 are shown. Missing bases are shown with a "-". Those bases that match all three isoforms are indicated by a "*". The bases matched in two isoforms are indicated using a ".".
Fig. 11 shows the results of comparing the nucleotide sequences of the cDNAs for RXRα2 (C-BNGH42008649), RXRα3 (C-BRAMY2003287) , and RXRα1. (continued from Fig. 10)
Fig. 12 shows the results of comparing the nucleotide sequences of the cDNAs for RXRα2 (C-BNGH42008649), RXRα3 (C-BRAMY2003287), and RXRα1. (continued from Fig. 11)
Fig. 13 shows the results of comparing the nucleotide sequences of the cDNAs for RXRα2 (C-BNGH42008649), RXRα3 (C-BRAMY2003287), and RXRα1. (continued from Fig. 12)
Fig. 14 shows the results of comparing the nucleotide sequences of the cDNAs for RXRα2 (C-BNGH42008649), RXRα3 (C-BRAMY2003287), and RXRα1. (continued from Fig. 13)
Fig. 15 shows the results of comparing the nucleotide sequences of the cDNAs for RXRα2 (C-BNGH42008649), RXRα3 (C-BRAMY2003287), and RXRα1. (continued from Fig. 14)
Fig. 16 shows the results of comparing the nucleotide sequences of the cDNAs for RXRα2 (C-BNGH42008649), RXRα3 (C-BRAMY2003287), and RXRα1. (continued from Fig. 15)
Fig. 17 shows the results of comparing the nucleotide sequences of the cDNAs for RXRα2 (C-BNGH42008649), RXRα3 (C-BRAMY2003287) , and RXRα1. (continued from Fig. 16)
Fig. 18 shows the results of comparing the nucleotide sequences of the cDNAs for RXRα2 (C-BNGH42008649), RXRα3 (C-BRAMY2003287) , and RXRα1. (continued from Fig. 17)
Fig. 19 shows the results of comparing the nucleotide sequences of the cDNAs for RXRα2 (C-BNGH42008649), RXRα3 (C-BRAMY2003287), and RXRα1. (continued from Fig. 18)
Fig. 20 shows the results of comparing the nucleotide sequences of the cDNAs for RXRα2 (C-BNGH42008649), RXRα3 (C-BRAMY2003287), and RXRα1. (continued from Fig. 19)
Fig. 21 shows the results of comparing the nucleotide sequences of the cDNAs for RXRα2 (C-BNGH42008649), RXRα3 (C-BRAMY2003287), and RXRα1. (continued from Fig. 20)
Fig. 22 shows the results of comparing the nucleotide sequences of the cDNAs for RXRα2 (C-BNGH42008649), RXRα3 (C-BRAMY2003287), and RXRα1. (continued from Fig. 21)
Fig. 23 shows the results of comparing the nucleotide sequences of the cDNAs for RXRα2 (C-BNGH42008649), RXRα3 (C-BRAMY2003287), and RXRα1. (continued from Fig. 22)
Fig. 24 shows the results of comparing the nucleotide sequences of the cDNAs for RXRα2 (C-BNGH42008649), RXRα3 (C-BRAMY2003287), and RXRα1. (continued from Fig. 23)
Fig. 25 shows the results of comparing the amino acid sequences encoded by the cDNAs for RXRα2 (C-BNGH42008649), RXRα3 (C-BRAMY2003287), and RXRα1. From the top, the amino acid sequences of RXRα1 (462 aa; SEQ ID NO: 6), RXRα2 (435 aa; SEQ ID NO: 2), and RXRα3 (365 aa; SEQ ID NO: 4) are shown. Missing amino acids are indicated by a "-", and those matched in all three isoforms are indicated by a "*". The amino acids matched in two isoforms are indicated using a ".".
Fig. 26 shows the results of searching the genomic sequence for RXRα2 (C-BNGH42008649) , RXRα3 (C-BRAMY2003287) , and RXRα1, and the predicted exons based on these results. BNGH, BRAMY, and hRXRα represent the predicted exons for RXRα2 (C-BNGH42008649), RXRα3 (C-BRAMY2003287), and RXRα1 respectively. Those exons unique to each isoform are shaded.
Fig. 27 shows photographs of the result of analysis of the expression level of RXRα2 (C-BNGH42008649; lane 4), RXRα3 (C-BRAMY2003287; lane 5), and RXRα1 (lane 3) in spleen and brain. Lane 1 shows 100 bp ladder, and lane 2 shows the level of GAPDH expression.
Fig. 28 is a chart showing the effect of LG100268 (an RXRα agonist) on the transcription-controlling activity of the homodimer of each RXRα isoform. The X-axis shows LG100268 concentration (M), and the Y-axis shows corrected luciferase activity. The columns show, from left to right, the results of the reporter plasmid alone (repRXRE alone), RXRα1 homodimer (rep+RXRa1), RXRα2 homodimer (rep+RXRa2), and RXRα3 homodimer (rep+RXRa3).
Fig. 29 is a chart showing the effect of LG100268 (a RXRα agonist) on the transcription-controlling activity of heterodimers of PPARγ and each RXRα isoform. The X-axis shows LG100268 concentration (M) , and the Y-axis shows corrected luciferase activity. The columns show, from left to right, the results of the reporter plasmid alone (repRXRE alone) , heterodimer of PPARγ and RXRα1 (rep+PPARg+RXRa1), heterodimer of PPARγ and RXRα2 (rep+PPARg+RXRa2), and heterodimer of PPARγ and RXRα3 (rep+PPARg+RXRa3).
Fig. 30 shows the differences in the transcriptional activation functions of the RXRα isoforms induced by ligands in the presence of different co-activators. The X-axis shows LG100268 concentration (M), and the Y-axis shows corrected luciferase activity.

### Best Mode for Carrying Out the Invention

This invention will be explained in further detail below with reference to Examples, but it is not to be construed as being limited thereto. All the references to prior arts cited in the present invention are comprised herein by reference.

### [Example 1] Construction of a cDNA library by oligo-capping method

### (1) mRNA extraction and purchased mRNA

Human cultured cells (H4 cells (ATCC #HTB-148)) were cultured using the method described in the catalogue, and messenger RNA was extracted from the cells as total RNA using the methods in the literature (J. Sambrook, E. F. Fritsch, and T. Maniatis, Molecular Cloning Second edition, Cold Spring Harbor Laboratory Press (1989)).

In addition, mRNA, extracted and purified as polyA(+) RNA from human brain amygdala (CLONTECH #6574-1), was purchased. cDNA libraries were constructed using RNA samples that were mixtures of polyA(-) RNAandpolyA(+) RNA from tissues. PolyA(-) RNA was prepared on oligo-dT cellulose by removing polyA(+) RNA from total RNA from whole brain (CLONTECH #64020-1).

### (2) Construction of cDNA libraries

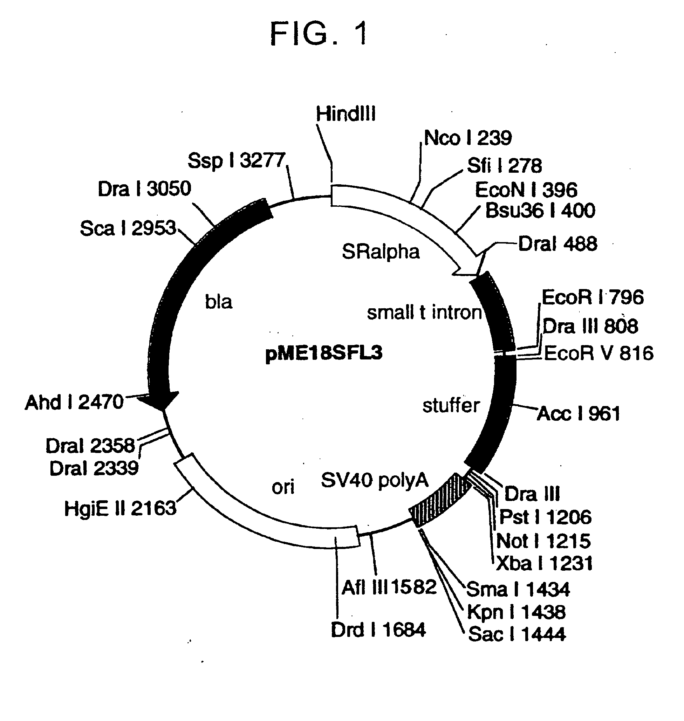

cDNA libraries were constructed from each RNA using a method (WO 01/04286) modified from oligo-capping (M. Maruyama and S. Sugano, Gene 138: 171-174 (1994)). Using an oligo-cap linker (SEQ ID NO: 7) , and an oligo-dT primer (SEQ ID NO: 8), BAP (bacterial alkaline phosphatase) treatment, TAP (tobacco acid pyrophosphatase) treatment, RNA ligation, first strand synthesis, and RNA removal were performed as described in WO 01/04286. Then, the cDNA was converted to double-stranded cDNA by PCR (polymerase chain reaction) using 5'-(SEQ ID NO: 9) , and 3'- (SEQ ID NO: 10) PCR primers, and then digested with SfiI. Then, cDNA fragments were typically fractioned to obtain cDNAs of 2 kb or longer (3 kb or longer in some case), which were unidirectionally cloned into DraIII-digested pME18SFL3 vectors (Fig. 1) (GenBank AB009864, Expression vector) to construct cDNA libraries.

cDNA libraries containing a high percentage of full-length clones (the average percentage of full-length 5'-ends for each library was 90%, calculated using the coding regions of known mRNAs as indicators) were constructed using a method modified from the oligo-capping method using the pME18SFL3 expression vector, which enables expression in eukaryotic cells. The pME18SFL3 vector contains a SRα promoter and an SV40 small t intron upstream of the cloning sites, and a SV40 polyA addition signal downstream. As the cloning sites contain asymmetric DraIII sites, and the cDNA fragments contain SfiI sites complementary to the DraIII sites at their ends, cloned cDNA fragments are unidirectionally inserted downstream of the SRα promoter. Therefore, clones containing full-length cDNA can be expressed transiently by introducing obtained plasmid into cells such as COS cells. Thus, it is very easy to experimentally analyze the gene products as a protein, or their biological activity.

### [Example 2] Analysis of the terminal sequence of cDNA clones and selection of clones for analyzing the full-length nucleotide sequence

The nucleotide sequences of the 5'-end of the cDNA clones obtained from each cDNA library were analyzed on a DNA sequencer (ABI PRISM 3700, PE Biosystems). Sequencing reactions were performed according to the manuals and by using DNA sequencing reagents (Dye Terminator Cycle Sequencing FS Ready Reaction Kit, dRhodamine Terminator Cycle Sequencing FS Ready Reaction Kit, or BigDye Terminator Cycle Sequencing FS Ready Reaction Kit, PE Biosystems). The data obtained was stored in a database.

The analyzed 5'-end sequences of the cDNA clones were searched for homology using BLAST, targeting recorded data of complete coding regions (CDS) in GenBank and UniGene. Sequences identical to the human mRNA sequences were removed. The sequences were then clustered into groups, whereby clones comprising homology of 90% or more and a consensus sequence of 50 bases or longer were deemed to be in the same group. Clones with a longer 5'-terminus were selected from the groups, and, as necessary, the 3'-end sequences of these selected clones were analyzed and obtained in the same way as for the 5'-end sequences. By analyzing these obtained terminal sequences, clones making contig at the 5'- and 3'- ends were removed. BLAST homology searches were performed again, as described above, to remove sequences identical to human mRNA sequences (including sequences patented or in patent applications). Clones whose full-length sequence was to be analyzed were obtained from the selected clones.

### [Example 3] Analysis of full-length nucleotide sequences

The nucleotide sequence of each full-length cDNA was determined for those clones selected for full-length sequence analysis. The nucleotide sequences were determined mainly by primer walking with dideoxy terminator methods using custom-synthesized DNA primers. Specifically, sequence reactions were performed using custom-synthesized DNA primers, and DNA sequencing reagents, according to the manual (PE Biosystems). Nucleotide sequences were analyzed on a sequencer from the same company (PE Biosystems). Some clones were also analyzed using a DNA sequencer from Licor.

Some clones were subjected to the shotgun method, which randomly cuts plasmids comprising cDNAs without using custom primers, to determine the nucleotide sequence with a DNA sequencer in the same way. Full-length sequences were finally obtained by overlapping the partial nucleotide sequences determined as above. These full-length nucleotide sequences were used to predict the regions translated into proteins, and to deduce amino acid sequences. BLAST homology searches were performed on databases containing the determined nucleotide sequences. The clones C-BNGH42008649 and C-BRAMY2003287, which showed high homology to nuclear receptor RXRα, were discovered.

### [Example 4] Analysis of the cDNA nucleotide sequence of RXRα2 (C-BNGH42008649) and RXRα3 (C-BRAMY2003287), and comparison with the cDNA nucleotide sequence encoding the known RXRα isoform

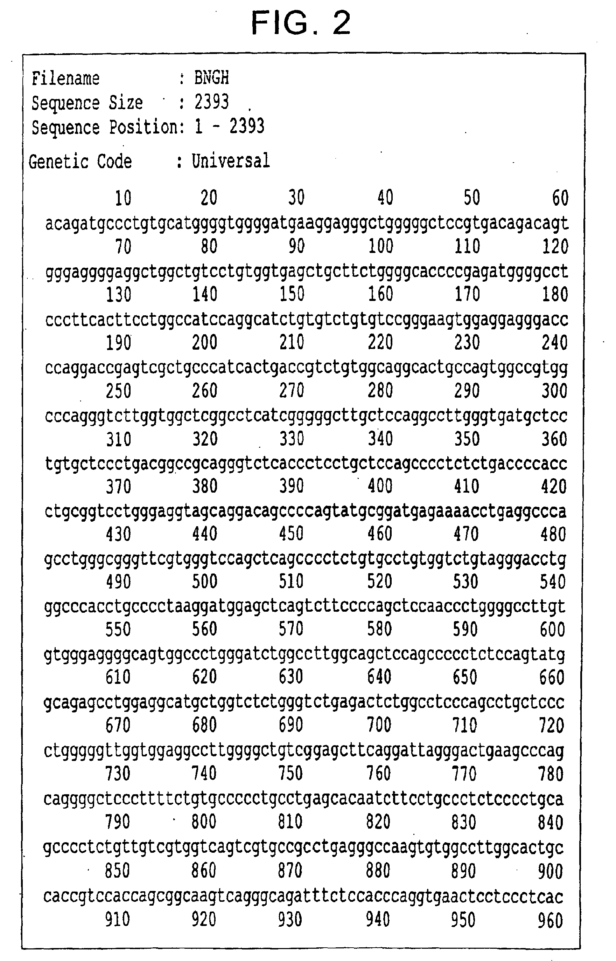

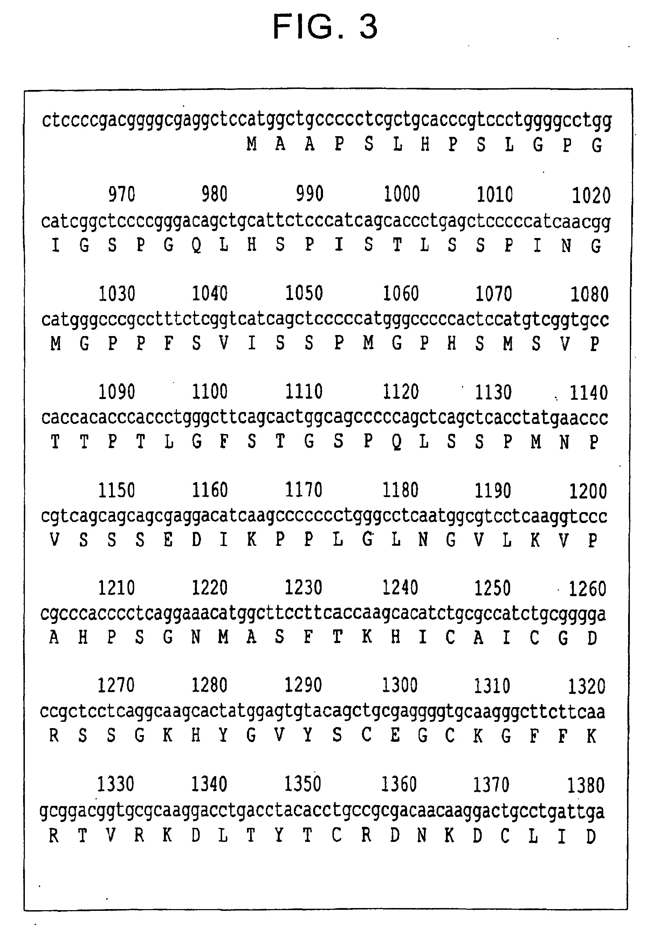

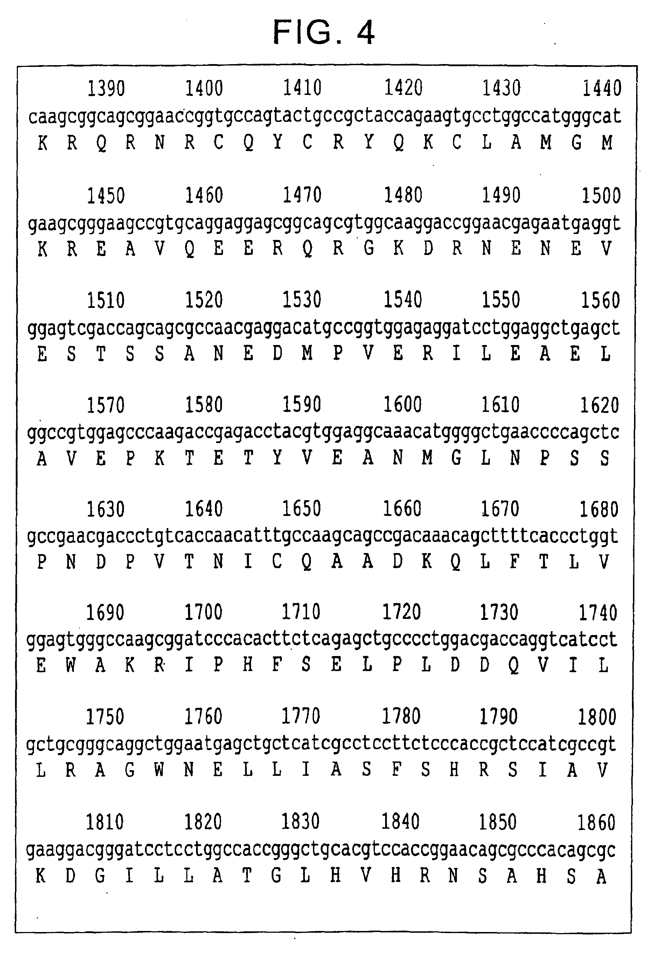

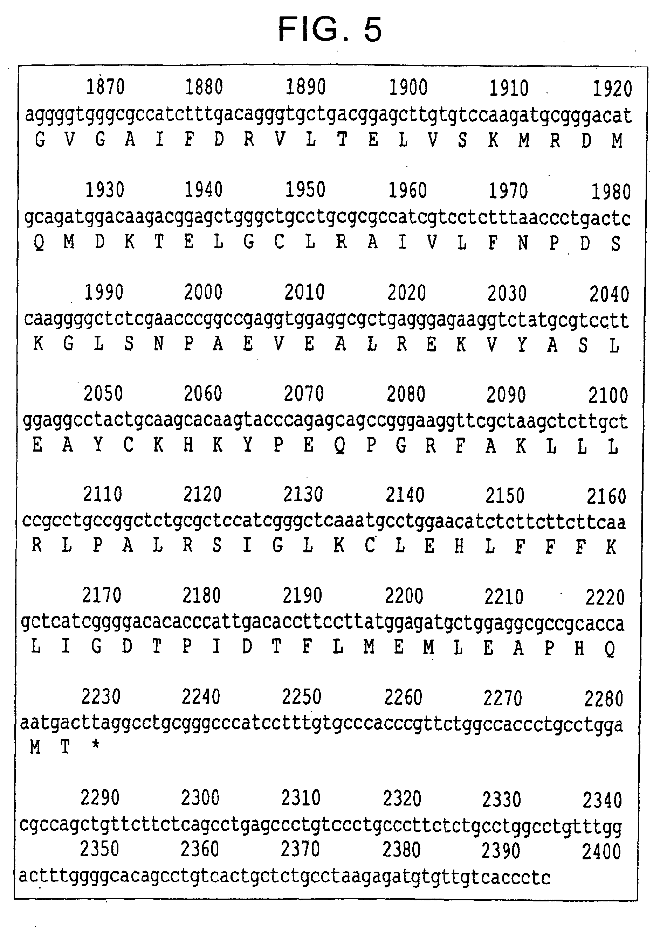

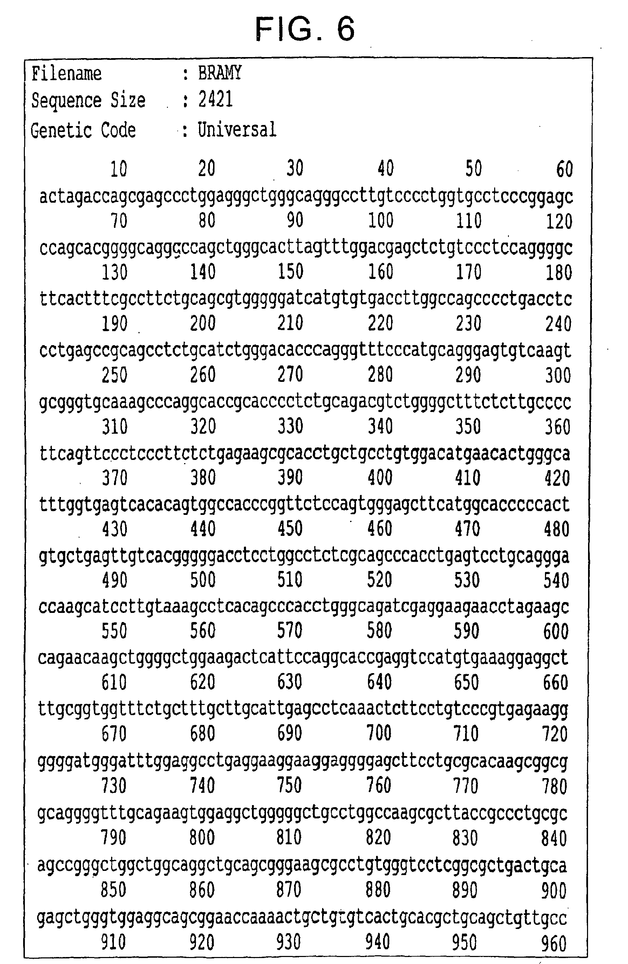

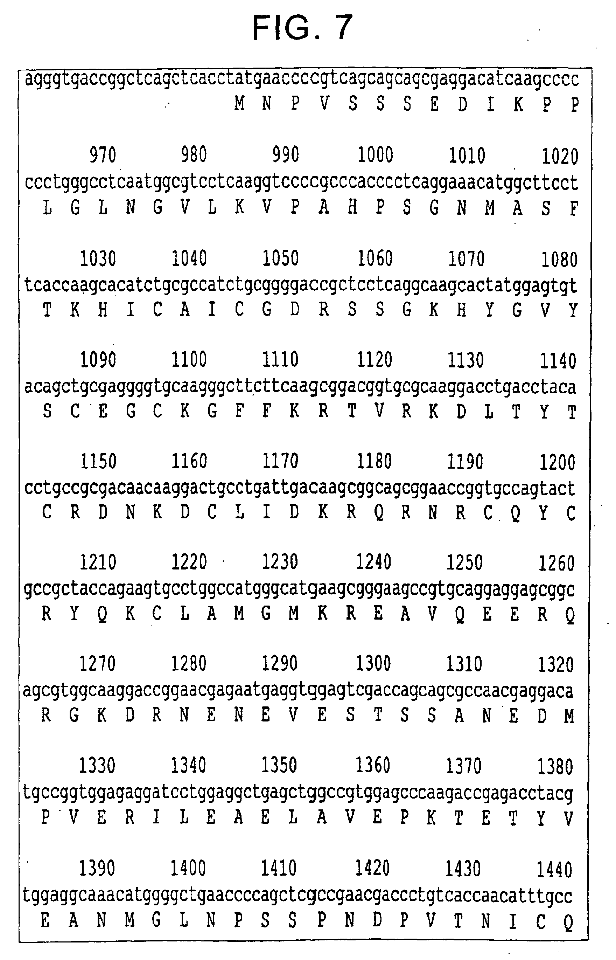

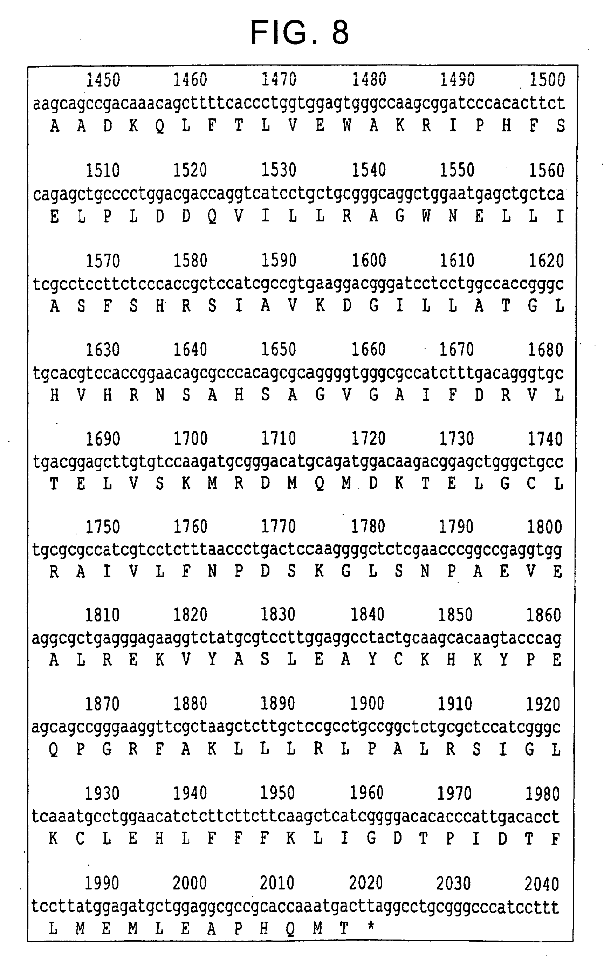

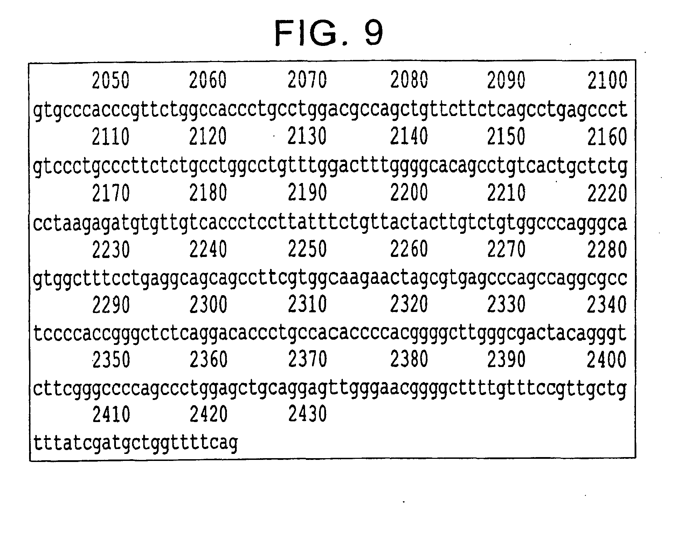

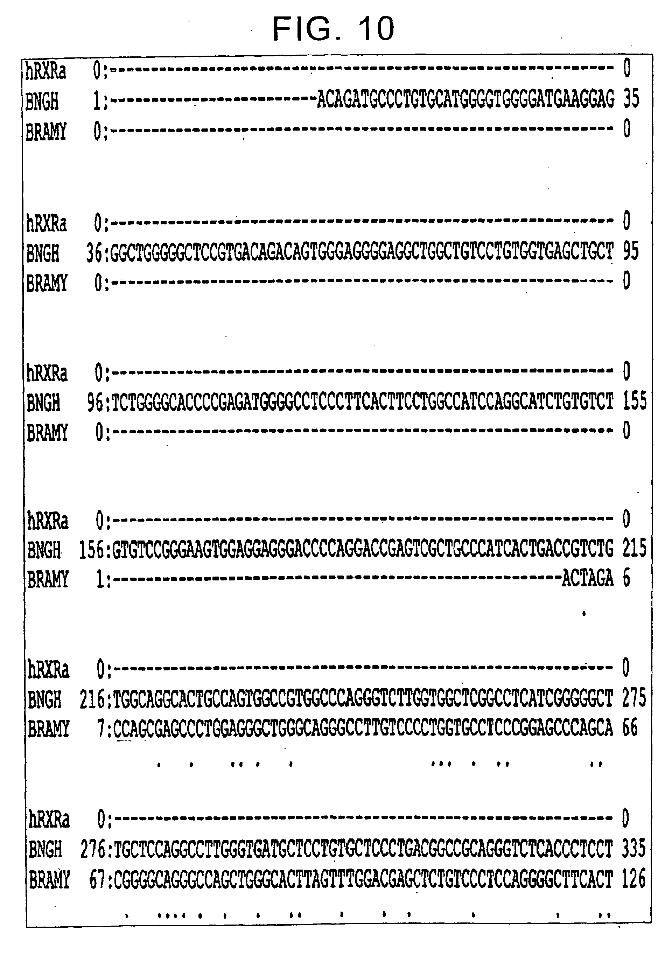

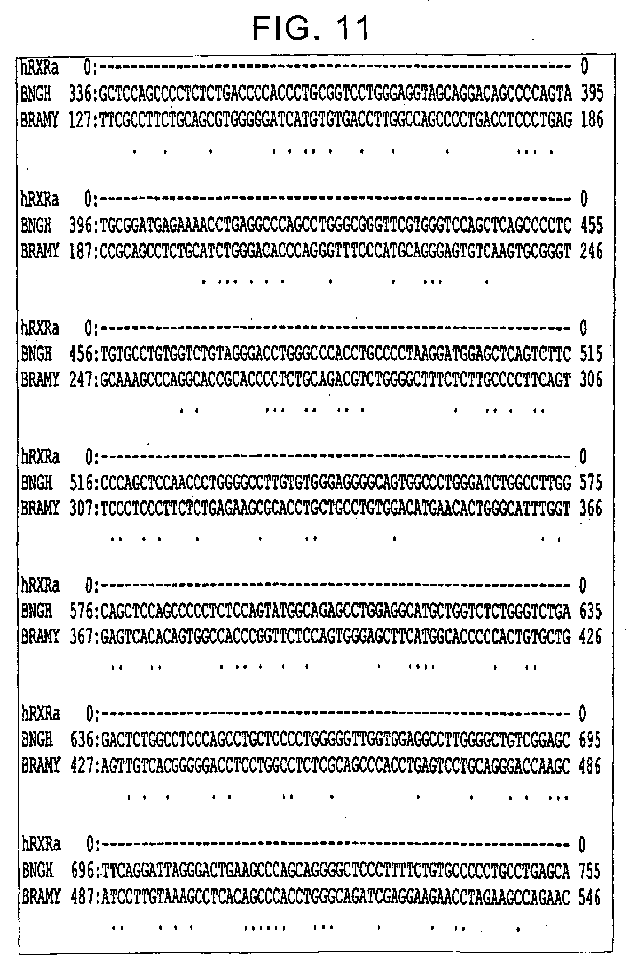

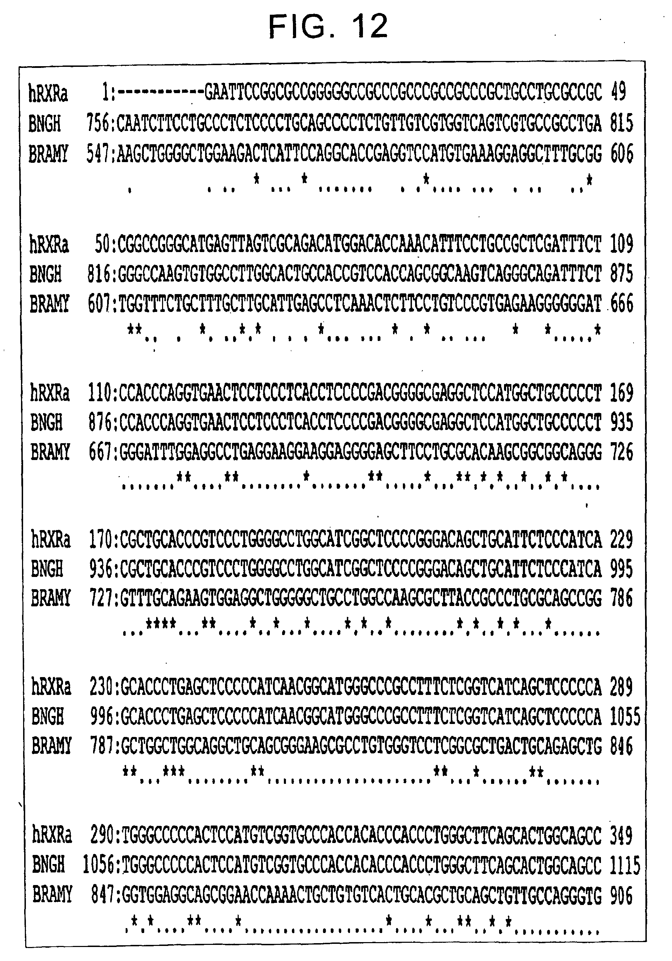

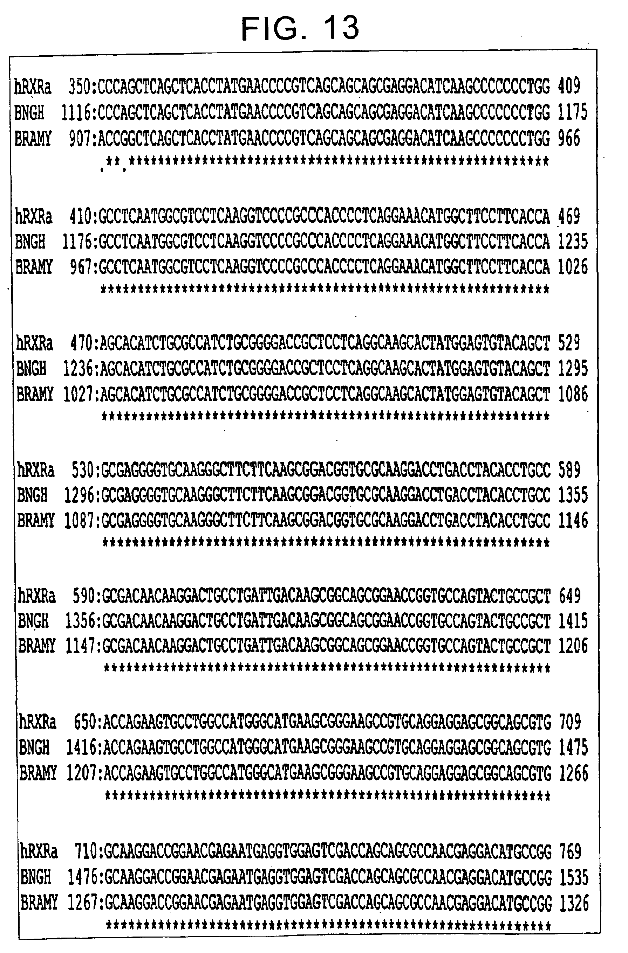

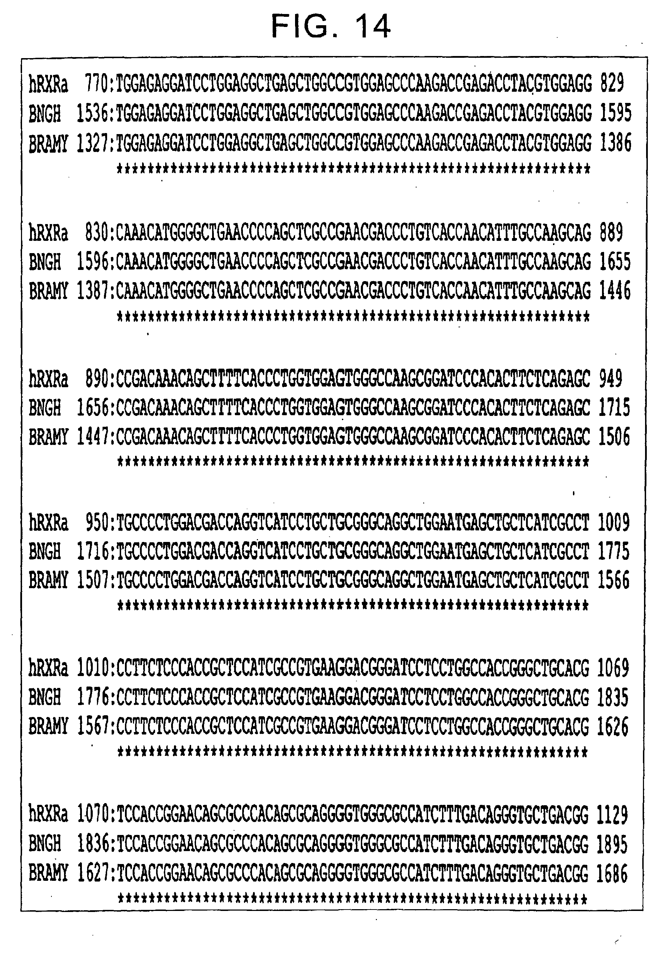

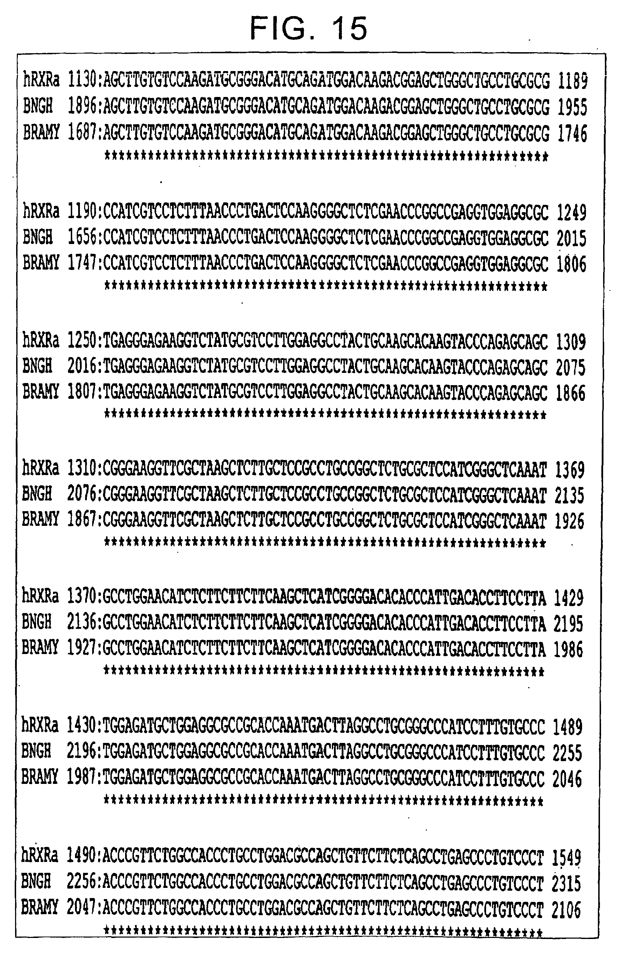

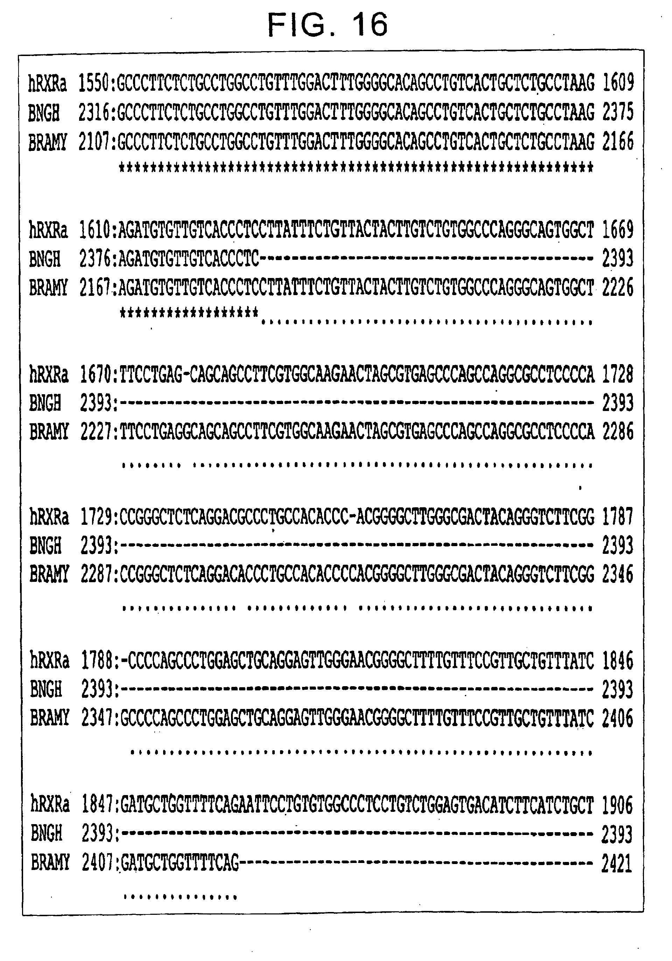

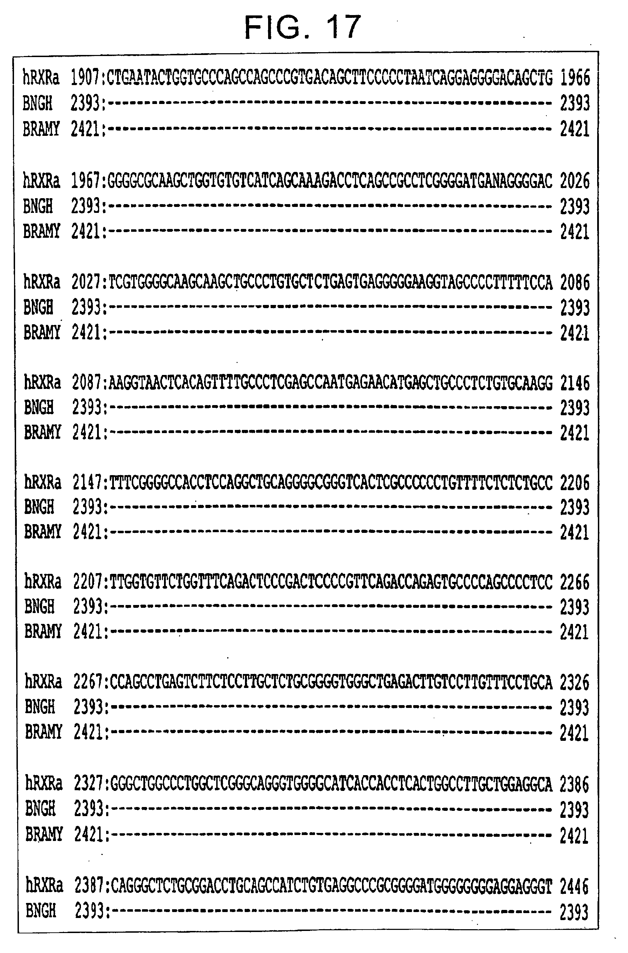

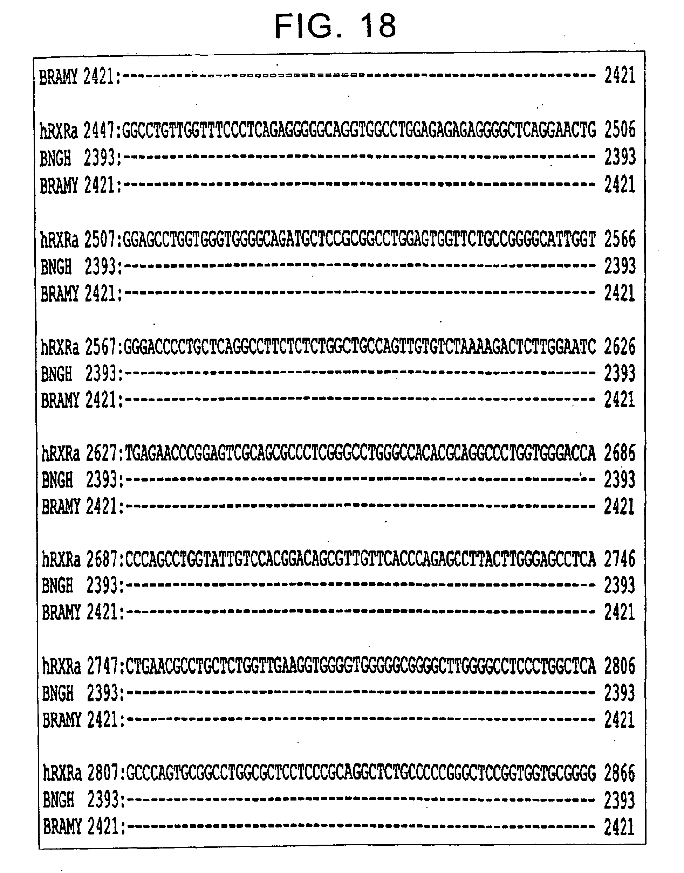

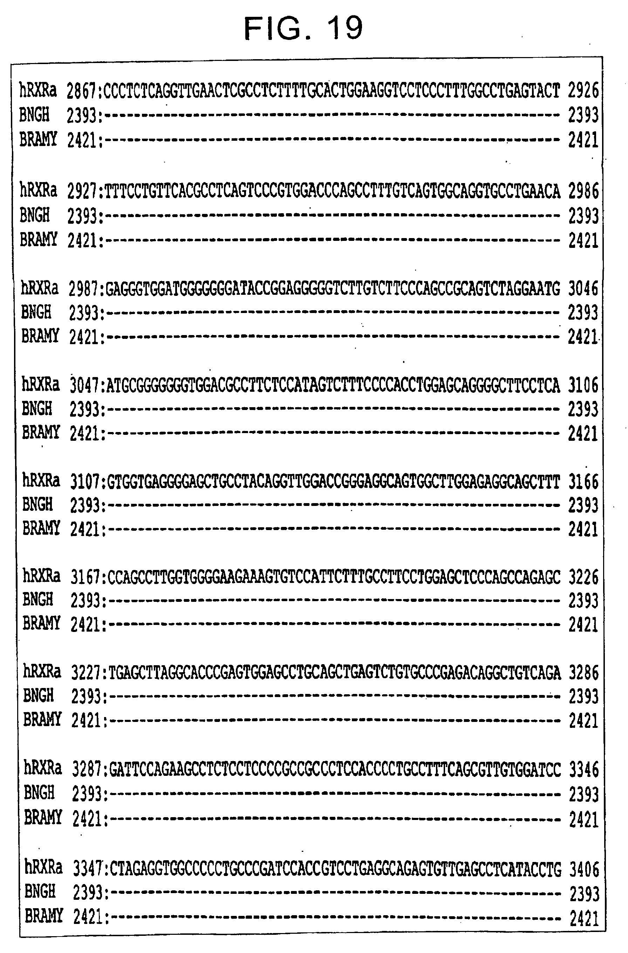

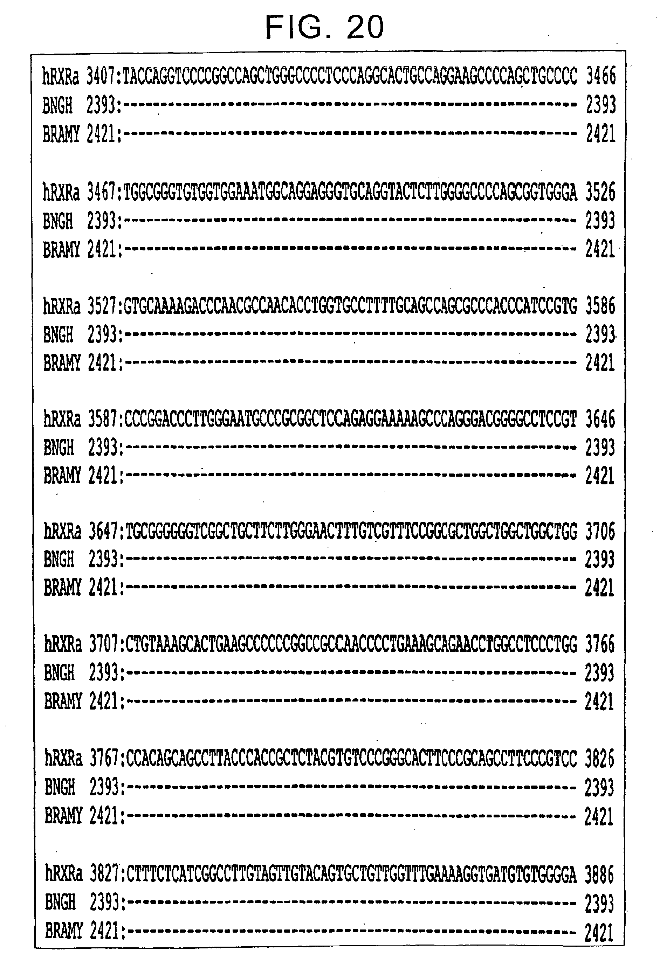

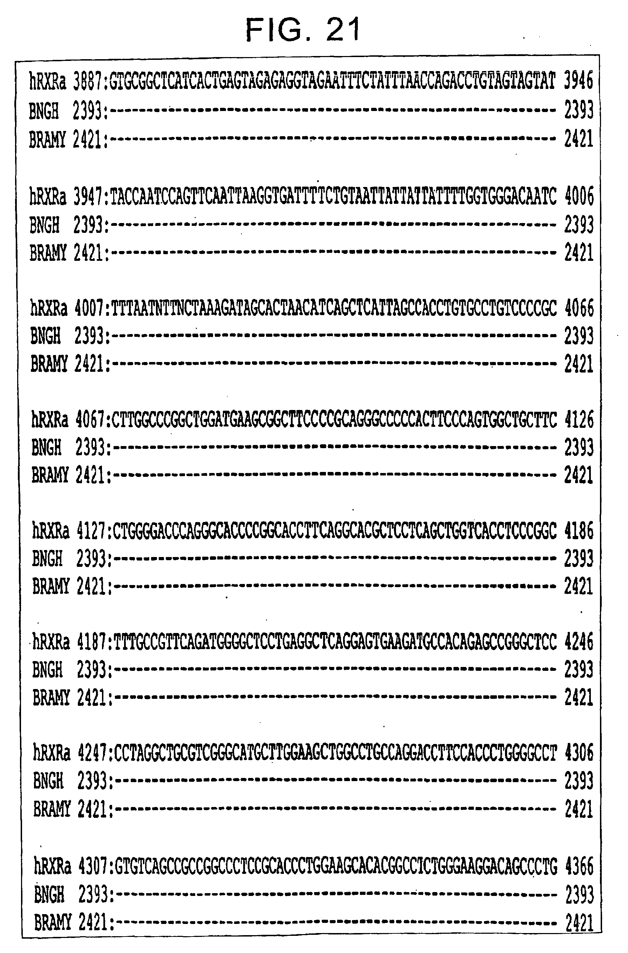

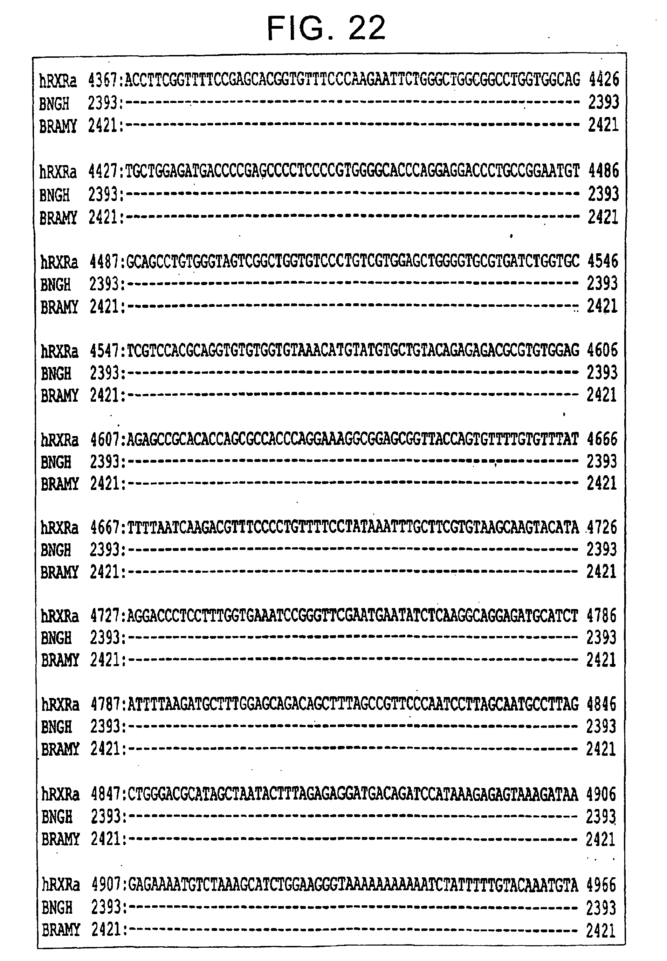

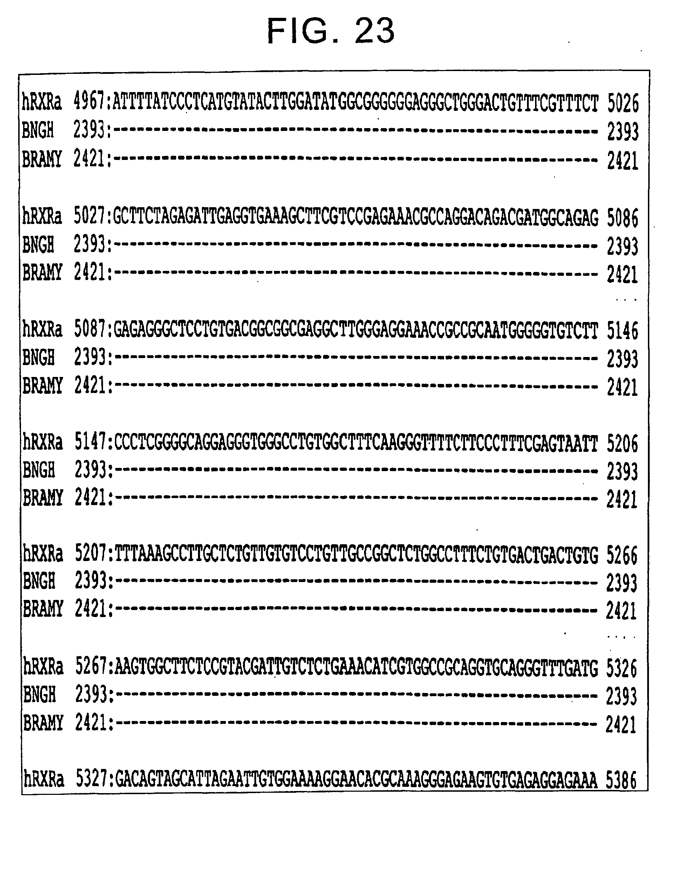

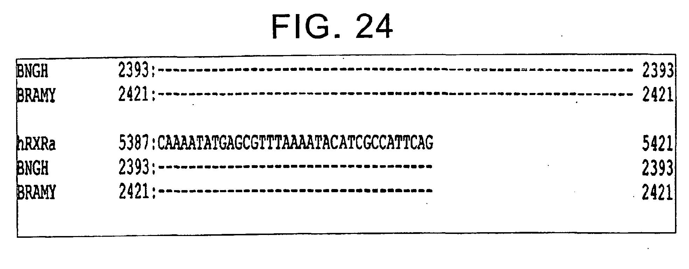

The 2393 base pair cDNA for RXRα2 (C-BNGH42008649) (Figs. 2-5; SEQ ID NO: 1), and the 2421 base pair cDNA for RXRα3 (C-BRAMY2003287) (Figs. 6-9; SEQ ID NO: 3) , were subj ected to sequence homology searches and functional motif searches using bioSCOUT DNA sequence analysis software (Lion) on GenBank-, EMBL- and SWISS-PROT-derived databases. Furthermore, the sequences were compared with those of RXRα isoforms previously reported, based on information in the literature (Figs. 10-24).

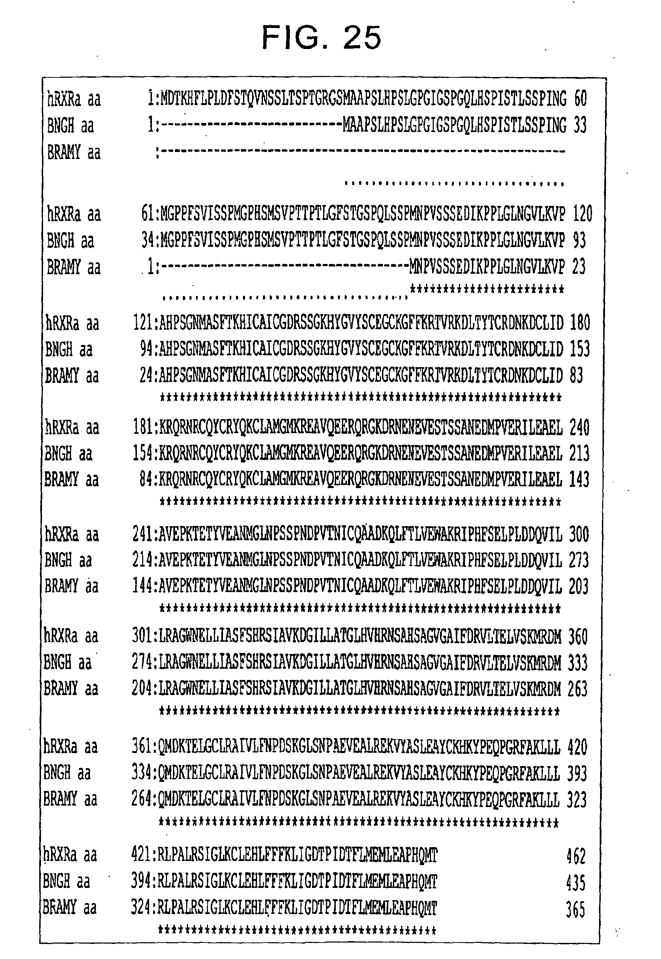

RXRα2 contains a region of 1305 base pairs (923-2230) encoding a protein of 435 amino acids. RXRα3 contains a region of 1095 base pairs (924-2021) encoding a protein of 365 amino acids. Both proteins contain a DNA binding domain (DBD) and a ligand-binding domain (LBD), common to the nuclear receptor family. RXRα1, a known isoform of RXRα, showed highest homology to both of these cDNAs. Comparison of the amino acid sequence of each revealed that RXRα2 and RXRα3 have a shorter N-terminus than RXRα1, with the deletion of 27 and 97 amino acids respectively (Fig. 25).

### [Example 5] In silico mapping of cDNAs for RXRα2 (C-BNGH42008649), and RXRα3 (C-BRAMY2003287) onto human chromosomes

The cDNA sequences of RXRα2 (C-BNGH42008649), and RXRα3 (C-BRAMY2003287) were searched against the GenBank and Sanger Center human genomic sequence databases using BLAST. With the exception of an unmatched part, the cDNA sequences matched the sequence of a clone on human chromosome 9q34.3, and the exon sequences were determined. Because the genomic sequence database is still a draft, literature reporting the result of experimental analysis of the genomic structure of RXRα1 (Li G., Walch E., Yang X., Lipman S.M., and Clifford J.L. Cloning and characterization of the human retinoid X receptor á gene: conservation of structure with the mouse homolog. BBRC 269: 54-57 (2000) ) was used as a reference to predict the genomic structure of each isoform (Fig. 26). The results indicated that the RXRα2 and RXRα3 genes are composed of at least 10 and 9 exons respectively. The boundary sequences of the intron sequences, which were unambiguously determined from these exons, fulfill the GT-AT rule, thus demonstrating that differences between the RXRα2 and RXRα3 cDNAs and the known isoform are not artifacts.

### [Example 6] Evaluation of RXRα2 and RXRα3 cDNA expression in human tissues

The tissue distribution of RXRα2 and RXRα3 expression was examined by PCR, using commercially available cDNA from human tissues as a template, and isoform-specific primer sets. 20 µL of PCR reaction mixture contains 1 µg of cDNA from human heart, kidney, liver, spleen, brain, or skeletal muscle, 2 µl of 10x Ex Taq Buffer, 1.6 µl of dNTP mixture, 1 µl of Ex Taq (TAKARA SHUZO), 1 µl of Perfect Match PCR Enhancer (STRATAGENE) , 1 µl of Forward primer, 1 µl of Reverse primer, and 11.4 µl of sterile water.

The nucleotide sequence of the primers is as follows:
For RXRα1 amplification: 
exrxra1s (AACATTTCCTGCCGCTCGATT/ SEQ ID NO: 11)
exrxra1a (CCTCATTCTCGTTCCGGTCCTT/SEQ ID NO: 12)
For RXRα2 amplification: 
exrxra2s (CCCTTTCTGTGCCCCCTGCCTGA/ SEQ ID NO: 13)
exrxra2a (CTTGTTGTCGCGGCAGGTGTAGGTCA/ SEQ ID NO: 14)
For RXRα3 amplification:

exrxra3s (GGGAAGCGCCTGTGGTCCTCG/ SEQ ID NO: 15)
exrxra3a (GGTTCAGCCCCATGTTTGCCTCCACGTA/ SEQ ID NO: 16)

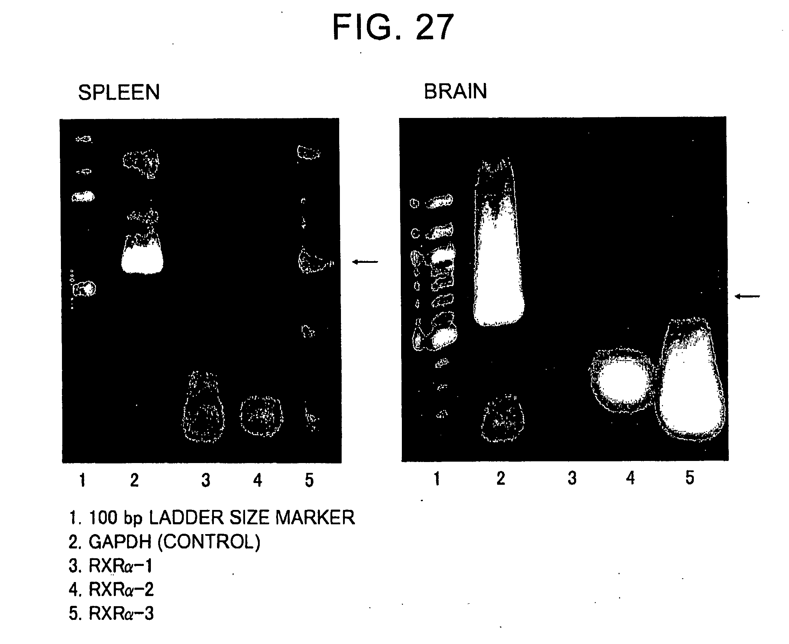

PCR amplification was performed for 35 cycles of 94°C for 1 minute, 55°C for 2 minutes, and 72°C for 3 minutes. Glyceraldehyde-3-phosphate dehydrogenase (GAPDH) was also amplified by PCR from each tissue under the same conditions, and used as an expression level control. RXRα1 expression was detected in kidney, spleen, liver, adipocytes, and prostate; RXRα3 expression was detected in brain, spleen, and prostate (Fig. 27). Under these experimental conditions, the tissue expression of RXRα2 was under detectable limits.

### [Example 7] Construction of a reporter gene assay system for evaluation of the transcription controlling activity of RXRα2 and RXRα3

In order to construct a reporter gene assay system for evaluation of the transcription-controlling activity of RXRα2 and RXRα3, a reporter plasmid into which RXRE (RXR response element) was inserted was prepared along with expression plasmids for RXRα2, RXRα3, and RXRα1, as follows: A reporter gene assay was performed by transfecting the reporter plasmid and expression plasmids into cultured cells by lipofection, followed by incubating the cells for a certain time, treating the cells with cell lysis solution, and measuring reporter activity with a luminometer using a Dual luciferase assay.

RXRE reporter plasmid was constructed by inserting multiple synthetic DNAs, corresponding to an RXRE sequence, into the BglII site of the luciferase reporter vector PGVP2 in tandem. Synthetic DNAs of 26 mer (5'-GATCTGAGGTCAGAGGTCAGAGAGCA-3'/SEQ ID NO: 17; 5'-GATCTGCTCTCTGACCTCTGACCTCA-3'/ SEQ ID NO: 18), corresponding to RXRE (RXR response element) and complementary to each other, were phosphorylated at the 5'-end with T4 polynucleotide kinase. The complementary strands were mixed at a 1:1 ratio, and annealed by heat denaturation at 65°C for 15 minutes, then slowly cooled to prepare the insert DNA fragment. The fragment was mixed with BglII-digested PGVP2 vector, ligated with T4 DNA ligase, and used to transform *E. coli* DH5α. Transformation was performed using commercial *E.coli* DH5α competent cells (TOYOBO) and standard methods, and transformants were selected for ampicillin resistance. The obtained transformants were examined by colony PCR for the presence or absence and size of the insert fragment in the recombinant plasmid. The nucleotide sequences of clones containing multiple inserts was then examined, and the number of inserted response elements and their orientation was confirmed. From these, a clone containing five response elements, in tandem and in the sense direction, was selected as a reporter plasmid.

Expression plasmids for RXRα1, RXRα2, and RXRα3 were constructed by inserting sequences comprising coding regions into the multi-cloning site of expression vector pcDNA3.1(+). The insert fragments were prepared by PCR amplification using pME18SFL3-C-BNGH42008649 as a template for RXRα2 (C-BNGH42008649), and pME18SFL3-C-BRAMY2003287 as a template for RXRα3 (C-BRAMY2003287). The control RXRα1 was prepared by PCR using cDNA from human kidney as a template. The primers used for PCR were as follows:
For RXRα1: 
5'-GAGAAGCTTAGTCGCAGACATGGACAC-3'/ SEQ ID NO: 19
5'-GCGTCTAGACAAAGTCCAAACAGGCCA-3'/ SEQ ID NO: 20
For RXRα2: 
5'-CACAAGCTTC ACCTATGAACCCCGTCA-3'/ SEQ ID NO: 21
5'-CGCTCTAGAACACATCTCTTAGGCAGA-3'/ SEQ ID NO: 22
For RXRα3:

5'-CACAAGCTTCAAGTCAGGGCAGATTTC-3'/ SEQ ID NO: 23
5'-GGGTCTAGATGACAACACATCTCTTAG-3'/ SEQ ID NO: 24

PCR amplification was performed on 35 cycles of 94°C for 1 minute, 55°C for 2 minutes, and 72°C for 3 minutes. The resulting insert fragments and expression vectors were digested with HindIII and XbaI, separated and purified by electrophoresis on an agarose gel, and then ligated with T4 DNA ligase. The reaction mixture was used to transform *E.coli* DH5α cells, and transformants were selected for ampicillin resistance. The obtained transformants were checked by colony PCR for the presence of inserts, and the nucleotide sequence was determined to confirm that the sequence did not contain mutations.

The DNAs of the reporter plasmids and expression plasmids were prepared by large scale culturing of strains carrying the plasmids, using a commercial DNA preparation kit (Qiagen). They were then subjected to reporter gene assays.

### [Example 8] The transcription-controlling activities of RXRα2 and RXRα3

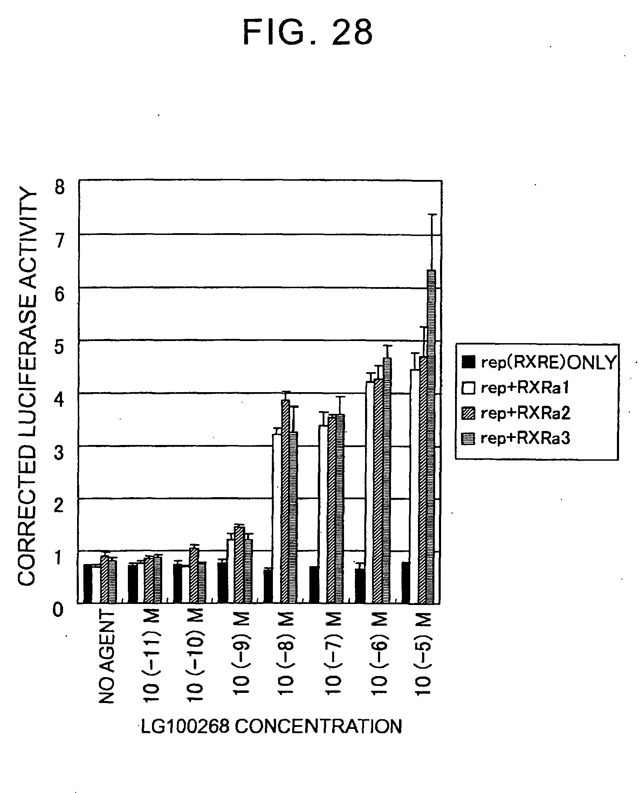

The transcription-controlling activities of RXRα2 and RXRα3 were evaluated by using a reporter gene assay. Using RXRα1 as a control, the activities of RXRα2 and RXRα3 were compared. In addition, the response of each to an RXRα agonist, LG100268, was evaluated (Fig. 28).

The reporter gene assay was performed using CV-1 cells (African green monkey kidney-derived cell line) as hosts. Host cells were transfected with luciferase reporter plasmids and expression plasmids for RXRα1, RXRα2, or RXRα3. They were then exposed to LG100268 at different concentrations (0, 10⁻¹⁰ to -10⁻⁵ M) for 24 hours, whereupon luciferase activity within the cells was measured.

Transfection was performed as follows: CV-1 cells were suspended at 2 x10⁵ cells/2.5 ml in DMEM medium supplemented with 10% fetal bovine serum, 2000 U/1 of penicillin G, and 100 mg/1 streptomycin. These cells were seeded to a 6-well plate at 2.5 ml/well, and cultured for 18 to 24 hours in a CO₂ incubator at 37°C in the presence of 5% CO₂. 1 µg of each of the transcription factor expression plasmid, the luciferase reporter plasmid, and another luciferase reporter plasmid for normalizing the measured value (pRL-TK) , were dissolved in Opti-MEM (Gibco BRL) to total of 150 µl. 7 µl of the cell transfection reagent LipofectAMINE 2000 (Gibco BRL) was diluted in 143 µl of Opti-MEM, and this was added to the mixture drop by drop while stirring. The mixture was incubated for 20 minutes.

The LipofectAMINE-DNA mixture was diluted in 1.5 ml of Opti-MEM, and added to cells that had been twice washed with 1 ml of PBS(-). The cells were cultured for four hours. The transfection reagent was removed from the culture, and 200 µl of trypsin/EDTA was added to the cells, which were then incubated at 37°C for five minutes. 1 µl of DMEM was added to suspend the cells, which were transferred to a centrifuge tube, and centrifuged for three minutes at 1000 rpm. After centrifugation, the cells were resuspended in DMEM to a concentration of 1.6 x10⁴ cells/100 µl, and 100 µl samples were seeded to a 96-well plate. When evaluating agents, a two-fold concentrated solution was made by pre-diluting a 1000 times concentrated agent DMSO solution with culture medium. 50 µl of the solution was mixed with 50 µl of a cell suspension at 1.6x10⁴ cells/50 µl, and the culture was incubated in a CO₂ incubator at 37°C and 5% CO₂ for 24 hours.

Luciferase activity was measured using the Dual-Luciferase™ Reporter Assay System (Promega). The culture supernatant was removed from each well of the 96-well plate and washed once with 100 µl of PBS(-). 20 µl of x 1 PLB (Passive Lysis Buffer) was added, and the mixture was incubated at room temperature for 15 minutes, and then stored at -20°C in the freezer until use. The luminometer used was a ARVO™ HTS 1420 multi-labeling counter (Wallac). The protocol for measurement was as follows: 50 µl of Luciferase Assay Reagent II was added to each well, stirred for 0.3 seconds and measured for one second. 50 ml of Stop&Glo™ Reagent was then added, stirred for 0.3 seconds, and measured for one second. The measured values for firefly luciferase activity were corrected by dividing by the value for Renilla luciferase activity, the internal control. Average values and standard errors were calculated from triplicate samples.

The novel isoforms RXRα2 and RXRα3 were both demonstrated to confer transcriptional activation function. However, there was no significant difference among the three receptors RXRα1, RXRα2 and RXRα3 in terms of their transcriptional activation function in response to LG100268.

### [Example 9] PPARγ-coupled transcriptional activation by RXRα2 and RXRα3

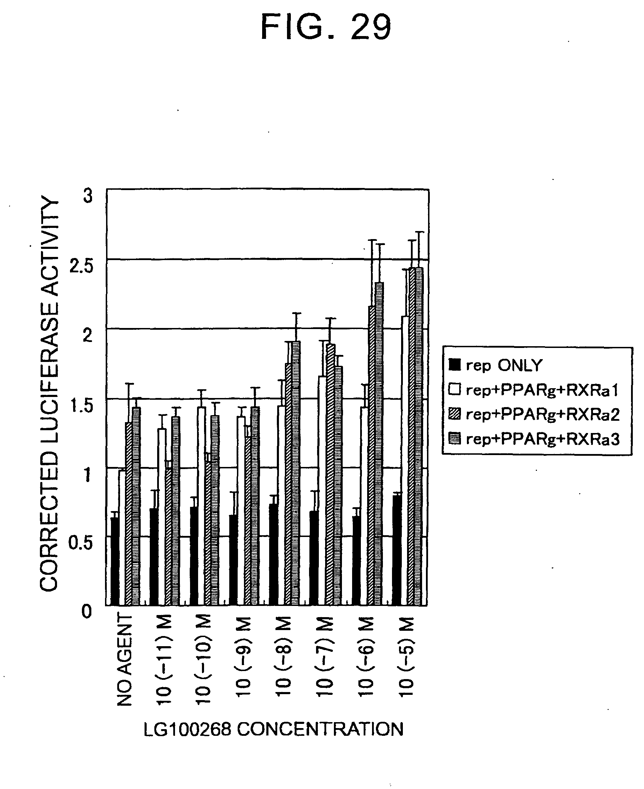

A reporter gene assay was used to compare the PPARγ-coupled transcription-controlling activities of RXRα2 and RXRα3 with that of RXRα1 (Fig. 29). The particulars of the reporter gene assay are as for Example 8, except that (1) the PPARE reporter plasmid was used in place of the RXRE reporter plasmid, and (2) the host cells were co-transfected with PPARγ expression plasmid and an expression plasmid for each RXRα.

The PPARγ-coupled transcription-controlling activities of RXRα1, RXRα2, and RXRα3 and their responses to LG100268 were not significantly different.

### [Example 10] PPARγ-coupled transcriptional activation by RXRα2 and RXRα3 in the presence of a co-activator

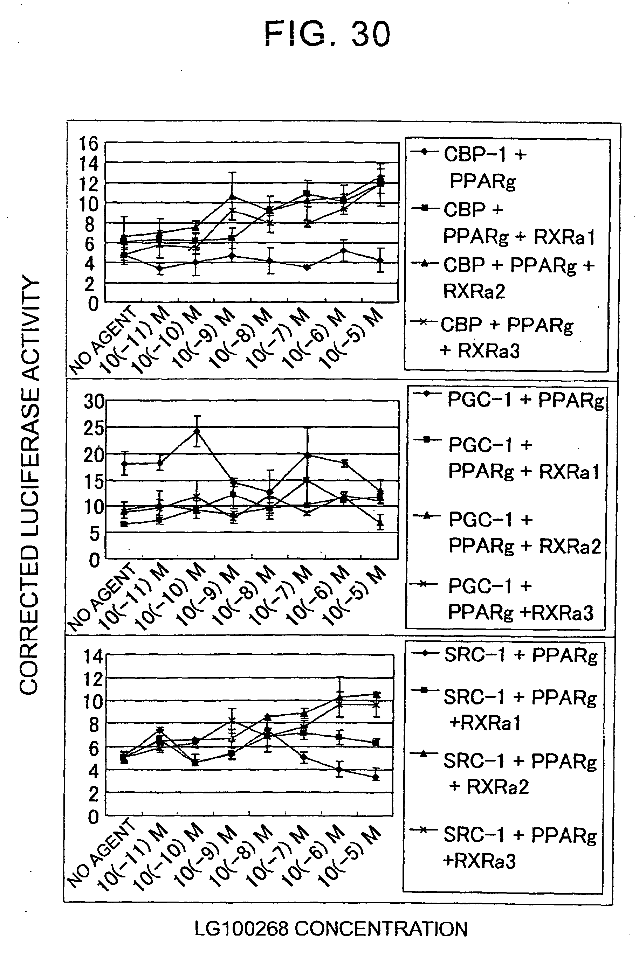

A reporter gene assay was used to compare the PPARγ-coupled transcription-controlling activities of RXRα2 and RXRα3 in the presence of a co-activator with that of RXRα1 (Fig. 30). The particulars of the reporter gene assay are as for Example 9, except that the host cells were co-transfected with PPARγ expression plasmid, each RXRα expression plasmid and each co-activator expression plasmid.

In the presence of co-activator CBP-1, the RXRα isoforms did not show a significant difference in LG100268 response. In contrast, RXRα2 and RXRα3 did not show any response to LG100268 in the presence of PCG-1, whereas RXRα1 elevated the transcriptional activation activity in a manner dependent on LG100268 concentration, though this was not remarkable. In the presence of SRC-1, RXRα1 did not shown any response to LG100268. On the other hand, RXRα2 and RXRα3 elevated their transcriptional activation activity in a manner dependent on LG100268 concentration. Since co-activators are expressed dependent on tissue and physiological conditions, and their tissue distribution patterns are different, the results of the present invention strongly suggest that the novel RXRα isoforms play physiological roles that are different from those of RXRα1.

### Industrial Applicability

The present invention provides the novel nuclear receptor isoforms, RXRα2 and RXRα3. These RXRα isoforms are nuclear receptors that bind to retinoic acid as a ligand and function with SRC-1 as a co-activator. Furthermore, as for known protein RXRα1, the RXRα2 and RXRα3 isoforms of the present invention were found to function with CBP as a co-activator.

SRC-1 co-activator is thought to have multiple in vivo functions through interaction with a variety of nuclear receptors. For example, Nishihara E. et al. constructed SRC-1 null (SRC-1 -/-) mice by gene targeting, and carried out functional analyses (Eijun Nishihara, Hiromi Yoshida-Komiya, Chi-Shing Chan, Lan Liao, Ronald L. Davis, Bert W. O'Malley, and Jianming Xu. "SRC-1 Null Mice Exhibit Moderate Motor dysfunction and Delayed Development of Cerebellar Purkinje Cells." The Journal of Neuroscience 23: 213-222 (2003)). The knockout mice were found to exhibit defective development of the uterus, prostate, testis, and mammary gland. Delays in the development of Purkinje cells were also observed from an early stage, leading to moderate motor disorder in adults. The fact that RXRα2 and RXRα3, discovered in the present invention, use SRC-1 as a co-activator is noteworthy in the context of this kind of defective organ development and motor disorder.

The RXRα isoforms discovered in the present invention are novel in their structure and co-activator specificity. By regulating the transcription-controlling activity of nuclear receptors, cellular activities can be controlled by signal transduction. Based on this kind of idea, a number of nuclear receptors have been functionally analyzed to date. Attempts have been made to regulate cellular activities by regulating the functions thus revealed. However, many of those attempts have focused on the interaction between a particular nuclear receptor and its ligand. The transcription-controlling activity of nuclear receptors is known to be controlled by co-activators. However, not much attention has been paid to the fact that there are some differences in their co-activator specificity, even among closely related molecules such as isoforms. However, the research of the present inventors has revealed that nuclear receptor activity is precisely controlled, not only by the ligand, but also by co-activator specificity. In other words, in order to regulate signal transduction pathways by targeting nuclear receptors, consideration is clearly required of not only functions with particular ligands, but also of interactions with co-activators that influence the nuclear receptor's transcription-controlling activity.

The present invention provides methods for evaluating the activity of controlling the function of co-activators that regulate the transcription-controlling activity of RXRα isoforms. Furthermore, the invention provides methods for evaluating the effect of a test substance on the transcription activation enhancing function of co-activators. These evaluation methods enable evaluation of a test substance's activity to regulate a co-activator's function in enhancing each RXRα isoform's transcriptional activation function. Accordingly, based on the present invention, compounds comprising the function of controlling the above-mentioned function of enhancing transcriptional activation can be screened. Compounds identified by the screening methods of the present invention can regulate the function of enhancing transcriptional activation by RXRα isoforms. Since RXRα isoforms differ in their co-activator specificity, isoform-specific activity can be regulated by the compounds selected by these screening methods. Such compounds are useful as agents that act on novel targets in controlling cellular activities.

In addition, RXRα2 or RXRα3 themselves, or compounds capable of controlling the activity or expression of these proteins, may be useful therapeutic agents for diseases caused by abnormalities in the transcription-controlling activity of nuclear receptors.

The RXRα2 or RXRα3 proteins may be useful in the treatment of diseases characterized by reduced nuclear receptor expression or activity. More specifically, a variety of cancers, hyperlipemia, arteriosclerosis, diabetes, a variety of inflammatory disease such as enteric inflammation and psoriasis, are considered due to impaired RXR function. Cancers include prostate cancers, acute promyelocytic leukemia, and hepatic cancers.

Furthermore, the present invention also provides antisenses for the polynucleotides encoding RXRα2 or RXRα3, as well as double-stranded RNAs, or proteins thereof. Antisenses or proteins comprising dominant negative function may be useful for treating diseases characterized by abnormalities in the transcription-controlling activity of nuclear receptors.

## Claims

1. A polynucleotide of any one of the following (A) to (E):
(A) a polynucleotide comprising the coding region of the nucleotide sequence of SEQ ID NO: 1 or 3;
(B) a polynucleotide encoding a protein comprising the amino acid sequence of SEQ ID NO: 2 or 4;
(C) a polynucleotide encoding a protein comprising the amino acid sequence wherein one or more amino acids are replaced, deleted, inserted, and/or added to the amino acid sequence of SEQ ID NO: 2 or 4, encoding a protein comprising the following (a) and (b):
(a) The transcriptional activation function is enhanced in response to binding to the ligand retinoic acid, or its agonist; and
(b) The protein requires SRC-1 as a co-activator;
(D) a polynucleotide that hybridizes under stringent conditions with a polynucleotide comprising the nucleotide sequence of SEQ ID NO: 1 or 3, encoding a protein comprising the following (a) and (b):
(a) The transcriptional activation function is enhanced in response to binding to the ligand retinoic acid, or its agonist; and
(b) The protein requires SRC-1 as a co-activator;
(E) a polynucleotide comprising a nucleotide sequence in which the nucleotide sequence from 1 to 102 of SEQ ID NO: 5 is deleted, or replaced with another nucleotide sequence, where the sequence has 70% or more homology to the nucleotide sequence from 103 to 1461 of SEQ ID NO: 5.

2. A protein encoded by the polynucleotide of claim 1.

3. A vector comprising the polynucleotide of claim 1.

4. A transformant maintaining the polynucleotide of claim 1; or the vector of claim 3.

5. A method for producing the protein of claim 2, comprising the steps of culturing the transformant of claim 4, and recovering the expressed product.

6. A method for detecting the activity of a test substance that regulates the transcription-controlling activity of a nuclear receptor, wherein the nuclear receptor is a protein encoded by the polynucleotide of any one of the following (A) to (E):
(A) a polynucleotide comprising the coding region of the nucleotide sequence of SEQ ID NO: 1 or 3;
(B) a polynucleotide encoding a protein comprising the amino acid sequence of SEQ ID NO: 2 or 4;
(C) a polynucleotide encoding a protein comprising an amino acid sequence wherein one or more amino acids are replaced, deleted, inserted, and/or added to the amino acid sequence of SEQ ID NO: 2 or 4, where the protein comprises the following (a) and (b):
(a) The transcriptional activation function is enhanced in response to binding to the ligand retinoic acid, or its agonist; and
(b) The protein requires.SRC-1 as a co-activator;
(D) a polynucleotide that hybridizes under stringent conditions with a polynucleotide comprising the nucleotide sequence of SEQ ID NO: 1 or 3, encoding a protein comprising the following (a) and (b):
(a) The transcriptional activation function is enhanced in response to binding to the ligand retinoic acid, or its agonist; and
(b) The protein requires SRC-1 as a co-activator;
(E) a polynucleotide comprising a nucleotide sequence in which the nucleotide sequence from 1 to 102 of SEQ ID NO: 5 is deleted, or replaced with another nucleotide sequence, and which comprises 70% or more homology to the nucleotide sequence from 103 to 1461 of SEQ ID NO: 5;
wherein the method comprises the following steps:
(1) contacting a test substance with cells, which express a nuclear receptor and comprise an expression cassette in which a reporter gene is inserted downstream of a responsive element for the nuclear receptor, in the presence of a co-activator that activates the nuclear receptor;
(2) culturing the cells under conditions enabling the nuclear receptor expression, and measuring the expression level of the reporter gene within the cells; and
(3) detecting the activity of the test substance regulating the transcription-controlling activity of the nuclear receptor, using the measurement result of step (2) as an index.

7. The method of claim 6, wherein the method additionally comprises the step of detecting the activity of a test substance on a nuclear receptor that is a protein encoded by the polynucleotide of the following (A') to (E'):
(A') a polynucleotide comprising the coding region of the nucleotide sequence of SEQ ID NO: 5;
(B') a polynucleotide encoding a protein comprising the amino acid sequence of SEQ ID NO: 6;
(C') a polynucleotide encoding a protein comprising an amino acid sequence wherein one or more amino acids are replaced, deleted, inserted, and/or added to the amino acid sequence of SEQ ID NO: 6, wherein the protein is functionally equivalent to a protein comprising the amino acid sequence of SEQ ID NO: 6;
(D') a polynucleotide that hybridizes with a polynucleotide comprising the nucleotide sequence of SEQ ID NO: 5 under stringent conditions, encoding a protein functionally equivalent to a protein comprising the amino acid sequence of SEQ ID NO: 6; and
(E') a polynucleotide comprising 60% or more homology to the nucleotide sequence of SEQ ID NO: 5, encoding a protein functionally equivalent to a protein comprising the amino acid sequence of SEQ ID NO: 6.

8. A method for detecting the activity of a test substance controlling the binding between a nuclear receptor and a co-activator that activates the receptor, wherein the nuclear receptor is a protein encoded by the polynucleotide of any one of the following (A) to (E) :
(A) a polynucleotide comprising the coding region of the nucleotide sequence of SEQ ID NO: 1 or 3;
(B) a polynucleotide encoding a protein comprising the amino acid sequence of SEQ ID NO: 2 or 4;
(C) a polynucleotide encoding a protein comprising an amino acid sequence wherein one or more amino acids are replaced, deleted, inserted, and/or added to the amino acid sequence of SEQ ID NO: 2 or 4, encoding a protein comprising the following (a) and (b):
(a) The transcriptional activation function is enhanced in response to binding to the ligand retinoic acid, or its agonist; and
(b) The protein requires SRC-1 as a co-activator;
(D) a polynucleotide that hybridizes with a polynucleotide comprising the nucleotide sequence of SEQ ID NO: 1 or 3 under stringent conditions, encoding a protein comprising the following (a) and (b):
(a) The transcriptional activation function is enhanced in response to binding to the ligand retinoic acid, or its agonist; and
(b) The protein requires SRC-1 as a co-activator;
(E) a polynucleotide comprising a nucleotide sequence in which the nucleotide sequence from 1 to 102 of SEQ ID NO: 5 is deleted, or replaced with another nucleotide sequence, and which comprises 70% or more homology to the nucleotide sequence from 103 to 1461 of SEQ ID NO: 5;
wherein the method comprises the following steps:
(1) contacting the nuclear receptor, SRC-1, and the test substance in any of the following orders i) to iii):
i) contacting the nuclear receptor and the test substance first, and then contacting with SRC-1;
ii) contacting the nuclear receptor and SRC-1 in the presence of the test substance; or
iii) contacting the nuclear receptor and SRC-1 first, and then contacting with the test substance;
(2) measuring the amount of SRC-1 or test substance bound to the nuclear receptor; and
(3) detecting the activity of the test substance in binding to the nuclear receptor, using the measurement result of (2) as an index.

9. A method for evaluating a substance capable of regulating the transcription-controlling activity of a nuclear receptor, comprising the following steps:
(1) detecting the activity of a test substance in regulating the transcription-controlling activity of a nuclear receptor using the method of claim 6; and
(2) selecting a test substance capable of inhibiting or enhancing the transcriptional activation function of the nuclear receptor by comparing with control.

10. A method for evaluating a test substance capable of regulating the transcription-controlling activity of a specific nuclear receptor isoform, where the method comprises the following steps:
(1) detecting the activity of a test substance in regulating the transcription-controlling activity of different kinds of nuclear receptors that comprise a protein encoded by the polynucleotide of any one of claim 6 (A) to (E), using the method of claim 6;
(2) comparing the activity of a test substance in regulating the transcription-controlling activity of each of the different kinds of nuclear receptor; and
(3) selecting a test substance whose effect on transcription-controlling activity differs between isoforms.

11. A method for evaluating a test substance capable of regulating the transcription-controlling activity of a nuclear receptor, comprising the following steps:
(1) detecting the activity of the test substance in controlling the binding between the nuclear receptor and a co-activator activating it, using the method of claim 8, and
(2) selecting, by comparison with a control, a test substance capable of inhibiting or enhancing the binding of the co-activator to the nuclear receptor.

12. An agent for controlling the activity of a nuclear receptor, comprising as an active ingredient a substance selected by the method of claim 9.

13. An agent for controlling the activity of a nuclear receptor in an isoform-specific manner, comprising as an active ingredient a substance selected by the method of claim 10.

14. An agent for controlling the activity of a nuclear receptor, comprising as an active ingredient a substance selected by the method of claim 11.

15. An agent for controlling the activity of a nuclear receptor, comprising as the main ingredient a polynucleotide of any one of the following (A) to (E), or a protein encoded by the polynucleotide,
(A) a polynucleotide comprising the coding region of the nucleotide sequence of SEQ ID NO: 1 or 3;
(B) a polynucleotide encoding the amino acid sequence of SEQ ID NO: 2 or 4;
(C) a polynucleotide encoding a protein comprising the amino acid sequence wherein one or more amino acids are replaced, deleted, inserted, and/or added to the amino acid sequence of SEQ ID NO: 2 or 4, encoding a protein comprising the following (a) and (b):
(a) The transcriptional activation function is enhanced in response to binding to the ligand retinoic acid, or its agonist; and
(b) The protein requires SRC-1 as a co-activator;
(D) a polynucleotide that hybridizes under stringent conditions with a polynucleotide comprising the nucleotide sequence of SEQ ID NO: 1 or 3, encoding a protein comprising the following (a) and (b):
(a) The transcriptional activation function is enhanced in response to binding to the ligand retinoic acid, or its agonist; and
(b) The protein requires SRC-1 as a co-activator;
(E) a polynucleotide comprising the nucleotide sequence in which the nucleotide sequence from 1 to 102 of SEQ ID NO: 5 is deleted, or replaced with another nucleotide sequence, and which comprises 70% or more homology to the nucleotide sequence from 103 to 1461 of SEQ ID NO: 5.

16. An agent for suppressing the activity of a nuclear receptor, comprising as an active ingredient a component of any one of the following (1) to (4);
(1) an antisense polynucleotide of the polynucleotide of the above (A) to (E);
(2) an antibody that recognizes a protein encoded by the polynucleotide of the above (A) to (E);
(3) a protein exerting a dominant-negative effect against a protein encoded by the polynucleotide of the above (A) to (E) ; and
(4) a double-stranded RNA that comprises 21 to 23 base pairs, comprising a sense RNA corresponding to a partial sequence of the nucleotide sequence of the polynucleotide of the above (A) to (E), and its antisense RNA.

17. A model animal wherein the activity of a nuclear receptor is controlled, wherein the animal is a transgenic non-human animal in which the expression of a protein encoded by the polynucleotide of any one of the following (A) to (E) is controlled,
(A) a polynucleotide comprising the coding region of the nucleotide sequence of SEQ ID NO: 1 or 3;
(B) a polynucleotide encoding the amino acid sequence of SEQ ID NO: 2 or 4;
(C) a polynucleotide encoding a protein comprising an amino acid sequence wherein one or more amino acids are replaced, deleted, inserted, and/or added to the amino acid sequence of SEQ ID NO: 2 or 4, encoding a protein comprising the following (a) and (b):
(a) The transcriptional activation function is enhanced in response to binding to the ligand retinoic acid, or its agonist; and
(b) The protein requires SRC-1 as a co-activator;
(D) a polynucleotide that hybridizes with a polynucleotide comprising the coding region of the nucleotide sequence of SEQ ID NO: 1 or 3 under stringent conditions, encoding a protein comprising the following (a) and (b):
(a) The transcriptional activation function is enhanced in response to binding to the ligand retinoic acid, or its agonist; and
(b) The protein requires SRC-1 as a co-activator;
(E) a polynucleotide comprising a nucleotide sequence in which the nucleotide sequence from 1 to 102 of SEQ ID NO: 5 is deleted, or replaced with another nucleotide sequence, and which comprises 70% or more homology to the nucleotide sequence from 103 to 1461 of SEQ ID NO: 5.

18. A method of diagnosing a disease caused by the abnormal activity of a nuclear receptor, comprising the steps of measuring the expression level of a polynucleotide in a biological sample collected from a subject, and judging that, if the expression level is low compared with a control, the subject has a disease resulting from impaired function of a protein encoded by the polynucleotide, and that, in contrast, if the expression level is high, the subject has a disease resulting from elevated function of the protein, wherein the polynucleotide is any one of the following (A) to (E):
(A) a polynucleotide comprising the coding region of the nucleotide sequence of SEQ ID NO: 1 or 3;
(B) a polynucleotide encoding the amino acid sequence of SEQ ID NO: 2 or 4;
(C) a polynucleotide encoding a protein comprising an amino acid sequence wherein one or more amino acids are replaced, deleted, inserted, and/or added to the amino acid sequence of SEQ ID NO: 2 or 4, encoding a protein comprising the following (a) and (b):
(a) The transcriptional activation function is enhanced in response to binding to the ligand retinoic acid, or its agonist; and
(b) The protein requires SRC-1 as a co-activator;
(D) a polynucleotide that hybridizes under stringent conditions with a polynucleotide comprising the coding region of the nucleotide sequence of SEQ ID NO: 1 or 3, encoding a protein comprising the following (a) and (b):
(a) The transcriptional activation function is enhanced in response to binding to the ligand retinoic acid, or its agonist; and
(b) The protein requires SRC-1 as a co-activator;
(E) a polynucleotide comprising a nucleotide sequence in which the nucleotide sequence from 1 to 102 of SEQ ID NO: 5 is deleted, or replaced with another nucleotide sequence, and which comprises 70% or more homology to the nucleotide sequence from 103 to 1461 of SEQ ID NO: 5.
